# EUROPEAN PATENT APPLICATION

(11) **EP 4 796 636 A1**
(43) Date of publication of application: **26.08.2026**
(21) Application number: 25160082.1
(22) Date of filing: 25.02.2025
(51) Int. Cl.: C12N 15/113

(54) **COMPOSITIONS AND METHODS FOR THE TREATMENT AND/OR PREVENTION OF A DISEASE OR CONDITION LINKED TO OXIDATIVE STRESS**

(71) Applicant: Ecole Polytechnique Fédérale de Lausanne (EPFL), 1015 Lausanne (CH)
(72) Inventor: ABRAMI, Laurence, 1025 Saint-Sulpice (CH); SARMENTO MESQUITA, Francisco, 1004 Lausanne (CH); TRONO, Didier, 1134 Vufflens-le-Château (CH); VAN DER GOOT, Gisou, 1270 Trélex (CH)
(74) Representative: KATZAROV S.A.

(57) **Abstract**

The present invention discloses compositions, such as pharmaceutical compositions, as well as methods, for use in the treatment and/or prevention of a disease or condition linked to oxidative stress, lipid peroxidation and ferroptosis. The pharmaceutical composition of the invention comprises an agent modulating the expression and/or activity of i) a KRAB-containing zinc finger protein ZNF354A (ZNF354A), ii) an mRNA encoding the ZNF354A, and/or iii) the ZNF354A gene.

## Description

### FIELD OF THE INVENTION

The present invention discloses compositions, such as pharmaceutical compositions, as well as methods, for use in the treatment and/or prevention of a disease or condition linked to oxidative stress, lipid peroxidation and ferroptosis. The pharmaceutical composition of the invention comprises an agent modulating the expression and/or activity of i) a KRAB-containing zinc finger protein ZNF354A (ZNF354A), ii) an mRNA encoding the ZNF354A, and/or iii) the ZNF354A gene.

### BACKGROUND OF THE INVENTION

Oxidative stress results from an imbalance between the production and detoxification of oxidizing free radicals. Excessive ROS results in damage of cellular components such as lipids and DNA, which leads to cell death and tissue dysfunction (Sies and Jones, 2020).

The Inventors have identified the Krüppel-associated box (KRAB) domain zinc finger protein (KZFP) ZNF354A as a key repressor of genes engaged in defence against oxidative stress, lipid peroxidation and ferroptosis, a form of ROS- and iron-dependent cell death. Depletion of ZNF354A resulted in up-regulation of antioxidative pathways and protection of cells against ROS-induced damage.

This mechanism can be therapeutically exploited to treat any disease or condition linked to oxidative stress, lipid peroxidation and ferroptosis.

### SUMMARY OF THE INVENTION

The present invention provides an agent modulating the expression and/or activity of i) a KRAB-containing zinc finger protein ZNF354A (ZNF354A), ii) an mRNA encoding the ZNF354A, and/or iii) the ZNF354A gene, for use in the treatment and/or prevention of disease.

The present invention further provides a short interfering ribonucleic acid (siRNA) for inhibiting the expression of ZNF354A.

The present invention further provides a guide ribonucleic acid (sgRNA or gRNA) targeting a genomic site of the ZNF354A gene as set forth in SEQ ID NO: 52, 53, 54 or 55, a fragment or a variant thereof , a regulatory sequence of said ZNF354A gene or a ZNF354A binding motif selected from the group of sequences set forth in SEQ ID NO: 46, 47, 48, 49, 50, and 51, or a fragment or variant of any one of these sequences.

The present invention further provides a plasmid or a vector comprising one or more nucleic acids encoding the siRNA, miRNA, piRNA, hnRNA, snRNA, gRNA, CRISPR-based loss-of-function system, esiRNA, shRNA, and antisense oligonucleotide (e.g. Gapmer antisens), or combination thereof, of the invention.

The present invention further provides a host cell comprising, or modified by the introduction of, i) a plasmid or vector of the invention, or ii) one or more nucleic acids encoding the siRNA, miRNA, piRNA, hnRNA, snRNA, gRNA, CRISPR-based loss-of-function system, esiRNA, shRNA, and antisense oligonucleotide, or combination thereof, of the invention.

The present invention further provides a pharmaceutical composition comprising i) a therapeutically effective amount of an agent modulating the expression and/or activity of i) a KRAB-containing zinc finger protein ZNF354A (ZNF354A), ii) an mRNA encoding said ZNF354A, and/or iii) the ZNF354A gene, of the invention, or ii) a plasmid or a vector of the invention, iii) a siRNA of the invention, iv) a gRNA of the invention, or iii) a host cell of the invention, and a pharmaceutically acceptable carrier or diluent.

The present invention further provides a i) ZNF354A protein as set forth in SEQ ID NO: 2, SEQ ID NO: 4, SEQ ID NO: 6, SEQ ID NO: 8, SEQ ID NO: 10, SEQ ID NO: 12, SEQ ID NO: 14, and a SEQ ID NO: 16, a fragment or a variant thereof, or ii) a nucleic acid encoding a ZNF354A protein as set forth in SEQ ID NO: 1, SEQ ID NO: 3, SEQ ID NO: 5, SEQ ID NO: 7, SEQ ID NO: 9, SEQ ID NO: 11, SEQ ID NO: 13, and a SEQ ID NO: 15, a fragment or a variant thereof, for use in the treatment and/or prevention of cancer.

The present invention further provides an agent modulating the expression and/or activity of i) a KRAB-containing zinc finger protein ZNF354A (ZNF354A), ii) an mRNA encoding said ZNF354A, and/or iii) the ZNF354A gene, for modulating cellular responses to oxidative stress.

The present invention further provides a method of detecting a disease linked to an over-activation of antioxidative pathways in a subject, the method comprising
i) detecting the level of phosphorylation of ZNF354A at serine 169,
ii) comparing the level of phosphorylation of ZNF354A at serine 169in said subject with a standard control,
wherein a high level of phosphorylation of of ZNF354A at serine 169 in said subject relative to said standard control indicates said subject suffers from a disease linked to an over-activation of antioxidative pathways.

### DESCRIPTION OF THE FIGURES

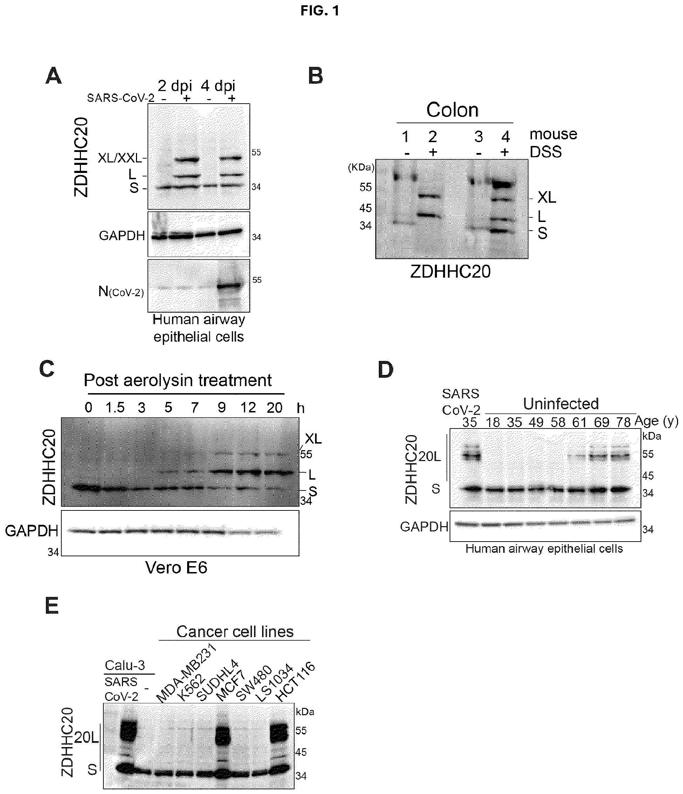

**Figure 1****. Differential regulation of the acyl-transferase ZDHHC20. (A)** WB of ZDHHC20, GAPDH (loading control), N (viral control) showing Short-S (42 KDa) and 20L (~49, 62 and 69 KDa) ZDHHC20 isoforms from lysates of: **A.** infected primary human airway epithelial cells (SARS-CoV-2, MOI 0.1); **B.** colon tissues. from mice treated or not with DSS for 7 days followed by 3 days recovery; **C.** Vero E6 cells treated 1 h with 10 ng/ml of proaerolysin at 37 °C, washed, and further incubated at 37 °C for the indicated time **D.** primary human airway epithelia from individuals with different ages or infected with SARS-CoV-2 MOI = 0.1, 48 h **E.** a panel of cancer cell lines. Calu-3 cells infected as in A for 24 h were used as a comparison.

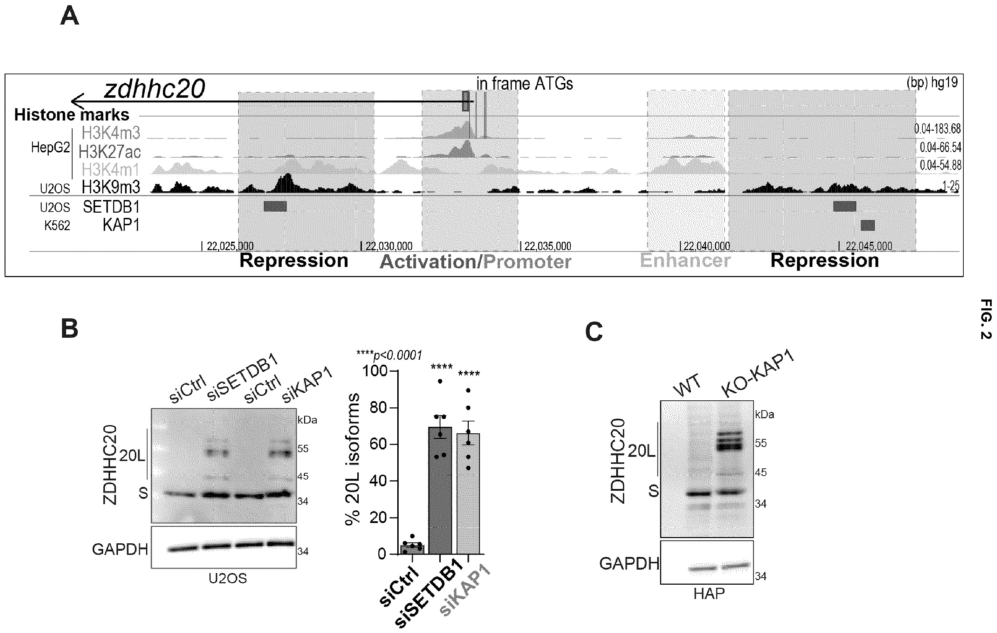

**Figure 2****. Differential expression of *Zdhhc20* isoforms is epigenetically regulated. A.** Overview of the *zdhhc20* locus (first intron, exon, and 5'UTR - version hg19), Tracks indicate: in-frame transcription start sites (ATGs); transcript (GENCODE-V4 0lift37), various histone modifications - grey shade ChIP-seq signals (ENCODE/Broad); and SETDB1/KAP1 ChIP-seq (ENCODE) peaks from the indicated cell types. **B.** WB of ZDHHC20 and GAPDH (control) in U2OS cells siControl (siCtrl), siSETDB1 or siKAP1-depleted for 72 h; and correspondent quantification of 20L band shifts. Results are mean ± SEM; each dot represents one independent experiment of n = 6; P values comparing to siCtrl were obtained by two-way ANOVA, Dunnet's multiple comparison. C. same as in B in HAP-1 wild-type or knockout for KAP1.

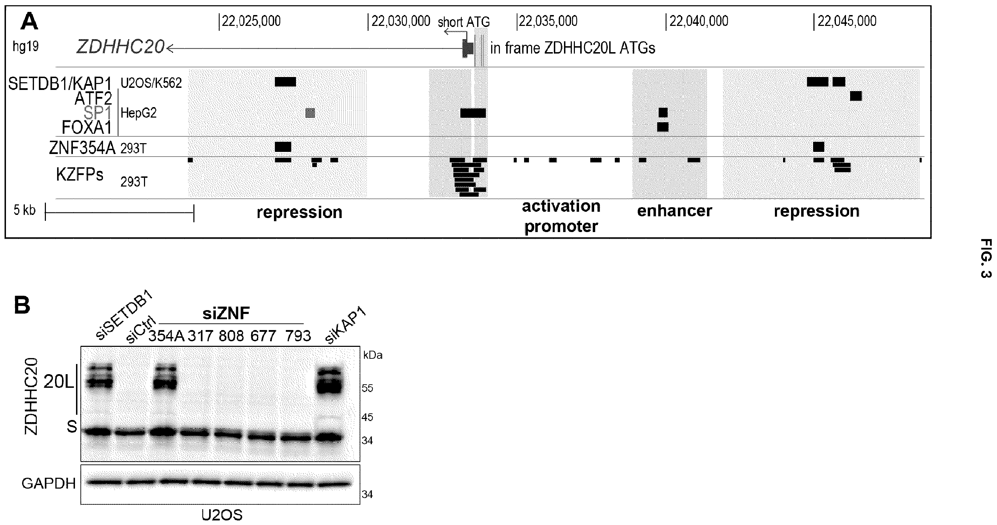

**Figure 3****. ZNF354A acts as repressor on an (alternative) upstream *zdhhc20* promoter.** A. Overview of the *zdhhc20* locus (first intron, exon, and 5'UTR - version hg19), Tracks indicate ChIP-seq peaks for: SETDB1/KAP1 (ENCODE - U2OS and K562 cells), ZNF354A and all detected KZFP bindings (HEK293T) B. WB of ZDHHC20 and GAPDH (control) in U2OS cells siControl (siCtrl), or depleted for the indicated proteins for 72 h.

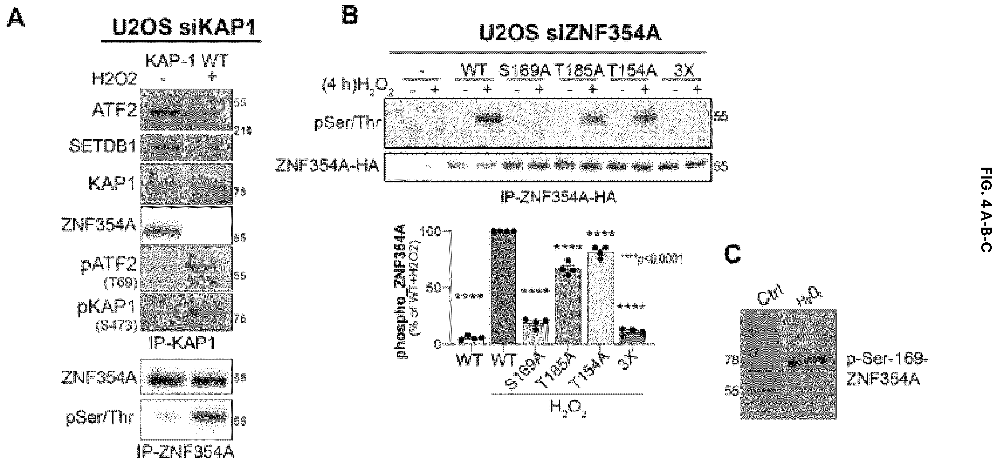

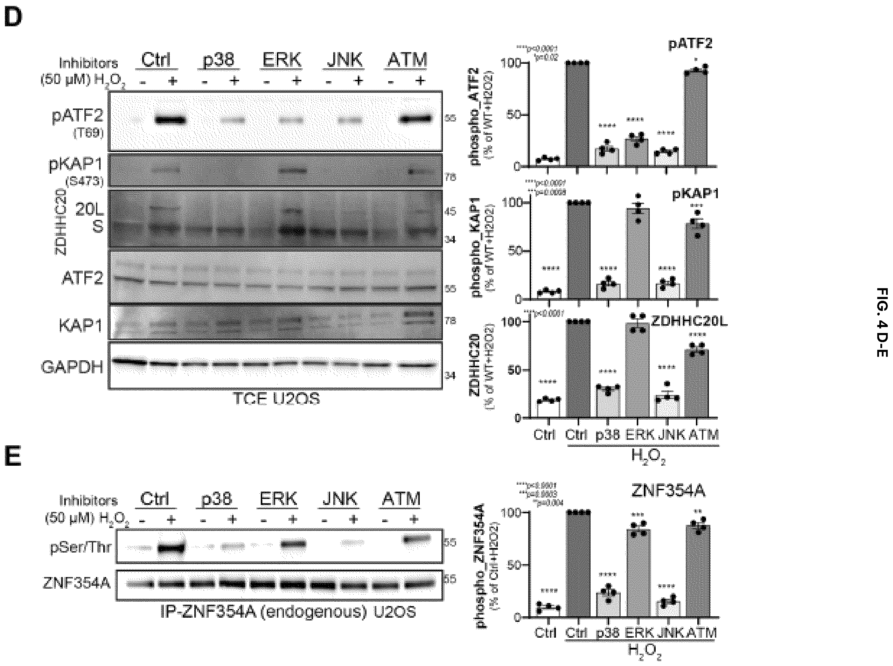

**Figure 4****. p38 and JNK kinases are is involved in activation of the LORD pathway A-C:** H₂O₂ treatment triggers phosphorylation of all three components of the repressive complex, ATF2 (Thr-69), KAP1 (Ser-473) and ZNF354A (Ser-169). An antibody was generated to specifically recognized the Ser-169 phosphorylated form of ZNF354A, with the aim of using the Ser-169 phosphorylated form of ZNF354A as a bio marker, potentially to stratify patients. **D-E:** Phosphorylation of all three component as well as ZDHHC20L expression are p38- and JNK-dependent. ATM inhibition had a mild negative effect on the phosphorylation of all three components (more pronounced for KAP1) and moderately diminished the expression of ZDHHC20L.

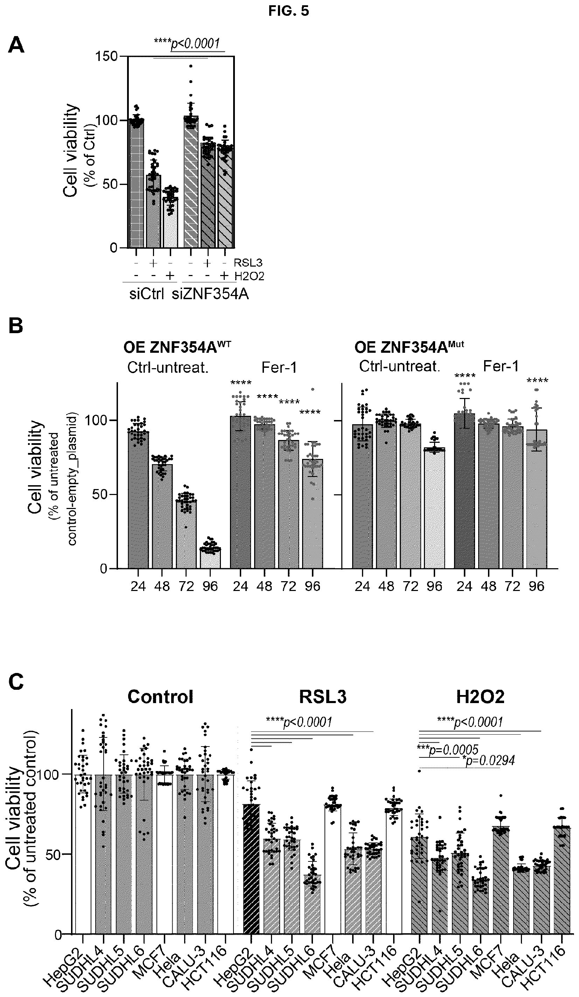

**Figure 5****. ZNF354A regulates cell sensitivity to ferroptosis** Cell viability was monitored by measuring ATP levels, following RSL3 or H₂O₂ treatment. Depletion of ZNF354A by siRNA led to a marked increase in viability both in response to RSL3 and H₂O₂ (Fig 5A). Given that silencing ZNF354A expression protects cells from ferroptosis, the inventors tested whether overexpression would sensitize them. To test this, an inactive ZNF354A mutant was designed by mutating the ZFP-KRAB-A box of ZNF354A, a well-conserved interaction domain of KZFP with KAP1. Significant cell death could be observed starting at 48 hours of ZNF354AWT, but not ZNF354AMut, overexpression (Fig 5B). Cell viability could be rescued by ferrostatin-1 (an inhibitor of ferroptosis) (Fig. 5B). Thus, ZNF354AWT overexpression leads to cell death induced by the accumulation of lipid peroxides.

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise/comprising" is generally used in the sense of include/including, that is to say permitting the presence of one or more features or components. The terms "comprise(s)" and "comprising" also encompass the more restricted ones "consist(s)", "consisting" as well as "consist/consisting essentially of", respectively.

As used in the specification and claims, the singular form "a", "an" and "the" include plural references unless the context clearly dictates otherwise.

As used herein, "at least one" means "one or more", "two or more", "three or more", etc.

As used herein the terms "subject"/"subject in need thereof", or "patient"/"patient in need thereof " are well-recognized in the art, and, are used interchangeably herein to refer to a mammal, including dog, cat, rat, mouse, monkey, cow, horse, goat, sheep, pig, camel, and, most preferably, a human. In some cases, the subject is a subject in need of treatment or a subject with a disease or disorder. However, in other aspects, the subject can be a normal subject. The term does not denote a particular age or sex. Thus, adult and newborn subjects, whether male or female, are intended to be covered. Preferably, the subject is a human, most preferably a human suffering from a disease or condition linked to oxidative stress, lipid peroxidation and ferroptosis. Preferably, the disease is linked, directly or indirectly, to the expression of ZNF354A.

The terms "nucleic acid", "polynucleotide," and "oligonucleotide" are used interchangeably and refer to any kind of deoxyribonucleotide (e.g. DNA, cDNA, ...) or ribonucleotide (e.g. RNA, mRNA, ...) polymer or a combination of deoxyribonucleotide and ribonucleotide (e.g. DNA/RNA) polymer, in linear or circular conformation, and in either single - or double - stranded form. These terms are not to be construed as limiting with respect to the length of a polymer and can encompass known analogues of natural nucleotides, as well as nucleotides that are modified in the base, sugar and/or phosphate moieties (e.g. phosphorothioate backbones). In general, an analogue of a particular nucleotide has the same base-pairing specificity, i.e., an analogue of A will base-pair with T.

The term nucleotides in the context of the present invention includes the classic ribonucleotide building blocks adenosine, guanosine, uridine (and ribosylthymin), cytidine, the classic deoxyribonucleotides deoxyadenosine, deoxyguanosine, thymidine, deoxyuridine and deoxycytidine. It further includes analogues of nucleic acids such as phosphotioates, 2'0-methylphosphothioates, peptide nucleic acids (PNA; N-(2-aminoethyl)-glycine units linked by peptide linkage, with the nucleobase attached to the alpha-carbon of the glycine) or locked nucleic acids (LNA; 2'O, 4'C methylene bridged RNA building blocks). The hybridizing sequence may be composed of any of the above nucleotides, or mixtures thereof.

The term "vector", as used herein, refers to a viral vector or to a nucleic acid (DNA or RNA) molecule such as a plasmid or other vehicle, which contains one or more heterologous nucleic acid sequence(s) of the invention and, preferably, is designed for transfer between different host cells. The terms "expression vector", "gene delivery vector" and "gene therapy vector" refer to any vector that is effective to incorporate and express one or more nucleic acid(s) of the invention, in a cell, preferably under the regulation of a promoter. A cloning or expression vector may comprise additional elements, for example, regulatory and/or post-transcriptional regulatory elements in addition to a promoter.

The term "about," particularly in reference to a given quantity, number or percentage, is meant to encompass deviations of plus or minus ten percent (± 10). For example, about 5% encompasses any value between 4.5% to 5.5%, such as 4.5, 4.6, 4.7, 4.8, 4.9, 5, 5.1, 5.2, 5.3, 5.4, or 5.5.

According to the present invention, the disease to be treated is a disease or condition linked to oxidative stress, lipid peroxidation and ferroptosis. Preferably, the disease is linked, directly or indirectly, to the expression of ZNF354A. More preferably, the disease is selected from the group comprising a cancer, a cardiovascular disease, a neurodegenerative disease and an inflammatory disease, or a combination of one of more of these diseases.

In one aspect of the invention, the disease is cancer, preferably a solid or a non-solid (liquid or hematologic) cancer. The solid cancer will be selected from the non-limiting group comprising carcinoma, sarcoma, and melanoma,. Preferably, the cancer is selected from the group comprising Adrenocortical Carcinoma, Adult, Childhood Adrenocortical Carcinoma, AIDS-Related Cancers, Kaposi Sarcoma (Soft Tissue Sarcoma), AIDS-Related Lymphoma (Lymphoma), Primary CNS Lymphoma (Lymphoma), Anal Cancer, Astrocytomas, Childhood (Brain Cancer), Atypical Teratoid/Rhabdoid Tumor, Childhood, Central Nervous System (Brain Cancer), Basal Cell Carcinoma of the Skin, Bile Duct Cancer, Bladder Cancer, Bone Cancer (includes Ewing Sarcoma and Osteosarcoma and Malignant Fibrous Histiocytoma), Brain Tumors, Breast Cancer, Childhood Breast Cancer, Childhood Bronchial Tumors, Burkitt Lymphoma, Carcinoid Tumor (Gastrointestinal), Childhood Carcinoid Tumors, Carcinoma of Unknown Primary, Cardiac (Heart) Tumors, Central Nervous System, Childhood Atypical Teratoid/Rhabdoid Tumor, Childhood Embryonal Tumors, Childhood Germ Cell Tumor, Primary CNS Lymphoma, Cervical Cancer, Childhood Cervical Cancer, Cholangiocarcinoma, Chordoma, Childhood, Chronic Myeloproliferative Neoplasms, Colorectal Cancer, Childhood Colorectal Cancer, Childhood Craniopharyngioma, Cutaneous T-Cell Lymphoma, Ductal Carcinoma In Situ (DCIS), Embryonal Tumors, Endometrial Cancer (Uterine Cancer), Childhood Ependymoma, Esophageal Cancer, Childhood Esophageal Cancer, Esthesio neuroblastoma, Ewing Sarcoma (Bone Cancer), Childhood Extracranial Germ Cell Tumor, Extragonadal Germ Cell Tumor, Eye Cancer, Childhood Intraocular Melanoma, Intraocular Melanoma, Retinoblastoma, Fallopian Tube Cancer, Fibrous Histiocytoma of Bone (Malignant, and Osteosarcoma), Gallbladder Cancer, Gastric,(Stomach) Cancer, Childhood Gastric (Stomach) Cancer, Gastrointestinal Carcinoid Tumor, Gastrointestinal Stromal Tumors (GIST) (Soft Tissue Sarcoma), Childhood Gastrointestinal Stromal Tumors, Germ Cell Tumors, Childhood Central Nervous System Germ Cell Tumors (Brain Cancer), Childhood Extracranial Germ Cell Tumors, Extragonadal Germ Cell Tumors, Ovarian Germ Cell Tumors, Testicular Cancer, Gestational Trophoblastic Disease, Hairy Cell Leukemia, Head and Neck Cancer, Childhood Head and Neck Cancers, Childhood Heart Tumors, Hepatocellular (Liver) Cancer, Langerhans Cell Histiocytosis, Hodgkin Lymphoma, Hypopharyngeal Cancer (Head and Neck Cancer), Intraocular Melanoma, Childhood Intraocular Melanoma, Islet Cell Tumors, Pancreatic Neuroendocrine Tumors, Kaposi Sarcoma (Soft Tissue Sarcoma), Kidney (Renal Cell) Cancer, Langerhans Cell Histiocytosis, Laryngeal Cancer (Head and Neck Cancer), Childhood Laryngeal Cancer, Papillomatosis, Leukemia, Lip and Oral Cavity Cancer (Head and Neck Cancer), Liver Cancer, Lung Cancer (Non-Small Cell and Small Cell), Childhood Lung Cancer, Male Breast Cancer, Malignant Fibrous Histiocytoma of Bone and Osteosarcoma, Melanoma, Childhood Melanoma, Intraocular (Eye)Melanoma, Childhood Intraocular Melanoma, Merkel Cell Carcinoma (Skin Cancer), Malignant Mesothelioma, Childhood Mesothelioma , Metastatic Squamous Neck Cancer with Occult Primary (Head and Neck Cancer), Midline Tract Carcinoma Involving NUT Gene, Mouth Cancer (Head and Neck Cancer), Multiple Endocrine Neoplasia Syndromes, Multiple Myeloma/Plasma Cell Neoplasms, Mycosis Fungoides (Lymphoma), Myelodysplasia Syndromes, Myelodysplastic/Myeloproliferative Neoplasms, Chronic Myeloproliferative Neoplasms, Nasal Cavity and Paranasal Sinus Cancer (Head and Neck Cancer), Nasopharyngeal Cancer (Head and Neck Cancer), Childhood Nasopharyngeal Cancer, Neuroblastoma, Non-Small Cell Lung Cancer, Oral Cancer, Lip and Oral Cavity Cancer and Oropharyngeal Cancer (Head and Neck Cancer), Childhood Oral Cavity Cancer, Osteosarcoma and Malignant Fibrous Histiocytoma of Bone, Ovarian Cancer, Childhood Ovarian Cancer, Pancreatic Cancer, Childhood Pancreatic Cancer, Pancreatic Neuroendocrine Tumors (Islet Cell Tumors), Papillomatosis, Paraganglioma, Childhood Paraganglioma, Paranasal Sinus and Nasal Cavity Cancer (Head and Neck Cancer), Parathyroid Cancer, Penile Cancer, Pharyngeal Cancer (Head and Neck Cancer), Pheochromocytoma, Childhood Pheochromocytoma, Pituitary Tumor, Plasma Cell Neoplasm/Multiple Myeloma, Pleuropulmonary Blastoma, Pregnancy and Breast Cancer, Primary Central Nervous System (CNS) Lymphoma, Primary Peritoneal Cancer, Prostate Cancer, Rectal Cancer, Recurrent Cancer, Renal Cell (Kidney) Cancer, Retinoblastoma, Childhood Rhabdomyosarcoma (Soft Tissue Sarcoma), Salivary Gland Cancer (Head and Neck Cancer), Childhood Salivary Gland Tumors, Sarcoma, Childhood Rhabdomyosarcoma (Soft Tissue Sarcoma), Childhood Vascular Tumors (Soft Tissue Sarcoma), Ewing Sarcoma (Bone Cancer), Kaposi Sarcoma (Soft Tissue Sarcoma), Osteosarcoma (Bone Cancer), Uterine Sarcoma, Sezary Syndrome (Lymphoma), Skin Cancer, Childhood Skin Cancer, Small Cell Lung Cancer, Small Intestine Cancer, Soft Tissue Sarcoma, Squamous Cell Carcinoma of the Skin, Squamous Neck Cancer with Occult Primary, Metastatic (Head and Neck Cancer), Stomach (Gastric) Cancer, Childhood Stomach (Gastric) Cancer, Cutaneous, Testicular Cancer, Childhood Testicular Cancer, Throat Cancer (Head and Neck Cancer), Nasopharyngeal Cancer, Oropharyngeal Cancer, Hypopharyngeal Cancer, Thymoma and Thymic Carcinoma, Thyroid Cancer, Childhood Thyroid Tumors, Transitional Cell Cancer of the Renal Pelvis and Ureter (Kidney (Renal Cell) Cancer), Carcinoma of Unknown Primary, Childhood Cancer of Unknown Primary, Unusual Cancers of Childhood, Ureter and Renal Pelvis, Transitional Cell Cancer (Kidney (Renal Cell) Cancer, Urethral Cancer, Endometrial Uterine Cancer, Uterine Sarcoma, Vaginal Cancer, Childhood Vaginal Cancer, Vascular Tumors (Soft Tissue Sarcoma), Vulvar Cancer, and Wilms Tumor or a combination of one of more of these cancers.

The liquid or non-solid cancer comprises hematologic cancers that are malignancies that affect the blood, bone marrow, lymphatic system, and related tissues. The hematologic cancer will be selected from the non-limiting group comprising leukemias, such as Acute Lymphoblastic Leukemia (ALL), Acute Myeloid Leukemia (AML), Chronic Lymphocytic Leukemia (CLL), and Chronic Myeloid Leukemia (CML); lymphomas, including Hodgkin Lymphoma (HL) and Non-Hodgkin Lymphoma (NHL) subtypes like Diffuse Large B-Cell Lymphoma (DLBCL), Follicular Lymphoma, Mantle Cell Lymphoma, Burkitt Lymphoma, Mucosa-Associated Lymphoid Tissue (MALT) Lymphoma, and Peripheral T-cell Lymphomas; myeloproliferative neoplasms like Chronic Myelogenous Leukemia (CML), Polycythemia Vera (PV), Essential Thrombocythemia (ET), Primary Myelofibrosis (PMF), and Chronic Eosinophilic Leukemia (CEL); myelodysplastic syndromes (MDS) such as Refractory Anemia and Chronic Myelomonocytic Leukemia (CMML); plasma cell disorders, including Multiple Myeloma, Waldenström Macroglobulinemia, Monoclonal Gammopathy of Undetermined Significance (MGUS), and Amyloidosis; and other hematologic malignancies like Histiocytic Sarcoma, Hemophagocytic Lymphohistiocytosis (HLH), and Adult T-cell Leukemia/Lymphoma (ATLL), or a combination of two or more of these cancers.

In one aspect of the invention, the disease is a cardiovascular disease. Non-limiting examples of cardiovascular diseases are selected from the group comprising hypertension, ischaemia, reperfusion injury, stroke including ischemic stroke, myocardial infarction including recurrent myocardial infarction, congestive heart failure, embolism, abdominal aortic aneurism, cardia fibrosis, and pericarditis including Dressler's syndrome or a combination of two or more of them.

In one aspect of the invention, the disease is a neurodegenerative disease affecting the central nervous system (CNS) or the peripheral nervous system (PNS).

Non-limiting examples of neurodegenerative diseases affecting the central nervous system diseases are selected from the group comprising Parkinson's disease, Friedreich ataxia, multiple sclerosis, Alzheimer's disease, dementia, motor neuron disease, Huntington's disease, cerebral malaria, brain injury from pneumococcal meningitis, intracranial aneurysms, and traumatic brain injury or a combination of two or more of them.

Non-limiting examples of neurodegenerative diseases affecting the peripheral nervous system diseases are selected from the group comprising a demyelinating and/or dysmyelinating PNS disease or associated disorder.

In one aspect of the invention, the disease is an inflammatory disease. Non-limiting examples of inflammatory diseases are selected from the group comprising anaphylaxis, septic shock, septic arthritis, rheumatoid arthritis, psoriatic arthritis, asthma, delayed type hypersensitivity, dermatitis, diabetes mellitus, juvenile onset diabetes, graft rejection, inflammatory bowel diseases, Crohn's disease, ulcerative colitis, enteritis, interstitial cystitis, multiple sclerosis, myasthemia gravis, Grave's disease, Hashimoto's thyroiditis, pneumonitis, nephritis, pneumonitis, chronic obstructive pulmonary disease, chronic bronchitis, chronic bronchitis rhinitis, spondyloarthropathies, scleroderma, and systemic lupus erythematosus, chronic hepatitis and psoriasis, or a combination of two or more of them.

The terms "KRAB-containing zinc finger proteins", "KZFP", "KRAB-ZnF", and "KRAB-ZFP" are used interchangeably herein to refer to KRAB-containing zinc finger proteins (KZFPs) that constitute a large family of transcription factors notably involved in controlling the expression of transposable element-embedded regulator sequences (TEeRS) (de Tribolet-Hardy et al., 2023; Imbeault et al., 2017). KZFPs and their genomic targets contribute to regulating many biological events, from early embryogenesis to brain development, and from immune responses to liver metabolism.

As used herein, the term "variant" of one or more nucleic acid sequence or polypeptide sequence of the invention refers to biologically active derivatives of said respective sequences. In general, the term "variant" refers to molecules having a native sequence and structure with one or more additions, substitutions (generally conservative in nature) and/or deletions (e.g. splice variants), relative to the native molecule, so long as the modifications do not destroy biological activity and which are "substantially homologous" to the reference molecule or sequence. In general, the sequences of such variants are functionally, i.e. biologically active variants and will have a high degree of sequence homology to the reference sequence, e.g., sequence homology of more than 50%, generally more than 60%-70%, even more particularly 80%-85% or more, such as at least 90% or 95%, 96%, 97% or more homology to the reference sequence, when the two sequences are aligned.

As used herein, a "fragment" of one or more nucleic acid sequence or polypeptide sequence of the invention refers to a sequence containing less nucleotides or amino acids in length than the respective sequences of the invention while retaining the biological activity described herein. Preferably, this fragment contains, at least 10%, 20%, 30%, 40%, 50%, 60%, 70%, 80%, or 90% of the entire length of the reference nucleic acid sequence or polypeptide sequence.

While focusing on the role of KZFP-mediated regulatory circuits in controlling specific gene networks across a vast array of biological processes, the Inventors surprisingly discovered the role of ZNF354A in the defence pathway to Reactive oxygen species (ROS)-triggered lipid damage.

ZNF354A is a member of the largest family of mammalian transcriptional modulators, the Krüppel-associated box (KRAB) domain zinc finger protein (KZFP). ZNF354A is part of a complex containing the global repressor KAP1 (TRIM28), the histone methyltransferase SETDB1, and the transcriptional activator ATF2 (Watson et al., 2017). Under homeostatic conditions, ZNF354A represses transcriptional programs involved in antioxidant defenses and innate immune responses. Accumulation of lipid peroxides leads to the disassembly of the repressive complex, and the release of ZNF354A from specific DNA loci, allowing activation of the lipid damage response pathway. While highlighting the underexplored dynamics of epigenetic repressive complexes, the Inventors revealed how cells genetically manage oxidative lipid damage responses and identified potential targets for therapeutic intervention in a variety of conditions such as cancers, cardiovascular diseases, neurodegenerative diseases and inflammatory diseases.

This mechanism can be therapeutically exploited to treat any disease or condition linked to oxidative stress, lipid peroxidation and ferroptosis (Stockwell et al., 2017; Yang et al., 2014; Zheng and Conrad, 2025)
1- In inflammatory, metabolic, cardiovascular and degenerative conditions, depleting or blocking ZNF354A can mitigate oxidative stress, protect cells from ROS-induced damage, and diminish inflammation.
2- In immunotherapies, depleting or blocking ZNF354A can enhance the persistence and cytotoxic ability of engineered immune cells (e.g. CAR-T cells). T cells indeed tend to dye, through so-called exhaustion, which is thought to be due to oxidative stress. Silencing or blocking ZNF354A would pre-trigger the LORD pathway, and thereby render cells more resilient to oxidative damage.
3- In cancer, activating or increasing the production of ZNF354A can sensitize cancer cells to oxidative stress and ferroptosis, increasing for instance their susceptibility to radio-chemotherapies.

The present invention thus provides an agent modulating the expression and/or activity of i) a KRAB-containing zinc finger protein 354A (ZNF354A), ii) an mRNA encoding said ZNF354A, and/or iii) the ZNF354A gene.

Preferably, such an agent is for use in the treatment and/or prevention of a disease. More preferably, the disease is a disease or condition linked to oxidative stress, lipid peroxidation and ferroptosis. Preferably, the disease is linked, directly or indirectly, to the expression of ZNF354A. More preferably, the disease is selected from the group comprising cancer, cardiovascular disease, neurodegenerative disease and inflammatory disease, or a combination of two or more of these diseases.

Where the disease to be treated is selected from the group comprising a cardiovascular disease, a neurodegenerative disease and an inflammatory disease, or a combination of two or more of these diseases, the agent will preferably inhibit the expression and/or activity of
i) the ZNF354A protein,
ii) an mRNA encoding the ZNF354A, and/or
iii) the ZNF354A gene.

Preferably, the agent will be selected from the group comprising a nucleic acid, a chemical compound, a peptide or analog thereof, an antibody or an antigen-binding fragment thereof, and an antibody mimetic, or a combination of two or more thereof.

In one aspect, the agent inhibits the translation of an mRNA encoding ZNF354A.

In another aspect, the agent inhibits the transcription of the gene encoding ZNF354A.

In another aspect, said agent modulates the activity of a ZNF354A protein i) by enhancing and/or favoring the degradation of a ZNF354A protein, ii) by preventing the recognition of its targets, iii) by modulating the phosphorylation of a ZNF354A protein, and/or iv) by any other means resulting in a loss of function.

Examples of ZNF354A protein sequences are selected from the group of sequences set forth in SEQ D NO: 2, 4, 6, 8, 10, 12, 14, and 16, or a fragment or variant of any one of these sequences.

In one aspect, the agent of the invention enhances and/or favors the degradation of said ZNF354A protein by inducing the ubiquitination and proteasomal degradation of said ZNF354A protein or degradation by autophagy. Examples of chemical agents inducing selective intracellular proteolysis comprise proteolysis targeting chimera (PROTAC) protein degraders and small-molecule chemical modulators of deubiquitinating enzymes upstream of or on the proteasome. As known in the art, PROTACs are heterobifunctional small molecules composed of two active domains and a linker capable of removing specific unwanted proteins (Békéset al., 2022). Any other targeted protein degradation (TPD) strategies including, but not limited to, molecular glue, Lysosome-Targeting Chimaera (LYTAC), and Antibody-based PROTAC (AbTAC), is also encompassed in the present invention (Zhao et al., 2022).

In other aspects, the agent of the invention is a nucleic acid that inhibits and/or impairs the binding of a ZNF354A protein to a target comprising a binding motif. In one aspect, the target DNA comprises a ZNF354A binding motif selected from the group of sequences set forth in SEQ ID NO: 46, 47, 48, 49, 50, and 51, or a fragment or variant of any one of these sequences.

In other aspects, the agent of the invention is an antibody, or an antigen binding fragment, that inhibits and/or impairs the binding of a ZNF354A protein to a target genomic DNA (e.g. by specifically targeting the zinc-finger motif of the C2H2 zinc finger motif or by specifically binging to an epitope encompassing the Ser-169 phosphorylation site), and/or the binding of the ZNF354A protein to its interacting protein(s) or other molecule(s). In an aspect, the antigen binding fragment is an antigen binding fragment of an antibody.

As used herein, an "antibody" is a protein molecule that reacts with a specific antigenic determinant or epitope and belongs to one or five distinct classes based on structural properties: IgA, IgD, IgE, IgG and IgM. The antibody may be a polyclonal (e.g. a polyclonal serum) or a monoclonal antibody, including but not limited to fully assembled antibody, single chain antibody, antibody fragment, an antibody-drug conjugate and chimeric antibody, humanized antibody as long as these molecules are still biologically active and still bind to at least one peptide or protein of the invention. Preferably the antibody is a monoclonal antibody. Preferably also the monoclonal antibody will be selected from the group comprising IgGl, IgG2, IgG2a, IgG2b, IgG3 and IgG4 or a combination thereof. Most preferably, the monoclonal antibody is selected from the group comprising IgGl, IgG2, IgG2a, and IgG2b, or a combination thereof.

The term "binding fragment," which can be used interchangeably with "antigen-binding fragment," refers herein to an antibody fragment formed from a portion of an antibody comprising one or more CDRs, or any other antibody fragment that specifically binds to an antigen but does not comprise an intact native antibody structure. Examples of antigen-binding fragment include, without limitation, a diabody, a Fab, a Fab', a F(ab')2, a F(ab)c, an Fv fragment, a disulfide stabilized Fv fragment (dsFv), a (dsFv)2, a bispecific dsFv (dsFv-dsFv'), a disulfide stabilized diabody (ds diabody), a triabody, a tetrabody, a single-chain antibody molecule (scFv), an scFv dimer, a multispecific antibody, a camelized single domain antibody, a nanobody, a minibody, a domain antibody, a bivalent domain antibody, a IgNAR, a V-NAR, and a hcIgG. Typically, binding fragments compete with the intact antibody from which they were derived for specific binding.. Binding fragments can be produced by recombinant DNA techniques, or by enzymatic or chemical separation of intact immunoglobulins.

In some aspects, the binding fragment refers to a peptide aptamers consisting of a conformationally constrained antibody variable region displayed by a platform protein, such as *E. coli* Thioredoxin A that are selected from combinatorial libraries by two hybrid methods.

In other aspects, the agent of the invention is dephosphorylating agent that inhibits and/or prevents the phosphorylation of the ZNF354A protein as shown herein. Non limiting examples are selected from the group comprising p38, JNK and ATM kinases.

Where the agent is a nucleic acid, said nucleic acid will be selected from the group comprising a nucleic acid encoding an siRNA, an miRNA, a piRNA, an hnRNA, an snRNA, a gRNA, a CRISPR-based loss-of-function system, an esiRNA, an shRNA, an aptamer and an antisense oligonucleotide (ASO), or a combination of two or more thereof.

The terms "siRNA" and "short interfering RNA" are interchangeable and refer to single-stranded or double-stranded RNA molecules that are capable of inducing RNA interference. siRNA molecules typically have a duplex region that is between 16 and 30, between 17 and 28, between 20 and 25, between 22 and 25 base pairs in length.

In one aspect, the siRNA may be 16, 17, 18, 19, 20, 21, 22, 23, 24, 25, 26, 27, 28, 29, or 30 nucleotides in length or base pairs in length.

Double stranded siRNA is usually derived from longer double stranded RNA molecules (dsRNA). The long dsRNA is cleaved by an endo-ribonuclease (called Dicer) to form double stranded siRNA. In a nucleoprotein complex (called RISC), the double stranded siRNA is unwound to form single stranded siRNA. RNA interference often works via binding of an siRNA molecule to an mRNA molecule having a complementary sequence, resulting in degradation of the mRNA. RNA interference is also possible by binding of an siRNA molecule to an intronic sequence of a pre-mRNA (an immature, non-spliced mRNA) within the nucleus of a cell, resulting in degradation of the pre-mRNA.

In certain aspects, the siRNA is at least 80% homologous to at least 2 to 30 contiguous nucleotides of the ZNF354A mRNA sequence as set forth in SEQ ID NO: 1, SEQ ID NO: 3, SEQ ID NO: 5, SEQ ID NO: 7, SEQ ID NO: 9, SEQ ID NO: 11, SEQ ID NO: 13, and a SEQ ID NO: 15, a fragment or a variant thereof

In certain aspects, the siRNA is characterized by a sequence reverse complementary to a sequence formed by juxtaposing any two consecutive sequences of the sequences defined above.

The term "siRNA" also encompasses single-stranded or double-stranded RNA molecules comprising one or more modified nucleotides i.e. analogues of nucleic acids. Non-limiting examples of modified nucleotides are selected from the group comprising phosphotioates, 2'0-methylphosphothioates, peptide nucleic acids (PNA; N-(2-aminoethyl)-glycine units linked by peptide linkage, with the nucleobase attached to the alpha-carbon of the glycine) or locked nucleic acids (LNA; 2'O, 4'C methylene bridged RNA building blocks). When reference is made herein to a sequence comprising or consisting of a number of nucleotides that are not shown to be modified in that sequence, the reference also encompasses the same nucleotide sequence in which one, several, such as two, three, four, five, six, seven or more, including all, nucleotides are modified by modifications such as 2'-OMe, 2'-F, or have a 3' end or 5' end modification or any other modification.

In one aspect, if the 5'-most nucleotide of the first strand of a nucleic acid is a nucleotide other than A or U, this nucleotide is replaced by an A or U. Preferably, if the 5'-most nucleotide of the first strand is a nucleotide other than a U, this nucleotide is replaced by U, and more preferably by U with a 5' (E)-vinylphosphonate, in the sequence.

In some aspects, the siRNA of the invention is conjugated to a ligand, optionally via a linker. Efficient delivery of oligonucleotides, in particular double-stranded nucleic acids, to cells *in vivo* is important and requires specific targeting and substantial protection from the extracellular environment, preferably serum proteins. One method of achieving specific targeting is to conjugate a ligand to the nucleic acid. In some aspects, the ligand helps in targeting the nucleic acid to a target cell which has a cell surface receptor that binds to and internalises the conjugated ligand. In such aspects, there is a need to conjugate appropriate ligands for the desired receptor molecules in order for the conjugated molecules to be taken up by the target cells by mechanisms such as different receptor-mediated endocytosis pathways or functionally analogous processes. In other aspects, a ligand which can mediate internalization of the nucleic acid into a target cell by mechanisms other than receptor mediated endocytosis may alternatively be conjugated to a nucleic acid of the invention for cell or tissue specific targeting.

When the conjugated nucleic acid has a first strand (antisense strand) and a second strand (sense strand), the ligand can be conjugated to either one of the strands, preferably at the end of a strand, more particularly to the last nucleotide at one end of a strand. The ligand is preferably conjugated to the ribose of a nucleotides, preferably the last of a strand. The ligand can be conjugated to the ribose of a nucleotide, preferably the last on in a strand, via the 2', 3' or 5' carbon of the ribose. Preferred are the 3' or 5' carbon. The ligand is preferably not conjugated to the 5' end of the first strand. The ligand is preferably conjugated to the 5' end of the second strand.

The ligand is preferably conjugated to the last nucleotide of a nucleic acid strand, preferably to the ribose moiety. The ligand can be conjugated to the first strand (the antisense strand) or the second strand (the antisense strand). Preferably, the ligand is conjugated to a nucleotide at the end of one of the strands of the nucleic acid, more preferably the nucleotide at the 3' or 5' end of the second (sense) strand.

Generally, the ligand can comprise a saccharide that is selected to have an affinity for at least one type of receptor on a target cell. In particular, the receptor is on the surface of a mammalian liver cell, for example, the hepatic asialoglycoprotein receptor complex described before (ASGP-R).

Non-limiting examples of a ligand comprise N-acetyl galactosamine (GalNAc), mannose, galactose, glucose, glucosamine and fucose. More preferably at least one N-acetyl galactosamine (GalNAc). For example, the asialoglycoprotein receptor complex (ASGP-R) which is highly abundant on hepatocytes, shows high affinity to GalNAc.

Any suitable linker can be used to conjugate the nucleic acid, such as the siRNA, to the ligand. Non-limiting examples of linkers can be found, e.g. in PCT/EP2022/055455 (WO2022/174852).

The terms "microRNA," "miRNA," and "MiR" are interchangeable and refer to endogenous or artificial non-coding RNAs that are capable of regulating gene expression. It is believed that miRNAs function via RNA interference.

The terms "piRNA" and "Piwi-interacting RNA" are interchangeable and refer to a class of small RNAs involved in gene silencing. PiRNA molecules typically are between about 26 and about 31 nucleotides in length.

Non-limiting examples of antisense oligonucleotides (ASOs) include the GapmeRs. As used herein, a GapmeR (also named Antisense LNA GapmeR) is a chimeric antisense oligonucleotide that contains a central block of deoxynucleotide monomers sufficiently long to induce RNase H cleavage. Usually, the GapmeRs of the invention are directed against one or more mRNA encoding the KZF of the invention at both pre-mRNA and/or mRNA levels. Modified ASOs such as modified GapmeRs are also encompassed in the present invention and comprise, e.g. GapmeRs with fixed chemical modification architectures selected from the group comprising i) a gapmer with five 2'-O-methoxyethyl (MOE) modifications in each flank, and a central gap of 10 unmodified dans (e.g. 5-10-5 MOE design), and ii) a gapmer employing three or four locked nucleic acid (LNA) modifications in each flank (e.g. 3-10-3 or 4-8-4 LNA designs), as well as a combination of two or more thereof.

Aptamers are oligonucleotide sequences with the capacity to recognize virtually any class of target molecules with high affinity and specificity. Such ligands may be isolated through Systematic Evolution of Ligands by EXponential enrichment (SELEX) of a random sequence library. The random sequence library is obtainable by combinatorial chemical synthesis of DNA. In this library, each member is a linear oligomer, eventually chemically modified, of a unique sequence.

The terms "sgRNA" and "guideRNA" are interchangeable and refer to a specific RNA sequence that recognizes the target DNA region of interest and directs the endonuclease there for editing. The gRNA is usually made up of two parts: crispr RNA (crRNA), a 17-20 nucleotide sequence (e.g. 17, 18, 19, or 20 nucleotides) complementary to the target DNA, i.e genomic DNA encoding the KZF of the invention or regulatory region controlling the genomic DNA encoding the KZF of the invention , and a tracr RNA, which serves as a binding scaffold for the Cas nuclease.

Any suitable engineered sgRNA, or crRNA and tracrRNA, can be employed as long as it is effective for recognizing a target DNA of the invention. The design of such sgRNA, or crRNA and tracrRNA, is within the skill of ordinary artisans.

Non-limiting examples of target DNA sequences are selected from the group comprising:
i) one or more sequences within the gene encoding ZNF354A (chromosome 5: 178730659-178711512) as set forth in SEQ ID NO: 17,
ii) one or more sequences within the promoter region controlling the gene encoding ZNF354A (chromosome: 5, 178728826:178731484) as set forth in SEQ ID NO: 32, or
iii) one or more DNA sequences selected from the group of sequences set forth in SEQ ID NO: 46, 47, 48, 49, 50, and 51, or a fragment or variant of any one of these sequences.

In certain aspects, the one or more sequences within the gene encoding ZNF354A are characterized by nucleotides 1-100, 101-200, 201-300, 301-400, 301-400, 401-500, 501-600, 601-700, 701-800, 801-900, 901-1000, 1001-1100, 1101-1200, 1201-1300, 1301-1400, 1501-1600, 1701-1800, 1801-1900, 1901-2000, 2001-2100, 2101-2200, 2201-2300, 2301-2400, 2501-2600, 2701-2800, 2801-2900, 2901-3000, 3001-3100, 3101-3200, 3201-3300, 3301-3400, 3401-3500, 3501-3600, 3601-3700, 3701-3800, 3801-3900, 3901-4000, 4001-4100, 4101-4200, 4201-4300, 4301-4400, 4401-4500, 4501-4600, 4601-4700, 4701-4800, 4801-4900, 4901-5000, 5001-5100, 5101-5200, 5201-5300, 5301-5400, 5401-5500, 5501-5600, 5601-5700, 5701-5800, 5801-5900, 5901-6000, 6001-6100, 6101-6200, 6201-6300, 6301-6400, 6401-6500, 6501-6600, 6601-6700, 6701-6800, 6801-6900, 6901-7000, 7001-7100, 7101-7200, 7201-7300, 7301-7400, 7401-7500, 7501-7600, 7601-7700, 7701-7800, 7801-7900, 7901-8000, 8001-8100, 8101-8200, 8201-8300, 8301-8400, 8401-8500, 8501-8600, 8601-8700, 8701-8800, 8801-8900, 8901-9000, 9001-9100, 9101-9200, 9201-9300, 9301-9400, 9401-9500, 9501-9600, 9601-9700, 9701-9800, 9801-9900, 9901-10000, 10001-10100, 10101-10200, 10201-10300, 10301-10400, 10401-10500, 10501-10600, 10601-10700, 10701-10800, 10801-10900, 10901-11000, 11001-11100, 11101-11200, 11201-11300, 11301-11400, 11401-11500, 11501-11600, 11601-11700, 11701-11800, 11801-11900, 11901-12000, 12001-12100, 12101-12200, 12201-12300, 12301-12400, 12401-12500, 12501-12600, 12601-12700, 12701-12800, 12801-12900, 12901-13000, 13001-13100, 13101-13200, 13201-13300, 13301-13400, 13401-13500, 13501-13600, 13601-13700, 13701-13800, 13801-13900, 13901-14000, 14001-14100, 14101-14200, 14201-14300, 14301-14400, 14401-14500, 14501-14600, 14601-14700, 14701-14800, 14801-14900, 14901-15000, 15001-15100, 15101-15200, 15201-15300, 15301-15400, 15401-15500, 15501-15600, 15601-15700, 15701-15800, 15801-15900, 15901-16000, 16001-16100, 16101-16200, 16201-16300, 16301-16400, 16401-16500, 16501-16600, 16601-16700, 16701-16800, 16801-16900, 16901-17000, 17001-17100, 17101-17200, 17201-17300, 17301-17400, 17401-17500, 17501-17600, 17601-17700, 17701-17800, 17801-17900, 17901-18000, 18001-18100, 18101-18200, 18201-18300, 18301-18400, 18401-18500, 18501-18600, 18601-18700, 18701-18800, 18801-18900, 18901-19000, 19001 to 19140 of SEQ ID NO: 17.

In certain aspects, the one or more sequences within the promoter region controlling the gene encoding ZNF354A are characterized by nucleotides 1-100, 101-200, 201-300, 301-400, 401-500, 501-600, 601-700, 701-800, 801-900, 901-1000, 1001-1100, 1101-1200, 1201-1300, 1301-1400, 1401-1500, 1501-1600, 1601-1700, 1701-1800, 1801-1900, 1901-2000, 2001-2100, 2101-2200, 2201-2300, 2301-2400, 2401-2500, 2501-2600, and 2601 to 2650 of SEQ ID NO: 32. In one aspect, the one or more sequences within the promoter region controlling the gene encoding ZNF354A are characterized by nucleotides 1-770, 770-1035, 1035-1825, 1825-2345, 2345-2510, and 2510-2655, of SEQ ID NO: 32.

In one aspect, the one or more sequences within the promoter region controlling the gene encoding ZNF354A is a transcription factor binding site characterized by nucleotides 1260-1283, 1657-1674, 1693-1709, 1896-1913, 1977-1994, and/or 2061-2067, of SEQ ID NO: 32, a fragment or a variant thereof.

Examples of siRNA sequences targeting a sequence within an mRNA encoding the KZF of the invention are selected from the non-limiting group of sequences set forth in SEQ ID NO: 18, 19, 20, 21, 22, 23, 24, 25, 26, 27, 28, 29, 30 and 31, a fragment or a variant thereof.

The terms "shRNA" as used herein refers to a nucleic acid molecule comprising at least two complementary portions hybridized or capable of specifically hybridizing to form a duplex structure sufficiently long to mediate RNAi (typically between about 15 to about 29 nucleotides in length), and at least one single-stranded portion, typically between approximately 1 and about 10 nucleotides in length that forms a loop connecting the ends of the two sequences that form the duplex. Exemplary shRNAs of the invention are designed to be uniquely and selectively recognizing a target sequence within an mRNA encoding the KZF of the invention. Selecting a suitable shRNA is well within the competences of one of ordinary skill in the art using routine experimentation, several commercial and noncommercial web sites available for shRNA design as well as the information provided herein.

Examples of shRNA sequences are selected from the non-limiting group of sequences set forth in SEQ ID NO: 33, 34, 35, 36, 37, 38, 39, 40, 41, 42, 43, 44, and 45, a fragment or a variant thereof.

CRISPR (clustered regularly interspaced short palindromic repeats) (CRISPR-associated protein), a Class 2 type II CRISPR system, can be easily programmed to introduce DNA double-strand breaking (DSB) or single-strand breaking (SSB) at the desired target sequences. Usually, the CRISPR is a CRISPR based gain/loss-of-function system comprising at least one single guide RNA (sgRNA), or crRNA and tracrRNA, a protospacer adjacent motif (PAM) and a structure-guided endonuclease such as an RNA-guided endonuclease.

Any suitable naturally occurring, or engineered, RNA-guided endonuclease can be employed as long as it is effective for binding a target DNA and it may be selected from the non-limiting group comprising Cas9, Cas 12, Cpfl, and FEN-1. Preferably, the RNA-guided endonuclease is Cas9.

One of ordinary skill in the art, using routine experimentation, several available commercial and noncommercial web sites for determining said target sequences as well as relevant information provided herein is able to determine a target sequence within an mRNA encoding ZNF354A.

The present invention also contemplates a guide ribonucleic acid (sgRNA or gRNA) targeting i) a genomic site of the ZNF354A gene as set forth in SEQ ID NO: 17, a fragment or a variant thereof or ii) a regulatory sequence of said ZNF354A gene as set forth in SEQ ID NO: 32, a fragment or a variant thereof.

Examples of sgRNA sequences are selected from the non-limiting group of sequences set forth in SEQ ID NO: 52, 53, 54 and 55, a fragment or a variant thereof.

Where the disease to be treated is cancer as described herein, the agent will preferably enhance the activity of a ZNF354A protein i) by inhibiting the degradation of a ZNF354A protein, ii) by increasing the expression of a ZNF354A protein, iii) by modulating the phosphorylation of a ZNF3 54A protein, and/or iv) by any other means resulting in a gain of function.

Although CRISPR/Cas systems have been commonly used to generate loss-of-function mutations, they have also been repurposed as a programmable platform for gain-of-function analysis by transcriptional activation of target genes. CRISPR/Cas systems, based on a deactivated Cas (dCas) protein fused with transcriptional activators are within the scope of the present invention.

In oen aspect, CRISPR comprises at least one single guide RNA (sgRNA), or crRNA and tracrRNA, and a structure-guided endonuclease such as an RNA-guided endonuclease.

Any suitable naturally occurring, or engineered, RNA-guided endonuclease can be employed as long as it is effective for binding a target DNA and it may be selected from the non-limiting group comprising Cas9, Cpfl, and FEN-1. Preferably, the RNA-guided endonuclease is Cas9.

Within the context of this disclosure, the Cas9 endonuclease is preferably a modified Cas9 endonuclease such as, e.g. an enzymatically dead Cas9. Specifically, both RuvC- and HNH-nuclease domains can be rendered inactive by point mutations (e.g. D10A and H840A in SpCas9), resulting in a nuclease dead Cas9 molecule that cannot cleave target DNA. However, the dead Cas9 molecule retains the ability to bind to target DNA based on the sgRNA targeting sequence, which sgRNA sequence is comprised in CRISPR-based gain -of-function system.

In one aspect, the enzymatically dead Cas9 is tagged with one or more transcriptional repressor or one or more transcriptional activator (see Didovyk et al. 2016, which is incorporated herein by reference).

In another aspect, the enzymatically dead Cas9 is tagged with one or more epitope that is/are recognized by one or more antibody-activator effector. This enzymatically tagged dead Cas9 can then target one or more sequences regulatory sequence resulting in robust transcription activation of downstream target gene encoding ZNF354A of the invention.

Usually and within the context of the invention, the regulatory sequence is a promoter or enhancer region which is either a cis-or a trans-acting regulatory sequence.

In one aspect, the enzymatically tagged dead Cas9 targets one or more sequences within the promoter region controlling the gene encoding ZNF354A that are characterized by nucleotides 1-100, 101-200, 201-300, 301-400, 401-500, 501-600, 601-700, 701-800, 801-900, 901-1000, 1001-1100, 1101-1200, 1201-1300, 1301-1400, 1401-1500, 1501-1600, 1601-1700, 1701-1800, 1801-1900, 1901-2000, 2001-2100, 2101-2200, 2201-2300, 2301-2400, 2401-2500, 2501-2600, and 2601 to 2650 of SEQ ID NO: 32.

The present invention also contemplates a gene delivery vector, preferably in the form of a plasmid or a vector, that comprises one or more nucleic acid(s) encoding an agent of the invention. Preferably, said agent is a nucleic acid selected from the group comprising an siRNA, miRNA, piRNA, hnRNA, snRNA, gRNA, CRISPR-based loss-of-function system, CRISPR-based gain-of-function system, esiRNA, shRNA, and antisense oligonucleotide (e.g. Gapmer antisens), or combination of one or more thereof.

Suitable vectors include derivatives of SV40 and known bacterial plasmids, e. g., E. coli plasmids col El, pCRl, pBR322, pMB9 and their derivatives, plasmids such as RP4; phage DNAs, e. g., the numerous derivatives of phage X, e. g., NM989, and other phage DNA, e. g., Ml 3 and filamentous single stranded phage DNA; yeast plasmids such as the 2µ plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like.

Various viral vectors are used for delivering nucleic acid to cells *in vitro* or *in vivo.* Non-limiting examples are vectors based on Herpes Viruses, Pox- viruses, Adeno-associated virus, Lentivirus, and others. In principle, all of them are suited to deliver an expression cassette comprising an expressible nucleic acid molecule that codes for an agent of the invention. In a preferred aspect, said viral vector is an adenoviral vector, preferably a replication competent adenovirus.

The present invention further contemplates a host cell, or population of host cells, comprising, or modified by the introduction of, i) a gene delivery vector of the invention (e.g. a plasmid or vector) or ii) one or more nucleic acids encoding the siRNA, miRNA, piRNA, hnRNA, snRNA, gRNA, CRISPR-based loss-of-function system, CRISPR-based gain-of-function system, esiRNA, shRNA, and antisense oligonucleotide, or combination of two or more thereof, as described herein. Host cells can be either eukaryotic or prokaryotic cells. In one aspect, the cell is a mammalian cell, whether an autologous or an allogeneic cell.

The present invention further contemplates an agent modulating the expression and/or activity of i) a KRAB-containing zinc finger protein ZNF354A (ZNF354A), ii) an mRNA encoding said ZNF354A, and/or iii) the ZNF354A gene.

Also contemplated in the present invention is a pharmaceutical composition comprising a therapeutically effective amount of an agent modulating the expression and/or activity of
i) a KRAB-containing zinc finger protein ZNF354A (ZNF354A), an mRNA encoding said ZNF354A, and/or the ZNF354A gene, or
ii) a plasmid or a vector of the invention,
iii) a siRNA of the invention,
iv) a gRNA of the invention, or
iii) a host cell of the invention,
and a pharmaceutically acceptable carrier or diluent.

Preferably, the pharmaceutical composition is for use in the treatment and/or prevention of a disease linked to the expression of ZNF354A. In one aspect, the disease linked to the expression of ZNF354A is selected from the group comprising cancer, cardiovascular disease, neurodegenerative disease and inflammatory disease.

The term "therapeutically effective amount" as used herein means an amount of an agent of the invention, ii) a gene delivery vector of the invention, or iii) a host cell of the invention, high enough to significantly positively modify the symptoms and/or condition to be treated, but low enough to avoid serious side effects (at a reasonable risk/benefit ratio), within the scope of sound medical judgment. The therapeutically effective amount of an agent of the invention, ii) a gene delivery vector of the invention, or iii) a host cell of the invention is selected in accordance with a variety of factors including type, species, age, weight, sex and medical condition of the patient; the severity of the condition to be treated; the route of administration; the renal and hepatic function of the patient.

"Pharmaceutically acceptable carrier and/or diluent" means a carrier and/or diluent that is useful in preparing a pharmaceutical composition that is generally safe, non-toxic, and desirable, and includes carriers or diluents that are acceptable for human pharmaceutical use. Such pharmaceutically acceptable carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions.

Pharmaceutically acceptable excipients include starch, glucose, lactose, sucrose, sodium stearate, glycerol monostearate, talc, sodium chloride, dried skim milk, glycerol, propylene glycol, water, ethanol and the like.

The pharmaceutical compositions may further contain one or more pharmaceutically acceptable salts such as, for example, a mineral acid salt such as a hydrochloride, a hydrobromide, a phosphate, a sulfate, etc.; and the salts of organic acids such as acetates, propionates, malonates, benzoates, etc. Additionally, auxiliary substances, such as wetting or emulsifying agents, pH buffering substances, gels or gelling materials, flavorings, colorants, microspheres, polymers, suspension agents, etc. may also be present herein. In addition, one or more other conventional pharmaceutical ingredients, such as preservatives, humectants, suspending agents, surfactants, antioxidants, anticaking agents, fillers, chelating agents, coating agents, chemical stabilizers, etc. may also be present, especially if the dosage form is a reconstitutable form. Suitable exemplary ingredients include macrocrystalline cellulose, carboxymethyf cellulose sodium, polysorbate 80, phenyletbyl alcohol, chiorobutanol, potassium sorbate, sorbic acid, sulfur dioxide, propyl gallate, the parabens, ethyl vanillin, glycerin, phenol, parachlorophenol, gelatin, albumin and a combination thereof. A thorough discussion of pharmaceutically acceptable excipients is available in REMINGTON'S PHARMACEUTICAL SCIENCES (Mack Pub. Co., N.J. 1991) which is incorporated by reference herein.

The pharmaceutical composition can also further comprise or provide, one or more additional therapy. Where the disease is, e.g., cancer, at least one additional anticancer agent or therapy is selected from the group comprising radiotherapy, chemotherapy, immunotherapy and hormone therapy, or a combination of one of more thereof.

The pharmaceutical composition comprising a therapeutically effective amount of a host cell and a pharmaceutically acceptable carrier and/or diluent can be administered to the subject in need thereof by any method and route known in the art and described herein.

Also contemplated in the present invention is a i) ZNF354A protein as set forth in SEQ ID NO: 2, SEQ ID NO: 4, SEQ ID NO: 6, SEQ ID NO: 8, SEQ ID NO: 10, SEQ ID NO: 12, SEQ ID NO: 14, and SEQ ID NO: 16, a fragment or a variant thereof, or ii) a nucleic acid encoding said ZNF354A protein as set forth in SEQ ID NO: 1, SEQ ID NO: 3, SEQ ID NO: 5, SEQ ID NO: 7, SEQ ID NO: 9, SEQ ID NO: 11, SEQ ID NO: 13, and SEQ ID NO: 15, a fragment or a variant thereof, for use in the treatment and/or prevention of cancer.

Also contemplated in the present invention is an agent modulating the expression and/or activity of i) a KRAB-containing zinc finger protein ZNF354A (ZNF354A), ii) an mRNA encoding said ZNF354A, and/or iii) the ZNF354A gene, for modulating cellular responses to oxidative stress.

The present invention further contemplates methods of treating and/or preventing a disease linked to the expression of ZNF354A in a subject in need thereof.

In one aspect of the invention, the method of treating and/or preventing a disease linked to the expression of ZNF354A in a subject in need thereof, comprises administering a therapeutically effective amount of an agent or agent for use of the invention, ii) a gene delivery vector of the invention, iii) a host cell of the invention, or iv) a pharmaceutical composition of the invention.

In one aspect of the invention, the method of treating and/or preventing a disease linked to the expression of ZNF354A in a subject in need thereof, comprises modifying a host cell, and reintroducing the host cell (e.g. single cell or population of cells) into the subject in need thereof.

In one aspect of the invention, the method of treating and/or preventing a cancer linked to the expression of ZNF354A in a subject in need thereof, comprises administering a therapeutically effective amount of a nucleic acid encoding a ZNF354A of the invention, ii) a gene delivery vector comprising a nucleic acid encoding a ZNF354A of the invention, iii) a host cell comprising a nucleic acid encoding a ZNF354A of the invention, or iv) a pharmaceutical composition comprising a nucleic acid encoding a ZNF354A of the invention.

In one aspect of the invention, the method of treating and/or preventing a disease linked to the expression of ZNF354A in a subject in need thereof, comprises modifying a host cell, and reintroducing the host cell (e.g. single cell or population of cells) into the subject in need thereof.

Preferably, a biopsy or other tissue or biological fluid sample comprising the single cell or the population of cells may be necessary. Cells such as fibroblast cells or stem cells that can be generated directly from adult cells, such as iPSCs, are particularly preferred in this regard.

A gene delivery vector (e.g. plasmid or vector) comprising a nucleic acid encoding an agent or agent for use of the invention or at least one acid nucleic of the invention can be introduced to host cell via one or more methods known in the art. These one or more methods include, without limitation, microinjection, electroporation, calcium phosphate-mediated transfection, cationic transfection, liposome transfection, dendrimer transfection, heat shock transfection, nucleofection transfection, magnetofection, lipofection, optical transfection, proprietary agent-enhanced uptake of nucleic acids, and delivery via liposomes, immunoliposomes, virosomes, or artificial virions.

The host cell (e.g. single cell or population of cells) of the invention is then reintroduced into the subject in need thereof by any route of administration and/or delivery methods known in the art, as described herein.

The invention also contemplates kits for performing a method according to the invention or for the treatment and/or prevention of a disease of the invention. In one aspect of the invention, the kit comprises a pharmaceutical composition comprising an agent of the invention.

In a further aspect, the invention contemplates a kit for the treatment and/or prevention of a disease of the invention comprising a host cell of the invention.

The kits of the invention may also comprise a container and a label or package insert on or associated with the container. Suitable containers include, for example, bottles, vials, syringes, etc. The containers may be formed from a variety of materials such as glass or plastic. The container holds a composition which is effective for treating the disease of disorder of the invention and may have a sterile access port (for example the container may be an intravenous solution bag or a vial having a stopper pierceable by a hypodermic injection needle). Alternatively, or additionally, the kits may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The label or package insert may comprise instructions for use thereof. Instructions included may be affixed to packaging material or may be included as a package insert. While the instructions are typically written or printed materials they are not limited to such. Any medium capable of storing such instructions and communicating them to an end user is contemplated by this disclosure.

Further contemplated in the present invention are nucleic acids encoding an siRNA, an shRNA, an snRNA, a siRNA capable of interfering the expression of short hairpin (sh), a piRNA, or a nucleic acid including an antisense oligonucleotide (e.g. ASOs, modified ASOs such as GapmeRs, ...) of the invention.

Further contemplated in the present invention is a method to boost the redox resistance of immunotherapy effectors (such as CAR-T cells, CAR-NK cells, ...), the method comprising the step of contacting the immunotherapy effectors with one or more agents of the invention to silence the expression of ZNF354A in these effectors for example in order to extend their life span, knowing that T cells exhaustion following activation is in part due to oxidative damage.

Further contemplated in the present invention is a method of detecting a disease linked to an over-activation of antioxidative pathways in a subject, the method comprising:
i) detecting the level of phosphorylation of ZNF354A at serine 169 in a biological sample of said subject,
ii) comparing the level of phosphorylation of ZNF354A at serine 169 in said subject with a standard control,
wherein a high level of phosphorylation of ZNF354A at serine 169 in said subject relative to said standard control indicates said subject suffers from a disease linked to an over-activation of antioxidative pathways.

In one aspect, the method of detecting a disease linked to an over-activation of antioxidative pathways in a subject comprises adding an antibody or antigen binding fragment thereof to said sample under conditions which allow for binding of said antibody, or antigen binding fragment to ZNF354A and detecting the amount of antibody-bound in said sample, wherein the antibody or antigen binding fragment thereof specifically targets and binds to an epitope encompassing the Ser-169 phosphorylation site.

Usually, the disease is linked to an over-activation of antioxidative pathways is a disease selected from the group comprising cancer, in particular cancer resistance to cell death (chemotherapy), chronic tissue inflammation/damage (e.g. neuron, cardiovascular and muscle diseases) and acute injury (e.g. ischemia, blood brain barrier disfunction, muscle rupture), or a combination thereof.

The induction of ferroptosis, a lipid peroxide-induced cell death, is of major interest in cancer research to kill tumor cells. To investigate the relevance of the LORD pathway in predicting and controlling the sensitivity of cancer lines to ferroptosis, the Inventors probed for the phosphorylation of ZNF354A. Only the cells expressing ZDHHC20L, i.e. HEPG2, MCF7 and HCT116 (Fig. 1) were more resistant to either RLS3 or H₂O₂, compared to all other lines (Fig. 5C).Thus, according to one aspect, the method further indicates whether the cancer is sensitivity or resistant to ferroptosis and determine the treatment to administered.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein. Various references are cited throughout this Specification, each of which is incorporated herein by reference in its entirety. The foregoing description will be more fully understood with reference to the following Examples.

### SEQUENCES

- **SEQ ID NO:1**
   **Homo sapiens zinc finger protein 354A (ZNF354A), transcript variant 1, mRNA NCBI Reference Sequence: NM_005649.3**
   >NM_005649.3 Homo sapiens zinc finger protein 354A (ZNF354A), transcript variant 1, mRNA/cDNA 
AGACGCCGUCGGGGCGGAGCCGGGUCGCGAGGCUGCAUCCCGGCCGGCGGUUCCGGAGCCUCGCGGCUGG
GGAGGCGCGGCCCGGGACGCUCGAGCUUAGGGAGAUCUGCCUUCUGGAGACUGCGCCGUCCUCCCGGGAG
AGCCAGAAAGAGGACAUGGCUGCUGGGCAGCGGGAAGCGAGGCCCCAGGUGUCACUGACGUUUGAGGAUG
UGGCUGUGCUGUUUACCCGAGAUGAGUGGAGAAAGCUGGCCCCUUCUCAGAGAAACUUGUACCGGGAUGU
GAUGCUGGAGAACUAUAGGAACCUGGUCUCACUGGGGCUCCCAUUUACCAAACCAAAAGUGAUCUCCCUG
UUGCAGCAAGGAGAAGAUCCCUGGGAGGUGGAGAAAGACGGUUCUGGCGUCUCCCUCUCUAGGAUCGAAGA
GCAGUCAUAAAACCACAAAGUCAACGCAAACACAAGACUCUUCAUUUCAGGGACUGAUACUGAAAAGAUC
CAACAGGAAUGUACCUUGGGAUUUGAAAUUAGAAAAGCCUUACAUAUGAAGGCAGAUUAGAGAAAAAG
CAGGAUAAAAAGGGAAGUUUUCAGAUAGUUUCAGCCACCCACAAAAAAAUCCCCACUAUAGAAAGAAGCC
AUAAAAAUACUGAAUUGAGCCAAAACUUCAGCCAAAGUCAGUGCUUAUUAGGCAACAGAUACUUCCCAG
AGAAAAAACACCACCAAAAUGUGAAAUACAAGGAAACAGCCUCAAACAGAAUUCACAAUUACUUAAUCAA
CCAAAAAUUACAGCAGAUAAACGCUAUAAAUGUAGUCUGUGUGAAAAAACCUUCAUUAACACUUCAUCCC
UUCGUAAACAUGAGAAAAACCAUAGUGGAGAGAAACUAUUUAAGUGUAAAGAAUGUUCAAAAGCCUUUAG
CCAAAGUUCAGCUCUUAUUCAACAUCAAAUAACGCAUACUGGAGAGAAACCCUACAUAUGUAAAGAAUGU
GGGAAAGCCUUUACUCUCAGUACAUCCCUUUAUAAACAUCUAAGAACCCAUACUGUGGAGAAAUCCUACA
GAUGUAAAGAAUGUGGUAAAUCCUUCAGCCGAAGGUCAGGCCUUUUUAUACAUCAAAAAAUUCAUGCUGA
AGAAAACCCUUGUAAGUAUAAUCCGGGUAGGAAGGCAUCUAGUUGCAGCACAUCCCUUUCUGGAUGUCAA
AGAAUUCAUUCUAGAAAGAAGUCCUACUUAUGUAAUGAAUGUGGCAACACCUUUAAGUCUAGCUCAUCCC
UUCGUUAUCAUCAGAGAAUUCACACUGGAGAGAAGCCUUUUAAAUGUAGUGAAUGUGGGAGAGCCUUCAG
CCAGAGUGCCUCUCUUAUUCAACAUGAAAGAAUUCACACCGGAGAAAAGCCCUAUAGAUGCAAUGAAUGU
GGGAAAGGCUUUACUUCUAUUUCACGACUUAAUAGACACCGAAUCAUUCAUACUGGAGAGAAGUUUUAUA
AUUGUAAUGAAUGUGGUAAAGCCUUAAGCUCCCACUCAACACUUAUUAUUCACGAGCGAAUUCAUACUGG
AGAAAAACCAUGUAAAUGUAAAGUAUGUGGAAAAGCCUUCAGACAGAGUUCAGCUCUCAUUCAACAUCAG
AGAAUGCAUACUGGAGAAAGACCCUAUAAAUGUAACGAGUGUGGAAAACAUUCAGGUGUAACUCAUCAC
UUAGUAAUCACCAGAGAAUUCAUACUGGAGAGAAACCAUAUCGAUGUGAGGAAUGUGGAUAUCUUUUGG
CCAAAGUUCAGCUCUUAUUCAGCAUCGAAGGAUUCAUACAGGAGAAAAACCCUUUAAAUGUAAUACAUGU
GGAAAAACUUUUAGACAAAGCUCAUCACGUAUUGCACAUCAGAGAAUUCAUACUGGAGAGAAACCCUAUG
AAUGUAAUACAUGUGGGAAACUUUUCAACCAUAGGUCAUCCCUUACUAAUCAUUAUAAAAUUCAUAUCGA
AGAGGACCCCUAGAAAGUAGAUUUGUAUGUGUGAAAGCCUUAAACCAAAGCUCAUCGAAGAAUACAUCCU
UGAGAGAGAUGUAAUAAAUGUAAUGGAUGUGAAAAAAACUGUAAUAAUUUAGCCCUCAUUAGGUAUUUAA
UUCCAUGGAUAAACCUCAGCUAUAUAAUAGAUAUGAGGAAAGUGUUUGUGCCUGUCAGACACUUAAAAAA
AUAACCUGAGAUGAAGAAUUUACAAUUGAAGACAUUGACUUUAGCCAUUUGUGAAAUGGGUUUGCUUUUU
CCCUUUUCCUACAGACGUAUAUGCUAGAUGUCACGUGAUCAUCAGAAACAGAUAUCCGAGUGGGUGGGGA
GGUGUGCUUUAGAUUUCUCAUUAGAAGACCACCAAACUGGUAAUAUUUUUAUAGCAUUUUAAUAGCUUGA
UCAAAUUGUACCUUUUUAGAGAAAAGGACCAAAAUAAAAGAAAAAUGAAUUAUGAACUACCUCUCAGUCU
CUGGGGUUUGUCCUUUUCCUACCCUGAUGUCAAACUUAUGCAUGGAUUUCAUUAAAAAAGAAAAAGAAAA
- **SEQ ID NO:2**
   **Homo sapiens zinc finger protein 354A (ZNF354A), Translated protein of transcript variant 1** 
MAAGQREARPQVSLTFEDVAVLFTRDEWRKLAPSQRNLYRDVML
ENYRNLVSLGLPFTKPKVISLLQQGEDPWEVEKDGSGVSSLGSKSSHKTTKSTQTQDS
SFQGLILKRSNRNVPWDLKLEKPYIYEGRLEKKQDKKGSFQIVSATHKKIPTIERSHK
 
NTELSQNFSPKSVLIRQQILPREKTPPKCEIQGNSLKQNSQLLNQPKITADKRYKCSL
CEKTFINTSSLRKHEKNHSGEKLFKCKECSKAFSQSSALIQHQITHTGEKPYICKECG
KAFTLSTSLYKHLRTHTVEKSYRCKECGKSFSRRSGLFIHQKIHAEENPCKYNPGRKA
SSCSTSLSGCQRIHSRKKSYLCNECGNTFKSSSSLRYHQRIHTGEKPFKCSECGRAFS
QSASLIQHERIHTGEKPYRCNECGKGFTSISRLNRHRIIHTGEKFYNCNECGKALSSH
STLIIHERIHTGEKPCKCKVCGKAFRQSSALIQHQRMHTGERPYKCNECGKTFRCNSS
LSNHQRIHTGEKPYRCEECGISFGQSSALIQHRRIHTGEKPFKCNTCGKTFRQSSSRI
AHQRIHTGEKPYECNTCGKLFNHRSSLTNHYKIHIEEDP
- **SEQ ID NO:3**
   **Homo sapiens zinc finger protein 354A (ZNF354A), transcript variant 2, mRNA NCBI Reference Sequence: NM_001324339.2**
   >NM_001324339.2 Homo sapiens zinc finger protein 354A (ZNF354A), transcript variant 2, mRNA/cDNA 
AGACGCCGUCGGGGCGGAGCCGGGUCGCGAGGCUGCAUCCCGGCCGGCGGUUCCGGAGCCUCGCGGCUGG
GGAGGCGCGGCCCGGGACGCUCGAGCUUAGGGAGGUGUCACUGACGUUUGAGGAUGUGGCUGUGCUGUUU
ACCCGAGAUGAGUGGAGAAAGCUGGCCCCUUCUCAGAGAAACUUGUACCGGGAUGUGAUGCUGGAGAACU
AUAGGAACCUGGUCUCACUGGGGCUCCCAUUUACCAAACCAAAAGUGAUCUCCCUGUUGCAGCAAGGAGA
AGAUCCCUGGGAGGUGGAGAAAGACGGUUCUGGCGUCUCCUCUCUAGGAUCGAAGAGCAGUCAUAAAACC
ACAAAGUCAACGCAAACACAAGACUCUUCAUUUCAGGGACUGAUACUGAAAAGAUCCAACAGGAAUGUAC
CUUGGGAUUUGAAAUUAGAAAAGCCUUACAUAUGAAGGCAGAUUAGAGAAAAAGCAGGAUAAAAAGGG
AAGUUUUCAGAUAGUUUCAGCCACCCACAAAAAAUCCCCACUAUAGAAAGAAGCCAUAAAAAUACUGAA
UUGAGCCAAAACUUCAGCCCAAAGUCAGUGCUUAUUAGGCAACAGAUACUUCCCAGAGAAAAAACACCAC
CAAAAUGUGAAAUACAAGGAAACAGCCUCAAACAGAAUUCACAAUUACUUAAUCAACCAAAAAUUACAGC
AGAUAAACGCUAUAAAUGUAGUCUGUGUGAAAAAACCUUCAUUAACACUUCAUCCCUUCGUAAACAUGAG
AAAAACCAUAGUGGAGAGAAACUAUUUAAGUGUAAAGAAUGUUCAAAAGCCUUUAGCCAAAGUUCAGCUC
UUAUUCAACAUCAAAUAACGCAUACUGGAGAGAAACCCUACAUAUGUAAAGAAUGUGGAAAGCCUUUAC
UCUCAGUACAUCCCUUUAUAAACAUCUAAGAACCCAUACUGUGGAGAAAUCCUACAGAUGUAAAGAAUGU
GGUAAAUCCUUCAGCCGAAGGUCAGGCCUUUUUAUACAUCAAAAAAUUCAUGCUGAAGAAAACCCUUGUA
AGUAUAAUCCGGGUAGGAAGGCAUCUAGUUGCAGCACAUCCCUUUCUGGAUGUCAAAGAAUUCAUUCUAG
AAAGAAGUCCUACUUAUGUAAUGAAUGUGGCAACACCUUUAAGUCUAGCUCAUCCCUUCGUUAUCAUCAG
AGAAUUCACACUGGAGAGAAGCCUUUUAAAUGUAGUGAAUGUGGGAGAGCCUUCAGCCAGAGUGCCUCUC
UUAUUCAACAUGAAAGAAUUCACACCGGAGAAAAGCCCUAUAGAUGCAAUGAAUGUGGGAAAGGCUUUAC
UUCUAUUUCACGACUUAAUAGACACCGAAUCAUUCAUACUGGAGAGAAGUUUUAUAAUUGUAAUGAAUGU
GGUAAAGCCUUAAGCUCCCACUCAACACUUAUUAUUCACGAGCGAAUUCAUACUGGAGAAAAACCAUGUA
AAUGUAAAGUAUGUGGAAAAGCCUUCAGACAGAGUUCAGCUCUCAUUCAACAUCAGAGAAUGCAUACUGG
AGAAAGACCCUAUAAAUGUAACGAGUGUGGAAAACAUUCAGGUGUAACUCAUCACUUAGUAAUCACCAG
AGAAUUCAUACUGGAGAGAAACCAUAUCGAUGUGAGGAAUGUGGAUAUCUUUGGCCAAAGUUCAGCUC
UUAUUCAGCAUCGAAGGAUUCAUACAGGAGAAAAACCCUUUAAAUGUAAUACAUGUGGAAAAACUUUUAG
ACAAAGCUCAUCACGUAUUGCACAUCAGAGAAUUCAUACUGGAGAGAAACCCUAUGAAUGUAAUACAUGU
GGGAAACUUUUCAACCAUAGGUCAUCCCUUACUAAUCAUUAUAAAAUUCAUAUCGAAGAGGACCCCUAGA
AAGUAGAUUUGUAUGUGUGAAAGCCUUAAACCAAAGCUCAUCGAAGAAUACAUCCUUGAGAGAGAUGUAA
UAAAUGUAAUGGAUGUGAAAAAAACUGUAAUAAUUUAGCCCUCAUUAGGUAUUUAAUUCCAUGGAUAAAC
CUCAGCUAUAUAAUAGAUAUGAGGAAAGUGUUUGUGCCUGUCAGACACUUAAAAAAAUAACCUGAGAUGA
AGAAUUUACAAUUGAAGACAUUGACUUUAGCCAUUUGUGAAAUGGGUUUGCUUUUUCCCUUUUCCUACAG
ACGUAUAUGCUAGAUGUCACGUGAUCAUCAGAAACAGAUAUCCGAGUGGGUGGGGAGGUGUGCUUUAGAU
UUCUCAUUAGAAGACCACCAAACUGGUAAUAUUUUUAUAGCAUUUUAAUAGCUUGAUCAAAUUGUACCUU
UUUAGAGAAAAGGACCAAAAUAAAAGAAAAAUGAAUUAUGAACUACCUCUCAGUCUCUGGGGUUUGUCCU
UUUCCUACCCUGAUGUCAAACUUAUGCAUGGAUUUCAUUAAAAAAGAAAAAGAAAAA
- **SEQ ID NO: 4**
   **Homo sapiens zinc finger protein 354A (ZNF354A), Translated Protein of transcript variant 2** 
MLENYRNLVSLGLPFTKPKVISLLQQGEDPWEVEKDGSGVSSLG
SKSSHKTTKSTQTQDSSFQGLILKRSNRNVPWDLKLEKPYIYEGRLEKKQDKKGSFQI
VSATHKKIPTIERSHKNTELSQNFSPKSVLIRQQILPREKTPPKCEIQGNSLKQNSQL
LNQPKITADKRYKCSLCEKTFINTSSLRKHEKNHSGEKLFKCKECSKAFSQSSALIQH
 
QITHTGEKPYICKECGKAFTLSTSLYKHLRTHTVEKSYRCKECGKSFSRRSGLFIHQK
IHAEENPCKYNPGRKASSCSTSLSGCQRIHSRKKSYLCNECGNTFKSSSSLRYHQRIH
TGEKPFKCSECGRAFSQSASLIQHERIHTGEKPYRCNECGKGFTSISRLNRHRIIHTG
EKFYNCNECGKALSSHSTLIIHERIHTGEKPCKCKVCGKAFRQSSALIQHQRMHTGER
PYKCNECGKTFRCNSSLSNHQRIHTGEKPYRCEECGISFGQSSALIQHRRIHTGEKPF
KCNTCGKTFRQSSSRIAHQRIHTGEKPYECNTCGKLFNHRSSLTNHYKIHIEEDP
- **SEQ ID NO: 5**
   **Homo sapiens zinc finger protein 354A (ZNF354A), transcript variant X3, mRNA. NCBI Reference Sequence: XM_011534645.3**
   >XM_011534645.3 PREDICTED: Homo sapiens zinc finger protein 354A (ZNF354A), transcript variant X3, mRNA **/cDNA** 
UCCUGCCACUGCGCUACUGCACUCCAGUCUGGCCAACAGAGCGAGACUCCGCCUCAAAAAAAAAAAAGCC
GUUUUCCGUGGAGUCGGGAGAUGAUUCGUUGGCGGGAGAGAGAGGAACCGGCUGGGAAAGGCUUGAGGCG
GAGGGAAGUCGUCCCGGGGCCGUCGUGGUGGGAGUCCCGGCCCGCCUCGCAGCGGCGUGAACGGGGCAGG
UGCCCCGGCGCUGCCUCAGUUUCCUGAGCGGUGCAGUGGGUGCUGGCAGUGCCAGCUGGCGCCGAGGAAC
UCAGCGGCGUGGGGCGAGCCCUGGCCCUGGUGGGCUCAGCGGGUCGCUGCUGCCACUGCGGCUCCAGCGU
CCCCUCCGUAAGCCCCAAGCCUGUGGGGCCUGGGCCUGGCCGGGCGGCCCAGCGCUGCUCUGGUCCGCGG
GCUCCUGGCUCCUCCCGAAUCCCUGUGAGGGCGCGCGGGGUCCUUCUCAGCGGGAGUCGGGGUUUUAGAG
CUGCGGAUUCCAGGGCUGGAAAGCAGAAGGGGUUCUUCCUGGCUCCCUUUUUCUUCUCAGAUCUGCCUUC
UGGAGACUGCGCCGUCCUCCCGGGAGAGCCAGAAAGAGGACAUGGCUGCUGGGCAGCGGGAAGCGAGGCC
CCAGUGGCUGCACUAGGCCUGAAUCUCACUGUGUGCUGCUCAGUAUACAUUUGUUACUGGCCAAUUAUUC
AUUAAACGAAUGAUCUGGAACCUUCAGAAGCUUGAAUAGUGUCACUGACGUUUGAGGAUGUGGCUGUGCU
GUUUACCCGAGAUGAGUGGAGAAAGCUGGCCCCUUCUCAGAGAAACUUGUACCGGGAUGUGAUGCUGGAG
AACUAUAGGAACCUGGUCUCACUGGGGCUCCCAUUUACCAAACCAAAAGUGAUCUCCCUGUUGCAGCAAG
GAGAAGAUCCCUGGGAGGUGGAGAAAGACGGUUCUGGCGUCUCCUCUCUAGGAUCGAAGAGCAGUCAUAA
AACCACAAAGUCAACGCAAACACAAGACUCUUCAUUUCAGGGACUGAUACUGAAAAGAUCCAACAGGAAU
GUACCUUGGGAUUUGAAAUUAGAAAAGCCUUACAUAUAUGAAGGCAGAUUAGAGAAAAAGCAGGAUAAAA
AGGGAAGUUUCAGAUAGUUUCAGCCACCCACAAAAAAUCCCCACUAUAGAAAGAAGCCAUAAAAAUAC
UGAAUUGAGCCAAAACUUCAGCCCAAAGUCAGUGCUUAUUAGGCAACAGAUACUUCCCAGAGAAAAAACA
CCACCAAAAUGUGAAAUACAAGGAAACAGCCUCAAACAGAAUUCACAAUUACUUAAUCAACCAAAAAUUA
CAGCAGAUAAACGCUAUAAAUGUAGUCUGUGUGAAAAACCUUCAUUAACACUUCAUCCCUUCGUAAACA
UGAGAAAAACCAUAGUGGAGAGAAACUAUUUAAGUGUAAAGAAUGUUCAAAAGCCUUUAGCCAAAGUUCA
GCUCUUAUUCAACAUCAAAUAACGCAUACUGGAGAGAAACCCUACAUAUGUAAAGAAUGUGGGAAAGCCU
UUACUCUCAGUACAUCCCUUUAUAAACAUCUAAGAACCCAUACUGUGGAGAAAUCCUACAGAUGUAAAGA
AUGUGGUAAAUCCUUCAGCCGAAGGUCAGGCCUUUUUAUACAUCAAAAAAUUCAUGCUGAAGAAAACCCU
UGUAAGUAUAAUCCGGGUAGGAAGGCAUCUAGUUGCAGCACAUCCCUUUCUGGAUGUCAAAGAAUUCAUU
CUAGAAAGAAGUCCUACUUAUGUAAUGAAUGUGGCAACACCUUUAAGUCUAGCUCAUCCCUUCGUUAUCA
UCAGAGAAUUCACACUGGAGAGAAGCCUUUUAAAUGUAGUGAAUGUGGGAGAGCCUUCAGCCAGAGUGCC
UCUCUUAUUCAACAUGAAAGAAUUCACACCGGAGAAAAGCCCUAUAGAUGCAAUGAAUGUGGGAAAGGCU
UUACUUCUAUUUCACGACUUAAUAGACACCGAAUCAUUCAUACUGGAGAGAAGUUUUAUAAUUGUAAUGA
AUGUGGUAAAGCCUUAAGCUCCCACUCAACACUUAUUAUUCACGAGCGAAUUCAUACUGGAGAAAAACCA
UGUAAAUGUAAAGUAUGUGGAAAAGCCUUCAGACAGAGUUCAGCUCUCAUUCAACAUCAGAGAAUGCAUA
CUGGAGAAAGACCCUAUAAAUGUAACGAGUGUGGAAAACAUUCAGGUGUAACUCAUCACUUAGUAAUCA
CCAGAGAAUUCAUACUGGAGAGAAACCAUAUCGAUGUGAGGAAUGUGGAUAUCUUUGGCCAAAGUUCA
GCUCUUAUUCAGCAUCGAAGGAUUCAUACAGGAGAAAAACCCUUUAAAUGUAAUACAUGUGGAAAAACUU
UUAGACAAAGCUCAUCACGUAUUGCACAUCAGAGAAUUCAUACUGGAGAGAAACCCUAUGAAUGUAAUAC
AUGUGGGAAACUUUUCAACCAUAGGUCAUCCCUUACUAAUCAUUAUAAAUUCAUAUCGAAGAGGACCCC
UAGAAAGUAGAUUUGUAUGUGUGAAAGCCUUAAACCAAAGCUCAUCGAAGAAUACAUCCUUGAGAGAGAU
GUAAUAAAUGUAAUGGAUGUGAAAAAAACUGUAAUAAUUUAGCCCUCAUUAGGUAUUUAAUUCCAUGGAU
AAACCUCAGCUAUAUAAUAGAUAUGAGGAAAGUGUUUGUGCCUGUCAGACACUUAAAAAAAUAACCUGAG
AUGAAGAAUUUACAAUUGAAGACAUUGACUUUAGCCAUUUGUGAAAUGGGUUUGCUUUUUCCCUUUUCCU
ACAGACGUAUAUGCUAGAUGUCACGUGAUCAUCAGAAACAGAUAUCCGAGUGGGUGGGGAGGUGUGCUUU
AGAUUUCUCAUUAGAAGACCACCAAACUGGUAAUAUUUUAUAGCAUUUUAAUAGCUUGAUCAAAUUGUA
CCUUUUUAGAGAAAAGGACCAAAAUAAAAGAAAAAUGAAUUAUGAACUACCUCUCAGUCUCUGGGGUUUG
UCCUUUUCCUACCCUGAUGUCAAACUUAUGCAUGGAUUUCAUUAAAAAAGAAAAAGAAAAA
- **SEQ ID NO: 6**
   **Homo sapiens zinc finger protein 354A (ZNF354A), Translated Protein of transcript variant X3** 
MLENYRNLVSLGLPFTKPKVISLLQQGEDPWEVEKDGSGVSSLG
SKSSHKTTKSTQTQDSSFQGLILKRSNRNVPWDLKLEKPYIYEGRLEKKQDKKGSFQI
VSATHKKIPTIERSHKNTELSQNFSPKSVLIRQQILPREKTPPKCEIQGNSLKQNSQL
LNQPKITADKRYKCSLCEKTFINTSSLRKHEKNHSGEKLFKCKECSKAFSQSSALIQH
QITHTGEKPYICKECGKAFTLSTSLYKHLRTHTVEKSYRCKECGKSFSRRSGLFIHQK
IHAEENPCKYNPGRKASSCSTSLSGCQRIHSRKKSYLCNECGNTFKSSSSLRYHQRIH
TGEKPFKCSECGRAFSQSASLIQHERIHTGEKPYRCNECGKGFTSISRLNRHRIIHTG
EKFYNCNECGKALSSHSTLIIHERIHTGEKPCKCKVCGKAFRQSSALIQHQRMHTGER
PYKCNECGKTFRCNSSLSNHQRIHTGEKPYRCEECGISFGQSSALIQHRRIHTGEKPF
KCNTCGKTFRQSSSRIAHQRIHTGEKPYECNTCGKLFNHRSSLTNHYKIHIEEDP
- **SEQ ID NO: 7**
   **Homo sapiens zinc finger protein 354A (ZNF354A), transcript variant X2, mRNA NCBI Reference Sequence: XM_017009791.2**
   >XM_017009791.2 PREDICTED: Homo sapiens zinc finger protein 354A (ZNF354A), transcript variant X2, mRNA/cDNA 
AGACGCCGUCGGGGCGGAGCCGGGUCGCGAGGCUGCAUCCCGGCCGGCGGUUCCGGAGCCUCGCGGCUGG
GGAGGCGCGGCCCGGGACGCUCGAGCUUAGGGAGGUGAGCGUCGGCGGCAGGCCAAGCCGAUCUGCCUUC
UGGAGACUGCGCCGUCCUCCCGGGAGAGCCAGAAAGAGGACAUGGCUGCUGGGCAGCGGGAAGCGAGGCC
CCAGGUGUCACUGACGUUUGAGGAUGUGGCUGUGCUGUUUACCCGAGAUGAGUGGAGAAAGCUGGCCCCU
UCUCAGAGAAACUUGUACCGGGAUGUGAUGCUGGAGAACUAUAGGAACCUGGUCUCACUGGGGCUCCCAU
UUACCAAACCAAAAGUGAUCUCCCUGUUGCAGCAAGGAGAAGAUCCCUGGGAGGUGGAGAAAGACGGUUC
UGGCGUCUCCUCUCUAGGAUCGAAGAGCAGUCAUAAACCACAAAGUCAACGCAAACACAAGACUCUUCA
UUUCAGGGACUGAUACUGAAAAGAUCCAACAGGAAUGUACCUUGGGAUUUGAAAUUAGAAAAGCCUUACA
UAUAUGAAGGCAGAUUAGAGAAAAAGCAGGAUAAAAAGGGAAGUUUUCAGAUAGUUUCAGCCACCCACAA
AAAAAUCCCACUAUAGAAAGAAGCAUAAAAAUACUGAAUUGAGCCAAAACUUCAGCCCAAAGUCAGUG
CUUAUUAGGCAACAGAUACUUCCCAGAGAAAAAACACCACCAAAAUGUGAAAUACAAGGAAACAGCCUCA
AACAGAAUUCACAAUUACUUAAUCAACCAAAAAUUACAGCAGAUAAACGCUAUAAAUGUAGUCUGUGUGA
AAAAACCUUCAUUAACACUUCAUCCCUUCGUAAACAUGAGAAAAACCAUAGUGGAGAGAAACUAUUUAAG
UGUAAAGAAUGUUCAAAAGCCUUUAGCCAAAGUUCAGCUCUUAUUCAACAUCAAAUAACGCAUACUGGAG
AGAAACCCUACAUAUGUAAAGAAUGUGGGAAAGCCUUUACUCUCAGUACAUCCCUUUAUAAACAUCUAAG
AACCCAUACUGUGGAGAAAUCCUACAGAUGUAAAGAAUGUGGUAAAUCCUUCAGCCGAAGGUCAGGCCUU
UUUAUACAUCAAAAAAUUCAUGCUGAAGAAAACCCUUGUAAGUAUAAUCCGGGUAGGAAGGCAUCUAGUU
GCAGCACAUCCCUUUCUGGAUGUCAAAGAAUUCAUUCUAGAAAGAAGUCCUACUUAUGUAAUGAAUGUGG
CAACACCUUUAAGUCUAGCUCAUCCCUUCGUUAUCAUCAGAGAAUUCACACUGGAGAGAAGCCUUUUAAA
UGUAGUGAAUGUGGGAGAGCCUUCAGCCAGAGUGCCUCUCUUAUUCAACAUGAAAGAAUUCACACCGGAG
AAAAGCCCUAUAGAUGCAAUGAAUGUGGGAAAGGCUUUACUUCUAUUUCACGACUUAAUAGACACCGAAU
CAUUCAUACUGGAGAGAAGUUUUAUAAUUGUAAUGAAUGUGGUAAAGCCUUAAGCUCCCACUCAACACUU
AUUAUUCACGAGCGAAUUCAUACUGGAGAAAAACCAUGUAAAUGUAAAGUAUGUGGAAAAGCCUUCAGAC
AGAGUUCAGCUCUCAUUCAACAUCAGAGAAUGCAUACUGGAGAAAGACCCUAUAAAUGUAACGAGUGUGG
GAAAACAUUCAGGUGUAACUCAUCACUUAGUAAUCACCAGAGAAUUCAUACUGGAGAGAAACCAUAUCGA
UGUGAGGAAUGUGGGAUAUCUUUUGGCCAAAGUUCAGCUCUUAUUCAGCAUCGAAGGAUUCAUACAGGAG
AAAAACCCUUUAAAUGUAAUACAUGUGGAAAAACUUUUAGACAAAGCUCAUCACGUAUUGCACAUCAGAG
AAUUCAUACUGGAGAGAAACCCUAUGAAUGUAAUACAUGUGGGAAACUUUUCAACCAUAGGUCAUCCCUU
ACUAAUCAUUAUAAAAUUCAUAUCGAAGAGGACCCCUAGAAAGUAGAUUUGUAUGUGUGAAAGCCUUAAA
CCAAAGCUCAUCGAAGAAUACAUCCUUGAGAGAGAUGUAAUAAAUGUAAUGGAUGUGAAAAAAACUGUAA
UAAUUUAGCCCUCAUUAGGUAUUUAAUUCCAUGGAUAAACCUCAGCUAUAUAAUAGAUAUGAGGAAAGUG
UUUGUGCCUGUCAGACACUUAAAAAAAUAACCUGAGAUGAAGAAUUUACAAUUGAAGACAUUGACUUUAG
CCAUUUGUGAAAUGGGUUUGCUUUUUCCCUUUUCCUACAGACGUAUAUGCUAGAUGUCACGUGAUCAUCA
GAAACAGAUAUCCGAGUGGGUGGGGAGGUGUGCUUUAGAUUUCUCAUUAGAAGACCACCAAACUGGUAAU
AUUUUUAUAGCAUUUUAAUAGCUUGAUCAAAUUGUACCUUUUUAGAGAAAAGGACCAAAAUAAAAGAAAA
AUGAAUUAUGAACUACCUCUCAGUCUCUGGGGUUUGUCCUUUUCCUACCCUGAUGUCAAACUUAUGCAUG
 GAUUUCAUUAAAAAGAAAAAGAAAAA
- **SEQ ID NO:8**
   **Homo sapiens zinc finger protein 354A (ZNF354A), Translated Protein of transcript variant X2** 
MAAGQREARPQVSLTFEDVAVLFTRDEWRKLAPSQRNLYRDVML
ENYRNLVSLGLPFTKPKVISLLQQGEDPWEVEKDGSGVSSLGSKSSHKTTKSTQTQDS
SFQGLILKRSNRNVPWDLKLEKPYIYEGRLEKKQDKKGSFQIVSATHKKIPTIERSHK
NTELSQNFSPKSVLIRQQILPREKTPPKCEIQGNSLKQNSQLLNQPKITADKRYKCSL
CEKTFINTSSLRKHEKNHSGEKLFKCKECSKAFSQSSALIQHQITHTGEKPYICKECG
KAFTLSTSLYKHLRTHTVEKSYRCKECGKSFSRRSGLFIHQKIHAEENPCKYNPGRKA
SSCSTSLSGCQRIHSRKKSYLCNECGNTFKSSSSLRYHQRIHTGEKPFKCSECGRAFS
QSASLIQHERIHTGEKPYRCNECGKGFTSISRLNRHRIIHTGEKFYNCNECGKALSSH
STLIIHERIHTGEKPCKCKVCGKAFRQSSALIQHQRMHTGERPYKCNECGKTFRCNSS
LSNHQRIHTGEKPYRCEECGISFGQSSALIQHRRIHTGEKPFKCNTCGKTFRQSSSRI
AHQRIHTGEKPYECNTCGKLFNHRSSLTNHYKIHIEEDP
- **SEQ ID NO: 9**
   **Homo sapiens zinc finger protein 354A (ZNF354A), transcript variant X1, mRNA,NCBI Reference Sequence: XM_047417644.1**
   >XM_047417644.1 PREDICTED: Homo sapiens zinc finger protein 354A (ZNF354A), transcript variant X1, mRNA/cDNA 
AGACGCCGUCGGGGCGGAGCCGGGUCGCGAGGCUGCAUCCCGGCCGGCGGUUCCGGAGCCUCGCGGCUGG
GGAGGCGCGGCCCGGGACGCUCGAGCUUAGGGAGGUGAGCGUCGGCGGCAGGCCAAGCCGGUAGGAGGCG
GCGCCCGGCACGGGGACUGCCUGCGGCUGCCGAGGCUCCGAAAGCCUUUGUGGGCGCUGGCGGGCACCCC
CGCGUCUGUCACCGGCCCCCGCGCCGGGAUCUGGACUCUCGCCCCCCCGCGGGCGCUCCCUCGCCGUUG
CUGGUGCACCCGCGUCCGGCUCGCCGUGGACGCCGGGCCUGGCGUUUCCUCCCGGGCCCGUCCUGCCCAC
UCCAUGUGGGCCUCGCGGACUCGGGGCACCGGACCCCGAGGGCAGGGCGGACGCAGCCGGGCCCCGGUU
UCCGAGUCCGUCCCAGGACGGCCACCCCUCCCGCCUCGGACACCCAGCGCUUUCCUCUUCAGAGCUGG
UGUUUGGCCGGGCGCGGUGGCUCACGCCUGUAAUCCCAGCACUUUGGGAGGCCGAAGCGGGUGGAUCACG
AGGUCAGGAGAUCGAGACCAUCCUGACUAACACGAUCUGCCUUCUGGAGACUGCGCCGUCCUCCCGGGAG
AGCCAGAAAGAGGACAUGGCUGCUGGGCAGCGGAAGCGAGGCCCCAGGUGUCACUGACGUUUGAGGAUG
UGGCUGUGCUGUUUACCCGAGAUGAGUGGAGAAAGCUGGCCCCUUCUCAGAGAAACUUGUACCGGGAUGU
GAUGCUGGAGAACUAUAGGAACCUGGUCUCACUGGGGCUCCCAUUUACCAAACCAAAAGUGAUCUCCCUG
UUGCAGCAAGGAGAAGAUCCCUGGGAGGUGGAGAAAGACGGUUCUGGCGUCUCCUCUCUAGGAUCGAAGA
GCAGUCAUAAAACCACAAAGUCAACGCAAACACAAGACUCUUCAUUUCAGGGACUGAUACUGAAAAGAUC
CAACAGGAAUGUACCUUGGGAUUUGAAAUUAGAAAAGCCUUACAUAUAUGAAGGCAGAUUAGAGAAAAAG
CAGGAUAAAAAGGGAAGUUUUCAGAUAGUUUCAGCCACCCACAAAAAAAUCCCCACUAUAGAAAGAAGCC
AUAAAAAUACUGAAUUGAGCCAAAACUUCAGCCCAAAGUCAGUGCUUAUUAGGCAACAGAUACUUCCCAG
AGAAAAAACACCACCAAAAUGUGAAAUACAAGGAAACAGCCUCAAACAGAAUUCACAAUUACUUAAUCAA
CCAAAAAUUACAGCAGAUAAACGCUAUAAAUGUAGUCUGUGUGAAAAAACCUUCAUUAACACUUCAUCCC
UUCGUAAACAUGAGAAAAACCAUAGUGGAGAGAAACUAUUUAAGUGUAAAGAAUGUUCAAAAGCCUUUAG
CCAAAGUUCAGCUCUUAUUCAACAUCAAAUAACGCAUACUGGAGAGAAACCCUACAUAUGUAAAGAAUGU
GGGAAAGCCUUUACUCUCAGUACAUCCCUUUAUAAACAUCUAAGAACCCAUACUGUGGAGAAAUCCUACA
GAUGUAAAGAAUGUGGUAAAUCCUUCAGCCGAAGGUCAGGCCUUUUUAUACAUCAAAAAAUUCAUGCUGA
AGAAAACCCUUGUAAGUAUAAUCCGGGUAGGAAGGCAUCUAGUUGCAGCACAUCCCUUUCUGGAUGUCAA
AGAAUUCAUUCUAGAAAGAAGUCCUACUUAUGUAAUGAAUGUGGCAACACCUUUAAGUCUAGCUCAUCCC
UUCGUUAUCAUCAGAGAAUUCACACUGGAGAGAAGCCUUUUAAAUGUAGUGAAUGUGGGAGAGCCUUCAG
CCAGAGUGCCUCUCUUAUUCAACAUGAAAGAAUUCACACCGGAGAAAAGCCCUAUAGAUGCAAUGAAUGU
GGGAAAGGCUUUACUUCUAUUUCACGACUUAAUAGACACCGAAUCAUUCAUACUGGAGAGAAGUUUUAUA
AUUGUAAUGAAUGUGGUAAAGCCUUAAGCUCCCACUCAACACUUAUUAUUCACGAGCGAAUUCAUACUGG
AGAAAAACCAUGUAAAUGUAAAGUAUGUGGAAAAGCCUUCAGACAGAGUUCAGCUCUCAUUCAACAUCAG
AGAAUGCAUACUGGAGAAAGACCCUAUAAAUGUAACGAGUGUGGGAAAACAUUCAGGUGUAACUCAUCAC
UUAGUAAUCACCAGAGAAUUCAUACUGGAGAGAAACCAUAUCGAUGUGAGGAAUGUGGGAUAUCUUUUGG
CCAAAGUUCAGCUCUUAUUCAGCAUCGAAGGAUUCAUACAGGAGAAAAACCCUUUAAAUGUAAUACAUGU
 
GGAAAAACUUUUAGACAAAGCUCAUCACGUAUUGCACAUCAGAGAAUUCAUACUGGAGAGAAACCCUAUG
AAUGUAAUACAUGUGGGAAACUUUUCAACCAUAGGUCAUCCCUUACUAAUCAUUAUAAAAUUCAUAUCGA
AGAGGACCCCUAGAAAGUAGAUUUGUAUGUGUGAAAGCCUUAAACCAAAGCUCAUCGAAGAAUACAUCCU
UGAGAGAGAUGUAAUAAAUGUAAUGGAUGUGAAAAAAACUGUAAUAAUUUAGCCCUCAUUAGGUAUUUAA
UUCCAUGGAUAAACCUCAGCUAUAUAAUAGAUAUGAGGAAAGUGUUUGUGCCUGUCAGACACUUAAAAAA
AUAACCUGAGAUGAAGAAUUUACAAUUGAAGACAUUGACUUUAGCCAUUUGUGAAAUGGGUUUGCUUUUU
CCCUUUUCCUACAGACGUAUAUGCUAGAUGUCACGUGAUCAUCAGAAACAGAUAUCCGAGUGGGUGGGGA
GGUGUGCUUUAGAUUUCUCAUUAGAAGACCACCAAACUGGUAAUAUUUUAUAGCAUUUUAAUAGCUUGA
UCAAAUUGUACCUUUUUAGAGAAAAGGACCAAAAUAAAAGAAAAAUGAAUUAUGAACUACCUCUCAGUCU
CUGGGGUUUGUCCUUUUCCUACCCUGAUGUCAAACUUAUGCAUGGAUUUCAUUAAAAAAGAAAAAGAAAA
A
- **SEQ ID NO: 10**
   **Homo sapiens zinc finger protein 354A (ZNF354A), Translated Protein of transcript variant X1** 
MAAGQREARPQVSLTFEDVAVLFTRDEWRKLAPSQRNLYRDVML
ENYRNLVSLGLPFTKPKVISLLQQGEDPWEVEKDGSGVSSLGSKSSHKTTKSTQTQDS
SFQGLILKRSNRNVPWDLKLEKPYIYEGRLEKKQDKKGSFQIVSATHKKIPTIERSHK
NTELSQNFSPKSVLIRQQILPREKTPPKCEIQGNSLKQNSQLLNQPKITADKRYKCSL
CEKTFINTSSLRKHEKNHSGEKLFKCKECSKAFSQSSALIQHQITHTGEKPYICKECG
KAFTLSTSLYKHLRTHTVEKSYRCKECGKSFSRRSGLFIHQKIHAEENPCKYNPGRKA
SSCSTSLSGCQRIHSRKKSYLCNECGNTFKSSSSLRYHQRIHTGEKPFKCSECGRAFS
QSASLIQHERIHTGEKPYRCNECGKGFTSISRLNRHRIIHTGEKFYNCNECGKALSSH
STLIIHERIHTGEKPCKCKVCGKAFRQSSALIQHQRMHTGERPYKCNECGKTFRCNSS
LSNHQRIHTGEKPYRCEECGISFGQSSALIQHRRIHTGEKPFKCNTCGKTFRQSSSRI
AHQRIHTGEKPYECNTCGKLFNHRSSLTNHYKIHIEEDP
- **SEQ ID NO:11**
   **Homo sapiens zinc finger protein 354A (ZNF354A), transcript variant X2, mRNA, NCBI Reference Sequence: XM_054353337.1**
   >XM_054353337.1 PREDICTED: Homo sapiens zinc finger protein 354A (ZNF354A), transcript variant X2, mRNA/cDNA 
GCCCCUCCUGGUCCCGGCGCGCCGCGGGGUGAGCGGCGCCGGGCCUGAGGUCGCCCAGACGCCGUCGGG
GCGGAGCCGGGUCGCGAGGCUGCAUCCCGGCCGGCGGUUCCGGAGCCUCGCGGCUGGGGAGGCGCGGCCC
GGGACGCUCGAGCUUAGGGAGGUGAGCGUCGGCGGCAGGCCAAGCCGAUCUGCCUUCUGGAGACUGCGCC
GUCCUCCCGGGAGAGCCAGAAAGAGGACAUGGCUGCUGGGCAGCGGAAGCGAGGCCCCAGGUGUCACUG
ACGUUUGAGGAUGUGGCUGUGCUGUUUACCCGAGAUGAGUGGAGAAAGCUGGCCCCUUCUCAGAGAAACU
UGUACCGGGAUGUGAUGCUGGAGAACUAUAGGAACCUGGUCUCACUGGGGCUCCCAUUUACCAAACCAAA
AGUGAUCUCCCUGUUGCAGCAAGGAGAAGAUCCCUGGGAGGUGGAGAAAGACGGUUCUGGCGUCUCCUCU
CUAGGAUCGAAGAGCAGUCAUAAAACCACAAAGUCAACGCAAACACAAGACUCUUCAUUUCAGGGACUGA
UACUGAAAAGAUCCAACAGGAAUGUACCUUGGGAUUUGAAAUUAGAAAAGCCUUACAUAUAUGAAGGCAG
AUUAGAGAAAAAGCAGGAUAAAAAGGGAAGUUUUCAGAUAGUUUCAGCCACCCACAAAAAAAUCCCCACU
AUAGAAAGAAGCCAUAAAAAUACUGAAUUGAGCCAAAACUUCAGCCCAAAGUCAGUGCUUAUUAGGCAAC
AGAUACUUCCCAGAGAAAAAACACCACCAAAAUGUGAAAUACAAGGAAACAGCCUCAAACAGAAUUCACA
AUUACUUAAUCAACCAAAAAUUACAGCAGAUAAACGCUAUAAAUGUAGUCUGUGUGAAAAACCUUCAUU
AACACUUCAUCCCUUCGUAAACAUGAGAAAAACCAUAGUGGAGAGAAACUAUUUAAGUGUAAAGAAUGUU
CAAAAGCCUUUAGCCAAAGUUCAGCUCUUAUUCAACAUCAAAUAACGCAUACUGGAGAGAAACCCUACAU
AUGUAAAGAAUGUGGGAAAGCCUUUACUCUCAGUACAUCCCUUUAUAAACAUCUAAGAACCCAUACUGUG
GAGAAAUCCUACAGAUGUAAAGAAUGUGGUAAAUCCUUCAGCCGAAGGUCAGGCCUUUUUAUACAUCAAA
AAAUUCAUGCUGAAGAAAACCCUUGUAAGUAUAAUCCGGGUAGGAAGGCAUCUAGUUGCAGCACAUCCCU
UUCUGGAUGUCAAAGAAUUCAUUCUAGAAAGAAGUCCUACUUAUGUAAUGAAUGUGGCAACACCUUUAAG
 
UCUAGCUCAUCCCUUCGUUAUCAUCAGAGAAUUCACACUGGAGAGAAGCCUUUUAAAUGUAGUGAAUGUG
GGAGAGCCUUCAGCCAGAGUGCCUCUCUUAUUCAACAUGAAAGAAUUCACACCGGAGAAAAGCCCUAUAG
AUGCAAUGAAUGUGGGAAAGGCUUUACUUCUAUUUCACGACUUAAUAGACACCGAAUCAUUCAUACUGGA
GAGAAGUUUUAUAAUUGUAAUGAAUGUGGUAAAGCCUUAAGCUCCCACUCAACACUUAUUAUUCACGAGC
GAAUUCAUACUGGAGAAAAACCAUGUAAAUGUAAAGUAUGUGGAAAAGCCUUCAGACAGAGUUCAGCUCU
CAUUCAACAUCAGAGAAUGCAUACUGGAGAAAGACCCUAUAAAUGUAACGAGUGUGGGAAAACAUUCAGG
UGUAACUCAUCACUUAGUAAUCACCAGAGAAUUCAUACUGGAGAGAAACCAUAUCGAUGUGAGGAAUGUG
GGAUAUCUUUUGGCCAAAGUUCAGCUCUUAUUCAGCAUCGAAGGAUUCAUACAGGAGAAAAACCCUUUAA
AUGUAAUACAUGUGGAAAAACUUUUAGACAAAGCUCAUCACGUAUUGCACAUCAGAGAAUUCAUACUGGA
GAGAAACCCUAUGAAUGUAAUACAUGUGGGAAACUUUUCAACCAUAGGUCAUCCCUUACUAAUCAUUAUA
AAAUUCAUAUCGAAGAGGACCCCUAGAAAGUAGAUUUGUAUGUGUGAAAGCCUUAAACCAAAGCUCAUCG
AAGAAUACAUCCUUGAGAGAGAUGUAAUAAAUGUAAUGGAUGUGAAAAAAACUGUAAUAAUUUAGCCCUC
AUUAGGUAUUUAAUUCCAUGGAUAAACCUCAGCUAUAUAAUAGAUAUGAGGAAAGUGUUUGUGCCUGUCA
GACACUUAAAAAAAUAACCUGAGAUGAAGAAUUUACAAUUGAAGACAUUGACUUUAGCCAUUUGUGAAAU
GGGUUUGCUUUUUCCCUUUUCCUACAGACGUAUAUGCUAGAUGUCACGUGAUCAUCAGAAACAGAUAUCC
GAGUGGGUGGGGAGGUGUGCUUUAGAUUUCUCAUUAGAAGACCACCAAACUGGUAAUAUUUUUAUAGCAU
UUUAAUAGCUUGAUCAAAUUGUACCUUUUUAGAGAAAAGGACCAAAAUAAAAGAAAAAUGAAUUAUGAAC
UACCUCUCAGUCUCUGGGGUUUGUCCUUUUCCUACCCUGAUGUCAAACUUAUGCAUGGAUUUCAUUAAAA
AAGAAAAAGAAAAA
- **SEQ ID NO:12**
   **Homo sapiens zinc finger protein 354A (ZNF354A), Translated Protein of transcript variant X2** 
MAAGQREARPQVSLTFEDVAVLFTRDEWRKLAPSQRNLYRDVML
ENYRNLVSLGLPFTKPKVISLLQQGEDPWEVEKDGSGVSSLGSKSSHKTTKSTQTQDS
SFQGLILKRSNRNVPWDLKLEKPYIYEGRLEKKQDKKGSFQIVSATHKKIPTIERSHK
NTELSQNFSPKSVLIRQQILPREKTPPKCEIQGNSLKQNSQLLNQPKITADKRYKCSL
CEKTFINTSSLRKHEKNHSGEKLFKCKECSKAFSQSSALIQHQITHTGEKPYICKECG
KAFTLSTSLYKHLRTHTVEKSYRCKECGKSFSRRSGLFIHQKIHAEENPCKYNPGRKA
SSCSTSLSGCQRIHSRKKSYLCNECGNTFKSSSSLRYHQRIHTGEKPFKCSECGRAFS
QSASLIQHERIHTGEKPYRCNECGKGFTSISRLNRHRIIHTGEKFYNCNECGKALSSH
STLIIHERIHTGEKPCKCKVCGKAFRQSSALIQHQRMHTGERPYKCNECGKTFRCNSS
LSNHQRIHTGEKPYRCEECGISFGQSSALIQHRRIHTGEKPFKCNTCGKTFRQSSSRI
AHQRIHTGEKPYECNTCGKLFNHRSSLTNHYKIHIEEDP
- **SEQ ID NO: 13**
   **Homo sapiens zinc finger protein 354A (ZNF354A), transcript variant X1, mRNA, NCBI Reference Sequence: XM_054353338.1**
   >XM_054353338.1 PREDICTED: Homo sapiens zinc finger protein 354A (ZNF354A), transcript variant X1, mRNA/cDNA 
GCCCCUCCUGGUCCCGGCGCGCCGCGGGUGAGCGGCGCCGGGCCUGAGGUCGCCCAGACGCCGUCGGG
GCGGAGCCGGGUCGCGAGGCUGCAUCCCGGCCGGCGGUUCCGGAGCCUCGCGGCUGGGGAGGCGCGGCCC
GGGACGCUCGAGCUUAGGGAGGUGAGCGUCGGCGGCAGGCCAAGCCGGUAGGAGGCGGCGCCCGGCACGG
GGACUGCCUGCGGCUGCCGAGGCUCCGAAAGCCUUUGUGGGCGCUGGCGGCACCCCCGCGUCUGUCACC
GGCCCCCGCGCCGGGAUCUGGACUCUCGCCCCCCCGCGGGGCGCUCCCUCGCCGUUGCUGGUGCACCCGC
GUCCGGCUCGCCGUGGACGCCGGGCCUGGCGUUUCCUCCCGGGCCCGUCCUGCCCACUCCAUGUGGGCCU
CGCGGACUCGGGGCACCGGGACCCCGAGGGCAGGGCGGACGCAGCCGGGCCCCGGUUUCCGAGUCCGUCC
CCAGGACGGCCACCCCUCCCGCCUCGGGACACCCAGCGCUUUCCUCUUCAGAGCUGGUGUUUGGCCGGGC
GCGGUGGCUCACGCCUGUAAUCCCAGCACUUUGGGAGGCCGAAGCGGGUGGAUCACGAGGUCAGGAGAUC
GAGACCAUCCUGACUAACACGAUCUGCCUUCUGGAGACUGCGCCGUCCUCCCGGGAGAGCCAGAAAGAGG
ACAUGGCUGCUGGGCAGCGGGAAGCGAGGCCCCAGGUGUCACUGACGUUUGAGGAUGUGGCUGUGCUGUU
UACCCGAGAUGAGUGGAGAAAGCUGGCCCCUUCUCAGAGAAACUUGUACCGGGAUGUGAUGCUGGAGAAC
 
UAUAGGAACCUGGUCUCACUGGGGCUCCCAUUUACCAAACCAAAAGUGAUCUCCCUGUUGCAGCAAGGAG
AAGAUCCCUGGGAGGUGGAGAAAGACGGUUCUGGCGUCUCCUCUCUAGGAUCGAAGAGCAGUCAUAAAAC
CACAAAGUCAACGCAAACACAAGACUCUUCAUUUCAGGGACUGAUACUGAAAAGAUCCAACAGGAAUGUA
CCUUGGGAUUUGAAAUUAGAAAAGCCUUACAUAUAUGAAGGCAGAUUAGAGAAAAAGCAGGAUAAAAAGG
GAAGUUUUCAGAUAGUUUCAGCCACCCACAAAAAAAUCCCCACUAUAGAAAGAAGCCAUAAAAAUACUGA
AUUGAGCCAAAACUUCAGCCCAAAGUCAGUGCUUAUUAGGCAACAGAUACUUCCCAGAGAAAAAACACCA
CCAAAAUGUGAAAUACAAGGAAACAGCCUCAAACAGAAUUCACAAUUACUUAAUCAACCAAAAAUUACAG
CAGAUAAACGCUAUAAAUGUAGUCUGUGUGAAAAAACCUUCAUUAACACUUCAUCCCUUCGUAAACAUGA
GAAAAACCAUAGUGGAGAGAAACUAUUUAAGUGUAAAGAAUGUUCAAAAGCCUUUAGCCAAAGUUCAGCU
CUUAUUCAACAUCAAAUAACGCAUACUGGAGAGAAACCCUACAUAUGUAAAGAAUGUGGAAAGCCUUUA
CUCUCAGUACAUCCCUUUAUAAACAUCUAAGAACCCAUACUGUGGAGAAAUCCUACAGAUGUAAAGAAUG
UGGUAAAUCCUUCAGCCGAAGGUCAGGCCUUUUUAUACAUCAAAAAAUUCAUGCUGAAGAAAACCCUUGU
AAGUAUAAUCCGGGUAGGAAGGCAUCUAGUUGCAGCACAUCCCUUUCUGGAUGUCAAAGAAUUCAUUCUA
GAAAGAAGUCCUACUUAUGUAAUGAAUGUGGCAACACCUUUAAGUCUAGCUCAUCCCUUCGUUAUCAUCA
GAGAAUUCACACUGGAGAGAAGCCUUUUAAAUGUAGUGAAUGUGGGAGAGCCUUCAGCCAGAGUGCCUCU
CUUAUUCAACAUGAAAGAAUUCACACCGGAGAAAAGCCCUAUAGAUGCAAUGAAUGUGGAAAGGCUUUA
CUUCUAUUUCACGACUUAAUAGACACCGAAUCAUUCAUACUGGAGAGAAGUUUUAUAAUUGUAAUGAAUG
UGGUAAAGCCUUAAGCUCCCACUCAACACUUAUUAUUCACGAGCGAAUUCAUACUGGAGAAAAACCAUGU
AAAUGUAAAGUAUGUGGAAAAGCCUUCAGACAGAGUUCAGCUCUCAUUCAACAUCAGAGAAUGCAUACUG
GAGAAAGACCCUAUAAAUGUAACGAGUGUGGAAAACAUUCAGGUGUAACUCAUCACUUAGUAAUCACCA
GAGAAUUCAUACUGGAGAGAAACCAUAUCGAUGUGAGGAAUGUGGGAUAUCUUUUGGCCAAAGUUCAGCU
CUUAUUCAGCAUCGAAGGAUUCAUACAGGAGAAAAACCCUUUAAAUGUAAUACAUGUGGAAAAACUUUUA
GACAAAGCUCAUCACGUAUUGCACAUCAGAGAAUUCAUACUGGAGAGAAACCCUAUGAAUGUAAUACAUG
UGGGAAACUUUUCAACCAUAGGUCAUCCCUUACUAAUCAUUAUAAAAUUCAUAUCGAAGAGGACCCCUAG
AAAGUAGAUUUGUAUGUGUGAAAGCCUUAAACCAAAGCUCAUCGAAGAAUACAUCCUUGAGAGAGAUGUA
AUAAAUGUAAUGGAUGUGAAAAAAACUGUAAUAAUUUAGCCCUCAUUAGGUAUUUAAUUCCAUGGAUAAA
CCUCAGCUAUAUAAUAGAUAUGAGGAAAGUGUUUGUGCCUGUCAGACACUUAAAAAAAUAACCUGAGAUG
AAGAAUUUACAAUUGAAGACAUUGACUUUAGCCAUUUGUGAAAUGGGUUUGCUUUUUCCCUUUUCCUACA
GACGUAUAUGCUAGAUGUCACGUGAUCAUCAGAAACAGAUAUCCGAGUGGGUGGGGAGGUGUGCUUUAGA
UUUCUCAUUAGAAGACCACCAAACUGGUAAUAUUUUUAUAGCAUUUUAAUAGCUUGAUCAAAUUGUACCU
UUUUAGAGAAAAGGACCAAAAUAAAAGAAAAAUGAAUUAUGAACUACCUCUCAGUCUCUGGGGUUUGUCC
UUUUCCUACCCUGAUGUCAAACUUAUGCAUGGAUUUCAUUAAAAAAGAAAAAGAAAAA
- **SEQ ID NO: 14**
   **Homo sapiens zinc finger protein 354A (ZNF354A), Translated Protein of transcript variant X1** 
MAAGQREARPQVSLTFEDVAVLFTRDEWRKLAPSQRNLYRDVML
ENYRNLVSLGLPFTKPKVISLLQQGEDPWEVEKDGSGVSSLGSKSSHKTTKSTQTQDS
SFQGLILKRSNRNVPWDLKLEKPYIYEGRLEKKQDKKGSFQIVSATHKKIPTIERSHK
NTELSQNFSPKSVLIRQQILPREKTPPKCEIQGNSLKQNSQLLNQPKITADKRYKCSL
CEKTFINTSSLRKHEKNHSGEKLFKCKECSKAFSQSSALIQHQITHTGEKPYICKECG
KAFTLSTSLYKHLRTHTVEKSYRCKECGKSFSRRSGLFIHQKIHAEENPCKYNPGRKA
SSCSTSLSGCQRIHSRKKSYLCNECGNTFKSSSSLRYHQRIHTGEKPFKCSECGRAFS
QSASLIQHERIHTGEKPYRCNECGKGFTSISRLNRHRIIHTGEKFYNCNECGKALSSH
STLIIHERIHTGEKPCKCKVCGKAFRQSSALIQHQRMHTGERPYKCNECGKTFRCNSS
LSNHQRIHTGEKPYRCEECGISFGQSSALIQHRRIHTGEKPFKCNTCGKTFRQSSSRI
AHQRIHTGEKPYECNTCGKLFNHRSSLTNHYKIHIEEDP
- **SEQ ID NO: 15**
   **Homo sapiens zinc finger protein 354A (ZNF354A), transcript variant X3, mRNA, NCBI Reference Sequence: XM_054353339.1**
   >XM_054353339.1 PREDICTED: Homo sapiens zinc finger protein 354A (ZNF354A), transcript variant X3, mRNA/cDNA 
UCCUGCCACUGCGCUACUGCACUCCAGUCUGGCCAACAGAGCGAGACUCCGCCUCAAAAAAAAAAAAGCC
GUUUUCCGUGGAGUCGGGAGAUGAUUCGUUGGCGGGAGAGAGAGGAACCGGCUGGGAAAGGCUUGAGGCG
GAGGGAAGUCGUCCCGGGGCCGUCGUGGUGGGAGUCCCGGCCCGCCUCGCAGCGGCGUGAACGGGGCAGG
UGCCCCGGCGCUGCCUCAGUUUCCUGAGCGGUGCAGUGGGUGCUGGCAGUGCCAGCUGGCGCCGAGGAAC
UCAGCGGCGUGGGGCGAGCCCUGGCCCUGGUGGGCUCAGCGGGUCGCUGCUGCCACUGCGGCUCCAGCGU
CCCCUCCGUAAGCCCCAAGCCUGUGGGGCCUGGGCCUGGCCGGGCGGCCCAGCGCUGCUCUGGUCCGCGG
GCUCCUGGCUCCUCCCGAAUCCCUGUGAGGGCGCGCGGGGUCCUUCUCAGCGGGAGUCGGGGUUUUAGAG
CUGCGGAUUCCAGGGCUGGAAAGCAGAAGGGGUUCUUCCUGGCUCCCUUUUUCUUCUCAGAUCUGCCUUC
UGGAGACUGCGCCGUCCUCCCGGGAGAGCCAGAAAGAGGACAUGGCUGCUGGGCAGCGGGAAGCGAGGCC
CCAGUGGCUGCACUAGGCCUGAAUCUCACUGUGUGCUGCUCAGUAUACAUUUGUUACUGGCCAAUUAUUC
AUUAAACGAAUGAUCUGGAACCUUCAGAAGCUUGAAUAGUGUCACUGACGUUUGAGGAUGUGGCUGUGCU
GUUUACCCGAGAUGAGUGGAGAAAGCUGGCCCCUUCUCAGAGAAACUUGUACCGGGAUGUGAUGCUGGAG
AACUAUAGGAACCUGGUCUCACUGGGGCUCCCAUUUACCAAACCAAAAGUGAUCUCCCUGUUGCAGCAAG
GAGAAGAUCCCUGGGAGGUGGAGAAAGACGGUUCUGGCGUCUCCUCUCUAGGAUCGAAGAGCAGUCAUAA
AACCACAAAGUCAACGCAAACACAAGACUCUUCAUUUCAGGGACUGAUACUGAAAAGAUCCAACAGGAAU
GUACCUUGGGAUUUGAAAUUAGAAAAGCCUUACAUAUAUGAAGGCAGAUUAGAGAAAAAGCAGGAUAAAA
AGGGAAGUUUUCAGAUAGUUUCAGCCACCCACAAAAAAAUCCCCACUAUAGAAAGAAGCCAUAAAAAUAC
UGAAUUGAGCCAAAACUUCAGCCCAAAGUCAGUGCUUAUUAGGCAACAGAUACUUCCCAGAGAAAAAACA
CCACCAAAAUGUGAAAUACAAGGAAACAGCCUCAAACAGAAUUCACAAUUACUUAAUCAACCAAAAAUUA
CAGCAGAUAAACGCUAUAAAUGUAGUCUGUGUGAAAAAACCUUCAUUAACACUUCAUCCCUUCGUAAACA
UGAGAAAAACCAUAGUGGAGAGAAACUAUUUAAGUGUAAAGAAUGUUCAAAAGCCUUUAGCCAAAGUUCA
GCUCUUAUUCAACAUCAAAUAACGCAUACUGGAGAGAAACCCUACAUAUGUAAAGAAUGUGGAAAGCCU
UUACUCUCAGUACAUCCCUUUAUAAACAUCUAAGAACCCAUACUGUGGAGAAAUCCUACAGAUGUAAAGA
AUGUGGUAAAUCCUUCAGCCGAAGGUCAGGCCUUUUUAUACAUCAAAAAAUUCAUGCUGAAGAAAACCCU
UGUAAGUAUAAUCCGGGUAGGAAGGCAUCUAGUUGCAGCACAUCCCUUUCUGGAUGUCAAAGAAUUCAUU
CUAGAAAGAAGUCCUACUUAUGUAAUGAAUGUGGCAACACCUUUAAGUCUAGCUCAUCCCUUCGUUAUCA
UCAGAGAAUUCACACUGGAGAGAAGCCUUUUAAAUGUAGUGAAUGUGGGAGAGCCUUCAGCCAGAGUGCC
UCUCUUAUUCAACAUGAAAGAAUUCACACCGGAGAAAAGCCCUAUAGAUGCAAUGAAUGUGGGAAAGGCU
UUACUUCUAUUUCACGACUUAAUAGACACCGAAUCAUUCAUACUGGAGAGAAGUUUUAUAAUUGUAAUGA
AUGUGGUAAAGCCUUAAGCUCCCACUCAACACUUAUUAUUCACGAGCGAAUUCAUACUGGAGAAAAACCA
UGUAAAUGUAAAGUAUGUGGAAAAGCCUUCAGACAGAGUUCAGCUCUCAUUCAACAUCAGAGAAUGCAUA
CUGGAGAAAGACCCUAUAAAUGUAACGAGUGUGGAAAACAUUCAGGUGUAACUCAUCACUUAGUAAUCA
CCAGAGAAUUCAUACUGGAGAGAAACCAUAUCGAUGUGAGGAAUGUGGAUAUCUUUUGGCCAAAGUUCA
GCUCUUAUUCAGCAUCGAAGGAUUCAUACAGGAGAAAAACCCUUUAAAUGUAAUACAUGUGGAAAAACUU
UUAGACAAAGCUCAUCACGUAUUGCACAUCAGAGAAUUCAUACUGGAGAGAAACCCUAUGAAUGUAAUAC
AUGUGGGAAACUUUUCAACCAUAGGUCAUCCCUUACUAAUCAUUAUAAAAUUCAUAUCGAAGAGGACCCC
UAGAAAGUAGAUUUGUAUGUGUGAAAGCCUUAAACCAAAGCUCAUCGAAGAAUACAUCCUUGAGAGAGAU
GUAAUAAAUGUAAUGGAUGUGAAAAAAACUGUAAUAAUUUAGCCCUCAUUAGGUAUUUAAUUCCAUGGAU
AAACCUCAGCUAUAUAAUAGAUAUGAGGAAAGUGUUUGUGCCUGUCAGACACUUAAAAAAAUAACCUGAG
AUGAAGAAUUUACAAUUGAAGACAUUGACUUUAGCCAUUUGUGAAAUGGGUUUGCUUUUUCCCUUUUCCU
ACAGACGUAUAUGCUAGAUGUCACGUGAUCAUCAGAAACAGAUAUCCGAGUGGGUGGGAGGUGUGCUUU
AGAUUUCUCAUUAGAAGACCACCAAACUGGUAAUAUUUUUAUAGCAUUUUAAUAGCUUGAUCAAAUUGUA
CCUUUUUAGAGAAAAGGACCAAAAUAAAAGAAAAAUGAAUUAUGAACUACCUCUCAGUCUCUGGGGUUUG
UCCUUUUCCUACCCUGAUGUCAAACUUAUGCAUGGAUUUCAUUAAAAAAGAAAAAGAAAAA
- **SEQ ID NO: 16**
   **Homo sapiens zinc finger protein 354A (ZNF354A), Translated Protein of transcript variant X3** 
MLENYRNLVSLGLPFTKPKVISLLQQGEDPWEVEKDGSGVSSLG
SKSSHKTTKSTQTQDSSFQGLILKRSNRNVPWDLKLEKPYIYEGRLEKKQDKKGSFQI
VSATHKKIPTIERSHKNTELSQNFSPKSVLIRQQILPREKTPPKCEIQGNSLKQNSQL
LNQPKITADKRYKCSLCEKTFINTSSLRKHEKNHSGEKLFKCKECSKAFSQSSALIQH
 
QITHTGEKPYICKECGKAFTLSTSLYKHLRTHTVEKSYRCKECGKSFSRRSGLFIHQK
IHAEENPCKYNPGRKASSCSTSLSGCQRIHSRKKSYLCNECGNTFKSSSSLRYHQRIH
TGEKPFKCSECGRAFSQSASLIQHERIHTGEKPYRCNECGKGFTSISRLNRHRIIHTG
EKFYNCNECGKALSSHSTLIIHERIHTGEKPCKCKVCGKAFRQSSALIQHQRMHTGER
PYKCNECGKTFRCNSSLSNHQRIHTGEKPYRCEECGISFGQSSALIQHRRIHTGEKPF
KCNTCGKTFRQSSSRIAHQRIHTGEKPYECNTCGKLFNHRSSLTNHYKIHIEEDP
- **SEQ ID NO: 17 - Genomic sequence**
   **https://www.nchi.nlm.nih.gov/gene/6940**
   >NC_000005.10:c178730659-178711512 Homo sapiens chromosome 5, GRCh38.p14 Primary Assembly 
AGACGCCGTCGGGGCGGAGCCGGGTCGCGAGGCTGCATCCCGGCCGGCGGTTCCGGAGCCTCGCGGCTGG
GGAGGCGCGGCCCGGGACGCTCGAGCTTAGGGAGGTGAGCGTCGGCGGCAGGCCAAGCCGGTAGGAGGCG
GCGCCCGGCACGGGGACTGCCTGCGGCTGCCGAGGCTCCGAAAGCCTTTGTGGGCGCTGGCGGGCACCCC
CGCGTCTGTCACCGGCCCCCGCGCCGGGATCTGGACTCTCGCCCCCCCGCGGGGCGCTCCCTCGCCGTTG
CTGGTGCACCCGCGTCCGGCTCGCCGTGGACGCCGGGCCTGGCGTTTCCTCCCGGGCCCGTCCTGCCCAC
TCCATGTGGGCCTCGCGGACTCGGGGCACCGGGACCCCGAGGGCAGGGCGGACGCAGCCGGGCCCCGGTT
TCCGAGTCCGTCCCCAGGACGGCCACCCCTCCCGCCTCGGGACACCCAGCGCTTTCCTCTTCAGAGCTGG
TGTTTGGCCGGGCGCGGTGGCTCACGCCTGTAATCCCAGCACTTTGGGAGGCCGAAGCGGGTGGATCACG
AGGTCAGGAGATCGAGACCATCCTGACTAACACGGTGAAACCCCGTCTCTACTAAAAATACAAAAAAAT
TAGCCGGGCGTAGTGGCGGGCGCCTGTAGTCCCAGCTACTTGTGAGGCCGAGGCAGGAGAATGGCGTGAA
CCCAGGAGGCGGAGCTTGCAGTGAGCCGAGATCAAGCCCTGCACTCCAGCCTGAGCGACAGAGCGAGACT
CCGTCTCAAAAAGAAACATCGTGTTAGAAATGGGATACATGGGGTCGGGCGTGGTGGCTCACGCCTGTA
ATCCTAGATTTTTGGGATGCCAAGGCGGGCAGATCACCTGAGATCAGGAGTTCGAGACCAGCCTGGGCAA
CACGGTGAAACCTCGTCTCTACTAAAAATACAAAAATGAGCCGGGCGCCTGTAGTCCCAGCTACTCGGGA
GGCTGAGGCAGGAGAATGGCTTGAACCCGGGAGGCAGAGCTTGCAGTGAGCCGAGATCCTGCCACTGCGC
TACTGCACTCCAGTCTGGCCAACAGAGCGAGACTCCGCCTCAAAAAAAAAAAAAGCCGTTTTCCGTGGAGT
CGGGAGATGATTCGTTGGCGGGAGAGAGAGGAACCGGCTGGGAAAGGCTTGAGGCGGAGGGAAGTCGTCC
CGGGGCCGTCGTGGTGGGAGTCCCGGCCCGCCTCGCAGCGGCGTGAACGGGGCAGGTGCCCCGGCGCTGC
CTCAGTTTCCTGAGCGGTGCAGTGGGTGCTGGCAGTGCCAGCTGGCGCCGAGGAACTCAGCGGCGTGGGG
CGAGCCCTGGCCCTGGTGGGCTCAGCGGGTCGCTGCTGCCACTGCGGCTCCAGCGTCCCCTCCGTAAGCC
CCAAGCCTGTGGGGCCTGGGCCTGGCCGGGCGGCCCAGCGCTGCTCTGGTCCGCGGGCTCCTGGCTCCTC
CCGAATCCCTGTGAGGGCGCGCGGGGTCCTTCTCAGCGGGAGTCGGGGTTTAGAGCTGCGGATTCCAGG
GCTGGAAAGCAGAAGGGGTTCTTCCTGGCTCCCTTTTTCTTCTCAGATCTGCCTTCTGGAGACTGCGCCG
TCCTCCCGGGAGAGCCAGAAAGAGGACATGGCTGCTGGGCAGCGGGAAGCGAGGCCCCAGGTGAGCTCAT
TGCCCTCCCAGATCCCAGTGGATGGATTTTCCCCACTAACTGGAGTTCTTCACCCGGGACCATCTCCAGC
CAGGATGGAGCCCAAGTCCTTTTCTCTTGGAGGAGCTGGTCCGCCCTCATGAGTGGTGGATTCTGTAGGG
AGGACCACCCACCCTCCCCACCCGTGTCTAGTGGTTCTCTTTTTTTTTTTTTTTTTGAATCTCGCTTTG
TCGCCCAGGCAGTGGCGGGATCTCGGCTCGCTGCAACCTCTGCCTCCCAGGTTGAAGCAATTCTCCTGCC
TCAGCCTCCCAAGTAGCTGGGATTACAGGCTCCCGCCATCACGCCCGGCTAAGTTTTTTTTTTGTGCTT
TTATTAGAGATGGGGTTTCACCACGTTGGCCAGGCTGGTCTCAAACTCCTGACCTCAGGTGATCCACCCG
CCTCAGCCTCCCAAAGTGCTGGGATTACAGATGTGCGCCACTGCACCCAGCCCGAATAAATCATTTTCTG
TACACATTTATTTGTGTGTGTGTGTTTGTGTATGCATGTTACCATCTATGTTACCATTTTAACCTTTTTT
TATTGTGGCAAAATATATATAACATAAAATATACCATTTAACTTTTAAGTGTATAATTCAGTGGTATT
TTAGGTACTTCCGCATTGCTGTGCAGCCATCATCGCTATCCATCTTTATTGTGTAGATTATCACATTTAT
TTCTTAACCCTAAGGGGCAGGCATTAAAAGGCAGTTTTACAGAGTAGTTAAGAATGCAAGCTCAGTGAGA
CATCTGCTGGATTTGAATTACACAAGAAAAAACAAAGTCACAGAGAGGATACAAAATTTGTAGAAGTCAG
CCGGCCACAGTGGCTCACACCTGTAATCCCAGCACTTTGGGAGGTCGAGGCCGGCAGATCGCCTGAGGTT
AGGAGTTTGAGACCAGCATGGTGGAACCCTGTCTCTACTAAAAATACAAAAATTAGCCGGGCATGGTGTC
TAGCACCTGTAATCCCACCTACTCAGGAGGCCGAGGCAAGAGAATCATTGGAACCCGGAAGGTGGAGGTT
ACAGTAAGCCAAGATCATGCCATTGCACTCCAGCCTAGGCAACAAGAGCCAAACTCTGTCTCAAAGAAAA
AAAAAAATGTGTACAAGTCACACAGCTGGTCAGTGACTGTGTCTGTGTGACCTGGACAAATTTCTTATCT
TGTCTGTGACTTTGTTTTCTCTTGTGTAAAGTGGCAACAATAATGGTATCGTCTTGATAGGATTGTTGTG
AATGTTAAATAAAGATTATACACTGCAAATTAACATAAAGCTCTAAGATCAATACTTGGCCCATAGAAAG
CCTTCAATAAATTATAGCTATTTACTTTTCGTCATTACTATCCTTAAGCTTGAGTAAGCACATAGAGAG
TACCTTCAGTTGCTTAACCTAACCTCCATCATTAAGTATTTAGATTATTCCTGGTTTTTTGCTCTTAAAA
ACACCATGAATATGTTTGTGTGTACTTTTGTGCACTGTTCATTATCGTACAAATAGAGTAATTGGTACTT
 
TAAAGAGTATACTTTGAGGGTTCTAATATGTATTAGCAAATCGCCATCAGAAAGAGTTTTCTAATTAACA
CTCCCACTAATGGCCGTAATTCCTCTACCCATCTCTTACAGTGGCTGCACTAGGCCTGAATCTCACTGTG
TGCTGCTCAGTATACATTTGTTACTGGCCAATTATTCATTAAACGAATGATCTGGAACCTTCAGAAGCTT
GAATAGTATGGGAAGTGTTTCACGGGTAGCGTGTCTTTCCCACTGCCGCCCCCTCTGCTCTGTGAGGGTG
GTCCTGGGTGAGCTGGAACGACTTGTCCTTACAGGTGTCACTGACGTTTGAGGATGTGGCTGTGCTGTTT
ACCCGAGATGAGTGGAGAAAGCTGGCCCCTTCTCAGAGAAACTTGTACCGGGATGTGATGCTGGAGAACT
ATAGGAACCTGGTCTCACTGGGTAAGGAAATTTTCCCTCTAGAAACAGAATTCAAAAATTGGGATATCTC
AGCACCTCCTTCCCTGAATAAAAGCGGTTGGTCACTGAAAAATTTGAGCTGAGTGTGTGAGGATTGTACA
AATCAGATCTTTATAAATAAACATTAAAAACCTATTCCCCCTTATAGGCATGAAATTGCCCTGTGTCTGG
GAATGGAAGAGGTGGTATTGATAATCATTCCTCTCTCTCTATGTAATCACACACTCCAAGTTTGTCCATG
ATTCAGGCATCGTTTCCCAACGATTCTGTAGTTTGCTTGTCTCCCAACAGGGAGCTTCATTCAGCCTCTA
GGCCTACCACCTGCCTTTCCCACTTGTCTTTTCCAGGGATTAGCAAACTTTTACTATAAAAAGCCATGTA
GGCTGGGCATGGTGGCTCACACCTGTAATCCCAGCACTTTGGGAGGCCAAGGCGGGCAGATCATGAGGTC
AAGAGATAGAGACCATTCTGGCTAACGTGGTGAAAGCCTGTCTCTACTAAAAAACACAAAAAAAATTAGC
TGGGTGTGATGCCGGGCGCCTGTAGTCCCCGCTACTTTGAAGGCTGAGGCAGGAGAATGGCGAGGAGCTT
GCAGTGAGCCAAGTTCCCGCCACTGCACTCCAGCCTGGGCGACAAAGCGAGACTCTGTCTCAAAAAAAAA
AAAGCCACGTAGTAAAGACTTTAGGCTTTGTGGGCTGCGTAATGGTCTCCGTAACATATTTCTCTTTTTT
TTTCCCACCATTCTTTGCACAGGGCCATACATAAACAGGCAGTGGTCTGAATTTGGCCCATGACCCGTAG
TTTGCTGATCACTGCCATCCTTAAAAGGGAGTCACATAGGCCGGGCGCGGTGGCTCACACCTGTAATCCC
AGCACTTTGGGAGGCCGAGGCGGGCAGATCACAAGGTCAGAAGATCAAGACCATCCTGGCCAACATGGTG
AAACCCTGTCTCTACTGAAAATACAAAACTTAGGCGTGGTGGCATGTTCCTGTAGTCCCCGCTACTTGGG
AGACTGAGGCAGGAAAATCACTTGAACCCGGGAGACGGAGGTTGCAGTGAGCCAAGATTGCGCCACTGCA
CTCCAGCCTGGCAATAGAGCGAGACTCCTCCTCAGAAAAAAAAAGCGGAGTCAGATAGTGTTTTCTCGTT
TTAGACAAGCTACCTGGTACATAATCCTTTTCATAGATAACCTACTTAGAAATTCTAATCAGTCTGTATA
TCTTAACATTTCCTCTTCTATAGAAAGCTAGTGGTATCCTATTACCAATACTGCATGTTGATCATATTTT
CCTAACCTATAATTAATTGTCCCAGAGTCATGTCCTACAAGTCACTTGTCTTTTCCTCACCCTACCGCCT
TACCCCCTTACCTCCCAGGGGCACAACTGAAGCCCTACACAGAAGCTCTTTGTACCATCCTGTGAGTTCT
GTATTTAAATTTGGGATAACTGTATCAATACAAAACCAAGTCTGATTTGTTTGGTTGTGGTTTTTTGTTT
ATGTGCAGGGCTCCCATTTACCAAACCAAAAGTGATCTCCCTGTTGCAGCAAGGAGAAGATCCCTGGGAG
GTGGAGAAAGACGGTTCTGGCGTCTCCTCTCTAGGTAAGTGGGTGGCCCGAGGTGCGGAATGGCCGCAGA
CAATGGTCTGGTTAATGAGAGGAGGTAGGAACGTTGGTTGGGAAACTCCCTTGAGAATTCTCAGGCCTCA
AGAAGTGATGGGGAAGCGGAAGCCCAGGCTCTTAGGCAGGATTCACTTCCCCATGAATCTCTCAGAATCT
CTTTCTTACTCTTTCCTGTTGTTTTAGAATAGGGCTGTATTTTCATGTATTCATTCAGCAAACATAGTCT
ATTTAAGGAATTTCAAATAATCACTCTGGCTGAAGCTCAGGGTGTATTTGGGAAAGGAGGAGGTGAGGAA
GGAAATAGCCTAGAAAGCTAGGGAAGGGCCGCGTGGTAAGTGCTTGGGCACGGTTCTAAGAGGTGGAAAC
TGTCCCATGGAGCACTGGAAATAATGGAAGGATTTGGGCAGGAATCATATGGTCCTTGGTATCTTAGAAA
AGTTGGTGTGAATCAGAAACAGCTGAGTCTCCATCCGTGTAAGAACAGCTAAGGGACCTGTGAGCATCAC
CACCCTGAAAACAAGAGTCCAGGGACGTGGACAGAGCTCCCAAAAAGTTACAGAGCATTGTTAGGACGCC
ACCATTTATGTCTAATGAAAATTATGTGTATGTGTAATGTGCACATAAGCAGAGAGAAAGGTTAGGAGGA
CACACCTTAGCTGATGACATTAGTTACACTTGGAATGGGATTGAGAATGGGTCCATGGAAGGGGGCATTA
CTTACATGTAGTATTTTAATGTTTCACAAAGAGAATATATTTGTGACGTTCTTATGTGATTTGAATTCAC
AAGAATGGTCACTTTGGAAGCATCGTGTTTGAAAGTGAGGCAGAACGCTGGGCACGGTGGCTCACGCCTG
TCATCCTAGCACTTTCAGAGGCCGAGGCGGGCGGATCACCTGAGGTCAGGAGTTTGAGACCAGCCTGGGC
AACATGGCAAAACCCTGCCTCTACTAAAAATACAAAAATTAGCCAAGCGTGGTGGGGCAGGCCTGTAATC
CCAGCTACTCCGGAGGCTGAGACAGGAGAAACACTTGAACCCGGGAGGCGGAGGTTGCAGTGAGCCGAGA
TTGCGCCACTTCACTCCAGCCTTGGCGACAGAGTGAGACTCTGTCTTAAAAAAAAAAAAAAAAGGCGGGAA
GCCTTGCAGAGCGATTGCAGTCATGGAGGGAGGCAGGAGGTGGAAGGGCCTGGATGAAGGCACTTGCCGT
GAGTTCGTGAGGGAGGAGGCGGCGCCCACGTGATCAGGGTACAGGAGCAGTTGGGTGTGGGATGGATTTC
ACTTGAGATGTGAGTGAAGTCGTCAGGTGGGGAACCCTGGTGGGCAGGTGTGTAAGTGACAGGGCCGAGA
CTCAAGGGAGGTCAGGGAGCCCATTAGCATGATGGGCATTAGAAGCTTGGAGCTGGCCAGGTGAGGGGGC
CAAGTGGAGGCCCGAATTTCAGGAAACAGCATTTGCGAGGTGCTTAAGCAGGAGGAATCTGACAGAGCCC
GAGAAGTAATCAGAGTAGAGAAACGGGGAGGGGCCATCGTGGCAGTGGGGGACTGAGAGTGGTTGAACTG
GGGGGGCTCTGGCTCTGGGGGATCAAGGTTGAACCCTCGCCCACAGCTTTGTATAGCATTGGGCAGAAAG
GGACACAGGTCTCCTGCTTCTGGAGTGCACGATCTAGTGGGGGACAAGGTGAACACACTCATTGCACACA
AGGCACAGAAGGACACATTCCAGTGAGGACCACGTGGGAAAGACACTCGGTGCTGTGAGAGTGCAGAGCA
GGCAGGTGGCGTCAGCAAAGGCTTCCCTGTGGAAGGGCCTGGGATCAAAGGACTGAAGAATTGCTCAGGC
CTAAAGAAAGGTGTTCATGAAGGTCCAGAGAAGCAAAGCCAGCGAGGAGAAGGTGGAGCACCCGAGAGAG
AGCCACGGGCCCTGTCACAGGGTTTGGTTGGTGCTTGAGAGCCAGGCACAGGTTCTGAGGAGCCGTAAGT
AGCCCAGGGCAGGCCTAGGCTGCTTTCTGCAGCCGCCAGCCCATGTGGAGAACCTGACGGCCACAGTGCA
GGGCTTGTCAGGTCCAGAGGGCTCAGTGATGGCCCCTTATGCCATGGGCCCAGCAAGGAGCTGCAGGGCA
GGCAGCCTCGGTCCCCACAGCCCCTGTGCTGGGGAGAGCTTTCTCCTCCCTGAAGAAAGGTGAGATTTTT
 
TTATATGGGAACCACACTTGTTTATCTTTTTAACTCTCCTTTGGGAGAAATACACTACGTGGTCTGGCCC
TGGCTACCTCTTCACTCTCAGCTCCTTCCACTCTGCCCTTCATTCGAGGCCTCTGATCACCCTTGGCCGC
CCTGCAGTGTGTCATGGGGGGCGCCAAGCAGGCCCCCACTCAGGGCCTCTGCCCTGCTTTTTCTGTCTGG
GACTGTTCTTCGTGGCGGTCATGCGTCCATGTGGCTCGCTCACTTCCTTGTGCTGCACAGGCTTCGGCTC
ACACAGGCACAGATCATCTGACACGCAGCCCTGCAGCCCCCACGCCATCTCCTCACCGTGCTTTATTTTC
CCTCCTCGTGCTTAGCGCTGTGTAATAGATCTGTTTCTTCATGATCTGTCTCTGCCTCTGCATTGTAAGC
TGCTGTGGGAGGGGTTGCCTGTTTCATTCACTCCTGTTTTCCAAGAACCTAAAACAGTCCCTGGCCAGGG
TATGCGTCCAGTTGTATAATGAATGCACGAGTGACTTGGACTAGGTTAGTGGTGTTGGAGACAGAAAGAA
GTGAACCAGTCCCAGGGATGTCCAGGAGATACAGCCAGCAGGACTGTTAGACATGGAAGATAGAGGAGAT
GGGGTAACAAAGAAAATAAAAGCAAACAAAAGGGAGATGGGGTTGTCAGGGATGCTTGCTTGGTTTCTGG
TTTGTTCTGTGACGGTTGGTGATGTCATTCAGTGAGATAGGAACACTAGAAGGAGATCAGATTTGGAGAT
GATGTAAATTTGGTTTTAGATTAATTGAGCTGTTTTTGGTTGACCTAGTTATCCCAGTGGCGATGACAAG
TAGTGTTGATTTCGGAGTACAGTACCTCAAAATGTAGACTGAGCTTGAAATGTAAATTTGGGAGAACCTG
ATCAATATAGGGTGATTGAAATAATGGGAGAGGATGAGTTGGCCTAGGGAGAGTGTTCACATTACAGTAA
GAGTAAGAAAGAGCCTAAGACTGAATCTTGAGGAACTCCAGCATTTCAGGAAGCCAGGCAGAGAGAAATG
AACCCATAGGGAAGACAGGTAAAAGCGTACTGTAAGGAAAATCAGGAGAGGCAGGTGACATTGGAGGCAA
GGCTCATGATGGAATGGTAGCAGTAGATGGGGAAAAGTAGACGAATGTGAGGCAGATTTAGATGGCAGAA
TAAACAGGTCTTGGTAATTGATTAGATGAGGGGGCTGAGGGAGGGAGAATTATTAAGGATAACTCTTAAG
GTTTTGATTCAGAGGTGGTGCCATTTACAGAGACAAGGGGTTGACCAGGTCGGAGGGAAGAATCGTGAGT
GTGGTTTTAGGTGTGTTGACTTTGAGGCATCTGAACTGCTCTTTTGAGTAGGTCGTCTGTAAGGGGGAGT
CGAGTATGGGAGATATCTTGCTTAGAGGATGAGTCTATGATTGAAGTTGCCTGTGGAAAGATGCAGAGTG
AGAAGAACTGGGGGCCTAGAACTAAAGCTAAGGCACTCCAAGGATGATAAACTGTGGTACGTCCACACAG
CTGTAAAGTGGAATGAGGAGGACCTCTGTAACTTCTACTATGGAGTGATCTCCAGAATGTGAGAATAAGT
GCAGAACAGCACAGTTAGTACTGTTATCTAAGAAAGGTGGGTGGGAATACCAAGAAAAGTGTGTGTGTAT
TTACTTACACTGTCATGGAGCACATAATGTTTCAGTCAATGATAGCCTACATATATGATGATGCTCCCAT
AGATGATAATACTGCATTTTTCCTGTCCCTTTTCTATGTTTAATATGTTGAGATGCACAAATACTTACA
ATTGTGTTACAGTAGCCTACAGTATTCAGCTAAGTAACATGCTGTATAGGGGTATAGCCTAGGTGTGTAG
TAGCCTATACCATCTAGATTTGTGTAACACACTCTGATGACTGCACAACATCGCCAACAACGCGTATCTC
AGAACATATCCCAGTCATTAAGCAGTGCAATTATTTTTTTAGAAAGATAAAAGCAAAAGAAAAAAAAGG
AGATCAATAAAAATGGTTATCTCTAAGGTGAAGGAAGAAAAAGGATGGAACAGGGGTAGAATCTATGACT
TTTTGAGGTCTCAAAATTTTGACTTCAGAACTATAAAAATGGTTTATAAAATTATAAGACAAAATTAAAC
AAAAATGAGAAAGTACCCCCTACATATTGAAAGCAGAATGAAACAACCCTAATTGTGTTCCAGGTTGGTG
GCTTAGCCACAAAAAGAATTATTTGAATTGCCTTTACATTGCAGCAGTTTGACTTATTAGTGTAGGTATT
CTAGGGGCAAAAGTAACTGCAAGCAGTGGTGTTAATTTTGATGGATGTGCTAAGAGCGGTTTTGATGTTT
TTACAGATGAAATGATTTTGCTGTCTGGGATGTGCTCTAGCATAACCTAGTGGACGAGATGAAAGGGTTT
GTGTTAGTTTGCTAGGGCTCCATACTGAAGTACCACAAGCTTCGTGGCTTAAAACAGTAGAGATTTACTG
TCTCACAGTTCTGAAGGTCAAAATCAAGATATTCGCAGGGCCCCGCTCCCTCTGAGACTCTGCATAGAAT
TCTTCCTTGCCTTTTGCTAGCTTGTAGCGATGGCCAACTTGGAGATCTCCGTGGCCTGCAGCTGCATCAG
TCCTATCTGTGCCTCCGTCTTCACACTGTGTTCGCCCGGTGTCTTCACATCATCTTTCCTTCTGGATGCC
TGTGTCCAAATTTCCCCAGTTTACGGGGACAGCAGTCACGCTGGATTAGGGCTCACCCCAATGACTTCAT
CTTAATTTGATTGAACGTGCAAAGACCATATTTTAAAGTAAGGTCACGTTCTGAGGTCCTGGAAGTTAGG
ACTTCTACGTCTCTCTTTTGAATGATGCGGTTCAGCCCATAACAAGGTTGTCAATAGAATAGTGTTGCTG
GTTATTGAAACTGGCTGGAGATTCACTGTACAATTCACACTACTTTTACATATGCTGAAAATTTCTATGT
TAAAAGCTTTAAAAATAAGAGTTACCACAACAACATTTCTAAAACAGCTTTTCTCTTTTCCACCAGCACA
AAGATGATAGAGGATGTTTACAAATAGGCAGTACTACCCCCATCATGGTTTATGTGCAGTTCCCAGCAGA
TTGCTCCTTGAGTTCTCTGACTCTAGAAAACCTTGTGAGACTGCAAATGCAAGTGACATTTTTTTTTTTT
TTTGAGACGGAGTCTCGCTCTGTCGCCCAGGCTGGAGTGCGGTGGCGGGATCTCGGCTCACTGCAAGCTC
CGCCTCCCGGGTTCACGCCATTCTCCTGCCTCAGCCTCCCAAGTAGCTGGGACTACAGGCGCCCGCCACT
ACGCCCGGCTAATTTTTTGTATTTTTAGTAGAGACGGGGTTTCACCGTTTTAGCCGGGATGGTCTCGATC
TCCTGACCTCGTGATCCGCCCGCCTCGGCCTCCCAAAGTGCTGGGATTACAGGCGTGAGCCACCGCGCCC
GGCCGCAAGTGACATTTTTATAGGTGGAGCTTTTACTCTGACTAGCTATAGGATAAGGTGGGGGTGGATA
CTTCAGCAAATAAGTCTGGAAAACTGACTTGCATTCAAAGACTTGGGTGCTGCCTTCAGCCCTTGTGCTA
CCCCTTCATCTAGCCTATGGCGATTCCTTTCACATCCTATCTTATATGTCAATTCTTACGAAACACTCCA
GCAGCGTCCTGCTGCACCCCTTTTCTACCTGCACCCTCACTATCTAAGGACAGTTGGATATGATTCTAAG
CTCGTGTTCACGTCCATTTCTAGCTTATGTTTCTATTGTAGAAATTGTCACATTGTATTGTGATTACCTA
CTGATGTCTTCATTTCTAATGTCTGACTATAAACTCTCTGAAGAAATCAACTGTTCCTTTTAATTACTGG
ATCCGTAGGGAACTGTGGATCACCGATGCCTAGGATAGAGCCTAATACATAGTAGGTACTTTTTAAATAT
TTGATAAATTAATAAAGTAAGGCGTGACCAATGCTTTTTGAAATAACATGACCAATTCTGTGTATTAAGA
GGGGTAAATCCAGTGGGAAATAATACTGGAAAGGCAAGAAACAGGTCAAAGGAATAACTTAAGATCAGGA
GCAAGTCACTCTACAGTGAGGAGATCCTGAACTTAGACATATTCACGATGGCTTATTAGAGACATTTTAG
TGATGAGATTTAAGAGAATGTGCAGCCTGGGCAACACAATGAAACCCTGTCACTACGCAAGATAAAAATA
 
TTAGCCAAGCTTGGCAGTGTGCACCCGTAGTTCCTGCTGCTCAGGAGGCTGAGGTGGGAGAATCGCTTGA
GCCCTGGAGTTTGAGGCTGCAGTGAGCTATGATGGCACCAGTGCATTTCAGCCTGGGCAACAGAGCAAGA
CCCTGTCTCTCAAAAAACAAAAAAATGAAGATTTTGATGACTGCCTAGGGAAGAGAAATTCTAAGATAAA
ACTAAGAATTTACGGTAAATTAACTGAAGAGATGATGGTGTCATTTTGAACTGGAAAACATATAAAAAAG
GTTTGAAAAGGAATGCTCTGGGTTCAGATTTGGACAGGCTGAATTTAAAAAGAAAGATACACAAGTGGAA
ATTCTAACCGGCAGTTGGAAAGACCTCTGTAAAGTATTTTTCAGGATAGTCGTACACATAAGAGGTGTGG
GAGTAAATGAAATCATCCAGGGAGATAGGGTTGAAGAAACACTCTCTGGGTGTAGTGAGAGTCCAGGTGG
AAGAGTCTAGACCTGATAACAGGAAGTGGTCAGGGCAGTGGCCATCAGCAAGATGATTCTGGCTTCTCAA
CTTCTGACCTAGGTCTTCACCTTGTTTAACAGGTAGCTGAGGACTGGGCTTGATTCTAGGAGGAAGCAAA
GGTTGAACTGCACCTCAGGAGACCTATTTGTAGGCCTCTAGAAAGTTCTAAACAGATCATAAGGAAGAGT
CAGGATATCCACAATCCAAAACTTACGTCTGCTTCTGTGAAAAACATGATTTCAGGACTAAAATATTGGC
ACTTTAGAGTTGGGATGAGTTTTCCAGAATTGATAATGGACTTCCGAGTCGTAATCATATCCATTAAAAA
ATAAAAGCTCCCCATGTCACATTTGCTTACTCTCCTTTAGAAACTGTTGAAAAGGGTTTCTGCTCTTGAT
GTTTCTATTTTCTCACCTCCCATTGTTTCCTCCATGCACTCCAGCTGGGCTTTCTTCCTTATAATTCCAC
TGATAGCTCTGTTATTAAATCCCAAACAACCTCCGTCTTGCCACAATCAGGATTAAGTTTTAGTCCTCA
AATTATTTGCCACAGTTATTCACTCCTCCCTGAAGTACTTCTTCCCTTGGCTTCTGGAACACTGTACTCT
CCTGATATTATTCTTCTTAACCTCGTAATATTTCAGTGTCTAAGTGATTCCTTTCAGTTCTTCAGCTTTA
AATCAAACCCACATGCTGCTGACCCCCAAATGCCTGTCTATAGCTCTGACCTCCCCCAGATCTCCAGAAT
AATAAATACAATCTAGTATTTCAGTATCTTCCTGCAACTCTATTAAGGCATCTCAGCCATCACAAACATA
CCAGAAACAGAATGCCCCCTCAGCCTTGCCCCTTTCCTGGTCTTCCCCTTCTAGCCTTGCCCCTTTCCTG
GTCTTTCCCTTCTTGGTGGTTGGTGGCAGCTCTCAGAATTCTGGCTAGAAACCTAGGCATGATCCCTAAC
TTTCCTGTTTCCCTCTCAGCTTCCTGGGAGTCCTGAGTCTTGTTATTCTTGGTCCCGCTAGCTCTTCCTT
CACTGTACAGCCTGGCATAGTCATCTGTACCTCCAGCCCTTTCTCACGTGAAGAGCTGCAGTAGCTTCTT
CAGTAGCCTCTCCCCTCCCTGCTTCCACTGGTGCCCCAGCGTAGTCGGTCCACGCAGCAGCCAACTCAAA
TGAACACATAATTCCCCAGTGGCTTCCCGTAGGCTTAAATTCCAGTCCTTAGGATGACCTGGGCCTTAGA
GGATTTGTCCCCCACCTCCTGCTCCACTCTTTTGTTTTTATTTTTGTTTACTTTTTTTTTTTTTTT
TTGAGATGGAGTCTCACTCTGTCGCCCAGGCTGGTGTGCAGTCATGTGATCTGGGCTCACTGCAGCCTCT
GCCTCCTGGGTTCAAGCAATTCTCTGCCTCAGCCTCCCGAGTACCTGGGATTATGGGCGTGTGCCACCAC
ACCCGGCTGATTTTTGTATTTTTAGTAGATAACAGATTTCACCGTGTTGGCCAGGCTGGCCTTGAATTCC
TGATCTCAGGTGATGCACCCACCTCGGCCTCCCAAAGTGCTAGGATTGCAGCTGTGAGCCACCACGCCCG
GCCCCGTTCCAGGCTTATTTTATCATTTCACCATTTTCCCTCTTCTCGCTCTACTCTGGCCAGTCTCTCT
GCTGTTCCTAAGTCAGCCTCACTTTTGTGTGTGTTGGTTCCTTTGCCCAGGGAGCTCTTGAGCACTTTCT
TCAGCTATCCACATGACTTTTATTACGTACTTGTTATTTGTCCCTTCATCCCCCTCCTTCCCCAGCACAT
GCGCACACTCCAAAGTATAAGCTCCTTAAAGGCTGGAACCCTGTCTTATTGAATACATTATCTGCATTCC
CCAGAACATTACCTGGCCCATAGTAGGCACTTAATGAGTACTTGTGAATGGAAAGGATGCTGCCAGCATA
TGTAAAGATAAACAAGTCAAGTCATGGAGTGCTCCTGAAGCTGCATGTAGTTCAGCATACCTGGCAAGAA
GCCAGGGAAGAGGGGGTGAGGACAGGTGGATAAAGGTCAGATCAGATAGTACTCTATTCACCATGTTAAG
GACGCCTAAGAGTCTTCAAGACTCTTAAGAGACTTCAGATGTGAATCATTGAAAATCTATTGGAGGACCT
TAAGGAAGTTTGCCACATAGTAAAATATTGATCTTAGAAAGATTGGCCGGGCATGATGGCTCACACCTGT
AATCCCAGCGCTTTGGGGAGGCTGAGGTGGGCGGAACATGAGATCAGGAGTTCGAGACCAGCCTGACCAA
CATGGTGAAACCCTGTCTCTACTAAAAATACAAAAATTAGCCAGGTGTGGTGGCGCACACCTGTAATCCC
AGCTACTTAGGAGGCTGAGGCAGGAGAATCACTTGAACCTGGGAGGGGGAGGTTGCAGTGAGCCGAGGTC
GCGCCACTGCACTCCAGCCTGGGTGACACAGTGAGACTCCATCTCAAAAAAAAAAAAAAACCACAGAGATT
ATTCTGGCTGCAGTGTGGAGACTGAATTGGCAGAGGGCAAGACTAGTTAGGAGATTATGGTAGAGATAGT
AATGCATGGCAAACAAAATACCACTTAAGTACCTGATCCCCGTGCTCAGGGCTGTAGCAAAGGTGAGGGA
CCTTTCTTTACACTAGGCCTAAGGCAGGTTCTGTATCATTGCCTTACAAGCTAGAATCCAAGTTGCAGAA
TCTTGCTGCTGCCTTCTTCCCAGCACATATATTAAGCCTAGAAACAACGACCGATATTCACAGGGAAAAA
GCACTTCATTTTTGCCACTGCCCCCTCCATATATTTGGCCTAAATAATGTATTAAAATTTGATCACTTAA
TTTAGCAAGACTGAAACGGAGAAGGAACTAGGAAGGTGTTAAGCATAAAGTTAAGAAACTCTGCTTTCCC
TTACAGTAAATAGTATAAAGATATAAGGCATGTGAAGTTAGTCAGGGAGGGTGTGTGGAGTGAAAAAAAA
ATCCAAAACCCTAGGGAATACCATTATTTAAAGAAGGAATGGAAGAAGAGTTATCCATGAAGGGAACTAG
TAAGTGAACACTAATAAGGGAAAAGCAAGATGTATTGTGTTTGTTAAGCAGAAGTAATTTGTAGATGTGT
GAAAGTGACTGTCATACAGGAATCGCAAATACAGTGAGACACAAATGGTCCCTTTAAACAGTTACCTCTA
CCCCAAGAGAAGATGCTAAGACTATGTGCATAACCGTTCCCCTGAGAGAATTTACTATCTATTGAATTGT
GTTCTTATCCAATTTTAATATTTCTTCACTTCTGTTCTTTACTTTTCCATGTTAACCATGCTTTATCTTA
TATATCCATGCCTGTCTTATTGTTGATCTTTGACTCTTTTCAGCCATCCCATGTAACCTCTCTTGCTTGA
TGCATGAGTAGGCTGCCTTCCTATCTTGCACCTCTTGGCTCTAGGCCTGTCTTGTGGCACCTCTTGTCGA
TTAATTCAATAACAGGGCCCTCCTGTTTGAACTGGCCCTGAAACCTCTCTAATCACCCATGTACTGGCCT
AGGAGGCAACTTCTACAAGGGTCTGAAATGTGTATGTGTATGTATGTATAACACATGTGCATATTTGTAT
ATATATGAAGTATGTATGTGTATGTATGTATAACACATGTGCATATTTGTATATATATATGAAATACGTAAG
AATATTTGACATTGTTCCAGTTAGGAATGTATAAGTAATTAAGGCAGATTAACAAAACATACAACTTTTG
 
TATATAATTGGAGTGCTGTCAAGAGGATCTGTAGGAAAATAGGGCATTCTGATCTGCTTGTGAAGAACAG
TTACTTCAGAAAAGGAAAGTGAGATACATACAGACATAGATATAGAACTATAATATACTATATATGTGTG
CATATATTATAAAGGTCAGTAATACATATGTTTAATACTCTGGTAAGTGCTATAGTAAGGAGAAGGGACC
GTGGCTGCTCAGAGTCAAAGGATTGAGAGAGTCCATTTTGAACTGAATCTGAAGGATATAAGGGAATTT
GACATGGGGTGGAGGGACTTTCCAGATTAAGAATAATTATATAAAGGCTGAGCACAGTGGCTCATGCCTG
TAATCCAAGCACTTTGGGAGGCCGAGGTGGGTAGATCACTTGAGGTCAGGAGTTCAAGGCCAGCCTGGCC
AACACGGTGAAACCCTGTCTCTACTAAAAATACAAAAATTAGCCAGGCATGATGGTGGGTGCCTGTAATC
CCAGCTACTCAGGAGGCTGAGGCACAAGAATTGCTTGAAGCCAGGAGACCGGTTGCAGTGAGCCGAGATC
GCGCCACTGCACTCGAGCCTGGGCGACAGAGTGAGACTGTCTCAAAAAAAATTACACAAAGAAGATAAAT
GTCAAAAAAAAAAACAAAAAAAAATTAGGATTCCATCATGGGTTTTGGGTCCTAACCCCAAACTTCCTC
TTTCAACTCATGATCCCTTTATAACCCTTTTCTCTGTTCTTCATTAGCCAATTCTTCTGGTCACTCTTTC
TCACCCTCTTTAAATGGGTTTGCTTTATTTTATTCTTCACCCTAGTACTTAAATTGCTGTCAATTATATT
TTATTATACTGTCTAACTGTCCTATTTTTTCTTTTTGCAATTTTTTCATACAGATATCAGGGTCTTATT
TAAATGTCACCAATATTTATTCCTTCTACACTTTTAGTCTCAGTAGTCAGATGCTATGATATCACATGAA
ACATTTTTGATTTTTTTTTTCCCTCCAGGATCGAAGAGCAGTCATAAAACCACAAAGTCAACGCAAACAC
AAGACTCTTCATTTCAGGGACTGATACTGAAAAGATCCAACAGGAATGTACCTTGGGATTTGAAATTAGA
AAAGCCTTACATATATGAAGGCAGATTAGAGAAAAAGCAGGATAAAAAGGGAAGTTTTCAGATAGTTTCA
GCCACCCACAAAAAAATCCCCACTATAGAAAGAAGCCATAAAAATACTGAATTGAGCCAAAACTTCAGCC
CAAAGTCAGTGCTTATTAGGCAACAGATACTTCCCAGAGAAAAAACACCACCAAAATGTGAAATACAAGG
AAACAGCCTCAAACAGAATTCACAATTACTTAATCAACCAAAAATTACAGCAGATAAACGCTATAAATGT
AGTCTGTGTGAAAAAACCTTCATTAACACTTCATCCCTTCGTAAACATGAGAAAAACCATAGTGGAGAGA
AACTATTTAAGTGTAAAGAATGTTCAAAAGCCTTTAGCCAAAGTTCAGCTCTTATTCAACATCAAATAAC
GCATACTGGAGAGAAACCCTACATATGTAAAGAATGTGGAAAGCCTTTACTCTCAGTACATCCCTTTAT
AAACATCTAAGAACCCATACTGTGGAGAAATCCTACAGATGTAAAGAATGTGGTAAATCCTTCAGCCGAA
GGTCAGGCCTTTTTATACATCAAAAAATTCATGCTGAAGAAAACCCTTGTAAGTATAATCCGGGTAGGAA
GGCATCTAGTTGCAGCACATCCCTTTCTGGATGTCAAAGAATTCATTCTAGAAAGAAGTCCTACTTATGT
AATGAATGTGGCAACACCTTTAAGTCTAGCTCATCCCTTCGTTATCATCAGAGAATTCACACTGGAGAGA
AGCCTTTTAAATGTAGTGAATGTGGGAGAGCCTTCAGCCAGAGTGCCTCTCTTATTCAACATGAAAGAAT
TCACACCGGAGAAAAGCCCTATAGATGCAATGAATGTGGAAAGGCTTTACTTCTATTTCACGACTTAAT
AGACACCGAATCATTCATACTGGAGAGAAGTTTTATAATTGTAATGAATGTGGTAAAGCCTTAAGCTCCC
ACTCAACACTTATTATTCACGAGCGAATTCATACTGGAGAAAAACCATGTAAATGTAAAGTATGTGGAAA
AGCCTTCAGACAGAGTTCAGCTCTCATTCAACATCAGAGAATGCATACTGGAGAAAGACCCTATAAATGT
AACGAGTGTGGGAAAACATTCAGGTGTAACTCATCACTTAGTAATCACCAGAGAATTCATACTGGAGAGA
AACCATATCGATGTGAGGAATGTGGGATATCTTTTGGCCAAAGTTCAGCTCTTATTCAGCATCGAAGGAT
TCATACAGGAGAAAAACCCTTTAAATGTAATACATGTGGAAAAACTTTTAGACAAAGCTCATCACGTATT
GCACATCAGAGAATTCATACTGGAGAGAAACCCTATGAATGTAATACATGTGGGAAACTTTTCAACCATA
GGTCATCCCTTACTAATCATTATAAAATTCATATCGAAGAGGACCCCTAGAAAGTAGATTTGTATGTGTG
AAAGCCTTAAACCAAAGCTCATCGAAGAATACATCCTTGAGAGAGATGTAATAAATGTAATGGATGTGAA
AAAAACTGTAATAATTTAGCCCTCATTAGGTATTTAATTCCATGGATAAACCTCAGCTATATAATAGATA
TGAGGAAAGTGTTTGTGCCTGTCAGACACTTAAAAAAATAACCTGAGATGAAGAATTTACAATTGAAGAC
ATTGACTTTAGCCATTTGTGAAATGGGTTTGCTTTTTCCCTTTTCCTACAGACGTATATGCTAGATGTCA
CGTGATCATCAGAAACAGATATCCGAGTGGGTGGGGAGGTGTGCTTTAGATTTCTCATTAGAAGACCACC
AAACTGGTAATATTTTTATAGCATTTTAATAGCTTGATCAAATTGTACCTTTTTAGAGAAAAGGACCAAA
ATAAAAGAAAAATGAATTATGAACTACCTCTCAGTCTCTGGGGTTTGTCCTTTTCCTACCCTGATGTCAA
ACTTATGCATGGATTTCATTAAAAAAGAAAAAGAAAAA
- **SEQ ID NO: 18**
   siRNA sc-91974A
   chr5 - 178140605 178140623. Targeting Exon5-intron4 Sense strand:
   GGAUCGAAGAGCAGUCAUAtt
- **SEQ ID NO: 19**
   siRNA sc-91974A
   chr5 - 178140605 178140623. Targeting Exon5-intron4
   Antisense strand:
      UAUGACUGCUCUUCGAUCCtt
- **SEQ ID NO: 20**
   siRNA sc-91974B
   chr5 - 178139978 178139996. Targeting Exon5
   Sense strand:
      GAGAAAUCCUACAGAUGUAtt
- **SEQ ID NO: 21**
   siRNA sc-91974B
   chr5 - 178139978 178139996. Targeting Exon5
   Antisense strand:
      UACAUCUGUAGGAUUUCUCtt
- **SEQ ID NO: 22**
   siRNA sc-91974C:
   chr5 - 178138746 178138764. Targeting 3'UTR
   Sens strand:
      GUCACGUGAUCAUCAGAAAtt
- **SEQ ID NO: 23**
   siRNA sc-91974C:
   chr5 - 178138746 178138764. Targeting 3'UTR
   Antisens strand:
      UUUCUGAUGAUCACGUGACtt
- **SEQ ID NO: 24**
   -QIAGEN SI04335226
   chr5 - 178138991 178139011 3'UTR Sense:
      UACAUCCUUGAGAGAGAUGUAtt
- **SEQ ID NO: 25**
   Antisense:
   UACAUCUCUCUCAAGGAUGUAtt
- **SEQ ID NO: 26**
   ORIGENE. SR304750,
   chr5 + 178139838 178139864, targeting exon 5 Sense:
      AAUUCUUUGACAUCCAGAAAGGGAUGU
- **SEQ ID NO: 27**
   - Antisense:
      ACAUCCCUUUCUGGAUGUCAAAGAAUU
- **SEQ ID NO: 28**
   chr5 + 178140534 178140560 exon5
   + Sense:
      AUUCCUGUUGGAUCUUUUCAGUAUCAG
- **SEQ ID NO: 29**
   - Antisense
      CUGAUACUGAAAAGAUCCAACAGGAAU
- **SEQ ID NO: 30**
   chr5 + 178138917 178138943. 3'UTR
   + Sense:
      GAGGUUUAUCCAUGGAAUUAAAUACCU
- **SEQ ID NO: 31**
   - Antisense
      AGGUAUUUAAUUCCAUGGAUAAACCUC
- **SEQ ID NO: 32**
   ZNF354A promoter, ENSEMBL GrCh38: chromosome:GRCh38:5:178728826:178731484:1 
GGGTGGGTGGTCCTCCCTACAGAATCCACCACTCATGAGGGCGGACCAGCTCCTCCAAGA
GAAAAGGACTTGGGCTCCATCCTGGCTGGAGATGGTCCCGGGTGAAGAACTCCAGTTAGT
GGGGAAAATCCATCCACTGGGATCTGGGAGGGCAATGAGCTCACCTGGGGCCTCGCTTCC
CGCTGCCCAGCAGCCATGTCCTCTTTCTGGCTCTCCCGGGAGGACGGCGCAGTCTCCAGA
AGGCAGATCTGAGAAGAAAAAGGGAGCCAGGAAGAACCCCTTCTGCTTTCCAGCCCTGGA
ATCCGCAGCTCTAAAACCCCGACTCCCGCTGAGAAGGACCCCGCGCGCCCTCACAGGGAT
TCGGGAGGAGCCAGGAGCCCGCGGACCAGAGCAGCGCTGGGCCGCCCGGCCAGGCCCAGG
CCCCACAGGCTTGGGGCTTACGGAGGGGACGCTGGAGCCGCAGTGGCAGCAGCGACCCGC
TGAGCCCACCAGGGCCAGGGCTCGCCCCACGCCGCTGAGTTCCTCGGCGCCAGCTGGCAC
TGCCAGCACCCACTGCACCGCTCAGGAAACTGAGGCAGCGCCGGGGCACCTGCCCCGTTC
ACGCCGCTGCGAGGCGGGCCGGGACTCCCACCACGACGGCCCCGGGACGACTTCCCTCCG
CCTCAAGCCTTTCCCAGCCGGTTCCTCTCTCTCCCGCCAACGAATCATCTCCCGACTCCA
CGGAAAACGGCTTTTTTTTTTTTTGAGGCGGAGTCTCGCTCTGTTGGCCAGACTGGAGTGC
AGTAGCGCAGTGGCAGGATCTCGGCTCACTGCAAGCTCTGCCTCCCGGGTTCAAGCCATT
CTCCTGCCTCAGCCTCCCGAGTAGCTGGGACTACAGGCGCCCGGCTCATTTTTGTATTTT
TAGTAGAGACGAGGTTTCACCGTGTTGCCCAGGCTGGTCTCGAACTCCTGATCTCAGGTG
ATCTGCCCGCCTTGGCATCCCAAAAATCTAGGATTACAGGCGTGAGCCACCACGCCCGAC
CCCATGTATCCCATTTCTAACACGATGTTTCTTTTTTGAGACGGAGTCTCGCTCTGTCGC
TCAGGCTGGAGTGCAGGGCTTGATCTCGGCTCACTGCAAGCTCCGCCTCCTGGGTTCACG
CCATTCTCCTGCCTCGGCCTCACAAGTAGCTGGGACTACAGGCGCCCGCCACTACGCCCG
GCTAATTTTTTTTGTATTTTTAGTAGAGACGGGGTTTCACCGTGTTAGTCAGGATGGTCT
CGATCTCCTGACCTCGTGATCCACCCGCTTCGGCCTCCCAAAGTGCTGGGATTACAGGCG
TGAGCCACCGCGCCCGGCCAAACACCAGCTCTGAAGAGGAAAGCGCTGGGTGTCCCGAGG
CGGGAGGGGTGGCCGTCCTGGGGACGGACTCGGAAACCGGGGCCCGGCTGCGTCCGCCCT
GCCCTCGGGGTCCCGGTGCCCCGAGTCCGCGAGGCCCACATGGAGTGGGCAGGACGGCC
CGGGAGGAAACGCCAGGCCCGGCGTCCACGGCGAGCCGGACGCGGGTGCACCAGCAACGG
CGAGGGAGCGCCCCGCGGGGGGCGAGAGTCCAGATCCGGCGCGGGGGCCGGTGACAGA
CGCGGGGGTGCCCGCCAGCGCCCACAAAGGCTTTCGGAGCCTCGGCAGCCGCAGGCAGTC
CCCGTGCCGGGCGCCGCCTCCTACCGGCTTGGCCTGCCGCCGACGCTCACCTCCCTAAGC
TCGAGCGTCCCGGGCCGCGCCTCCCCAGCCGCGAGGCTCCGGAACCGCCGGCCGGGATGC
AGCCTCGCGACCCGGCTCCGCCCCGACGGCGTCTGGGCGACCTCAGGCCCGGCGCCGCTC
ACCCCGCGGCGCGCCGGGGACCAGGAGGGGCGGGGCAGCCGCTGAGGCCGCTGGGAAATG
GAGTCCACGAGAGGACACCTGCATTCGGGGAAGTGTAGTTCCATGCGTTTGAGGCGGGGT
CCCGGCTGAGCTGTTACGCGCCCACATGCCACAAGAGGGCCCCTGTGAGCTGGAGGGCCC
CAGGTTCCTGGTTATAGGGAAGATAAGGGGAGGTTCGCTGAGGGCAGTAACTTGCAACTC
TGTGTCTGTGGGAATTTCGGGAGGGAGGGCGTGGGCGGCAGGAAAACGGCCCAGAACAGA
CCTAGTGCCCCTCAGTTATTCACCAGAGGGGCACTGAGCACCTGCTGTGTGCCAAGCACG
GTTGCAAGTGGTTTCACATGTATCAATCAATGAAAGAAACGTCACTGCCCTGGAGAACAT
TTTTTTGGGGGGTAGGGAGCAGACAATAAGCCATACACATAATTATATAACATTTTCGGG
GATAAATGTGAGGAGAAACAGGGTATGTGGAGGGGTTTCGTCGCTGAGGTGAGAATGGAC
GAGGACTCCCAGGAAGTGCCTGAAGCAAAGGGGAGGGAGGTGCCAGCTACTGTGAAGCCA
ACGGATGTATGTATGTATATGTGTGTGTGTGTGTGTATATATATATATATTTTTTTTA
AACTAAATCTTCTCAGTTACAAAAAGAACACCGGCTCCATTTAGAAAGTTTGGCGCTTCC
CTACTTAAGAATATGCCTTGAACATTTGAATTTGTCAATAAATATTCTCCCTCAGTGGCA
TTTTTGATAGCTTCATTGC
   ZNF354A - entire promoter region - chr5: 178,728,826-178,731,484 Underline - Enhancer 1 - chr5:178,728,992-178,729,587
   Italic - Enhancer 2 - chr5:178,729,863-178,730,649
   Underline/italic - Promoter - chr5:178,730,650-178,731,225
   Underline - Enhancer 3 - chr5:178,731,168-178,731,345
   Experimentally confirmed TF binding motifs
   chr5:178,730,085-178,730,108
   chr5:178,730,482-178,730,499
   chr5:178,730,518-178,730,534
   chr5:178,730,721-178,730,738
   chr5:178,730,803-178,730,819
   chr5:178,730,886-178,730,892
   The TSS is underlined and the transcript is encoded on the reverse strand. Experimentally confirmed TF binding motifs are highlighted in black.
   Source: GRCh38.p14, Ensembl release 113

### Human sh RNA

- **SEQ ID NO: 33**
   GGAUCGAAGAGCAGUCAUA
- **SEQ ID NO: 34**
   GAGAAAUCCUACAGAUGUA
- **SEQ ID NO: 35**
   GUCACGUGAUCAUCAGAAA
- **SEQ ID NO: 36**
   GCCCTCATTAGGTATTTAATT
- **SEQ ID NO: 37**
   GCCCAAAGTCAGTGCTTATTA
- **SEQ ID NO: 38**
   CAGCTCTTATTCAGCATCGAA
- **SEQ ID NO: 39**
   CAACGCAAACACAAGACTCTT
- **SEQ ID NO: 40**
   CTCTCAGTACATCCCTTTATA
- **SEQ ID NO: 41**
   ATCCCTTTCTGGATGTCAAAG
- **SEQ ID NO: 42**
   AGCCCAAAGTCAGTGCTTATT
- **SEQ ID NO: 43**
   CAAACACAAGACTCTTCATTT
- **SEQ ID NO: 44**
   TCTCAGTACATCCCTTTATAA
- **SEQ ID NO: 45**
   TTAGCCCTCATTAGGTATTTA

### ZNF354A binding motifs

M07622_2.00
   - **SEQ ID NO: 46**
Fw.
RTTTARHCCATTTAC
Rev.
   - **SEQ ID NO: 47**
GTAAATGGDYTAAAY
M08331_2.00
Fw.
   - **SEQ ID NO: 48**
RTTTARDCCATTTAY
Rev.
   - **SEQ ID NO: 49**
RTAAATGGHYTAAAY
M08914_2.00
Fw.
   - **SEQ ID NO: 50**
DTTYHDNYCHTTTMYWTTTADTGW
Rev.
   - **SEQ ID NO: 51**
WCAHT AAA WRKAAADGRNHDRAAH

**Table 1**

| Code for Nucleotide representation | |
|---|---|
| **Letter** | **Nucleotides it represents** |
| A | Adenine (A) |
| C | Cytosine (C) |
| G | Guanine (G) |
| T | Thymine (T) |
| W | Weak (A or T) |
| S | Strong (C or G) |
| M | Amino (A or C) |
| K | Keto (G or T) |
| R | Purine (A or G) |
| Y | Pyrimidine (C or T) |
| B | Not A (C, G, or T) |
| D | Not C (A, G, or T) |
| H | Not G (A, C, or T) |
| V | Not T (A, C, or G) |
| N | Any nucleotide (A, C, G, or T) |

### sgRNA to crispr ZNF354A human

- **SEQ ID NO: 52**
   AGCCCAAAGTCAGTGCTTATT
- **SEQ ID NO: 53**
   CAAACACAAGACTCTTCATTT
- **SEQ ID NO: 54**
   TCTCAGTACATCCCTTTATAA
- **SEQ ID NO: 55**
   TTAGCCCTCATTAGGTATTTA

### EXAMPLES

### MATERIALS AND METHODS

### Cell lines

U2OS (ATCC: HTB_96), MDA-MB-231 (ATCC: HTB_26), K-562 (ATCC: CCL_243), SUDHL4 (ATCC: CRL_2957), SUDHL5 (ATCC: CRL_2958), SUDHL6 (ATCC: CRL_2959), MCF7 (ATCC: HTB_22), SW480 (ATCC: CCL_228), LS1034 (ATCC: CRL 2158), HCT116 (ATCC: CRL_3502), HAP-1 (Horizon Discovery: HZGGHC9001), HAP-1 KO_KAP1 (Horizon Discovery: HZGHC000293c003), HEPG2 (ATCC: HB_8065), Calu-3 (ATCC: HTB_55), 3T3 (ATCC: CRL_1658), VERO-E6 (ATCC: CRL_1586) cells were grown in Dulbecco's Modified Eagle Medium supplemented with 10% FBS, penicillin, and streptomycin. HELA (ATCC: CCL_2) cells were grown in MEM Media supplemented with 10% fetal calf serum, 1% penicillin-streptomycin, 1% NEAA and 1% glutamine (all media from ThermoFisher Scientific).

### Antibodies and Reagents

The antibodies and reagents were acquired from: ATF2 (Abcam: ab_131484; RRID: AB_11156678, rabbit: 1:2000x), phosphor-ATF2 (T69) (Abcam : 131106; RRID: AB_11157608;rabbit:1:1000x), GAPDH (Thermofisher : 398600; RRID: AB_2533438; mouse: 1:4000x), ZDHHC20 (Sigma: SAB4501054; RRID: AB_10744838; rabbit: 1:2000x), HA-HRP (Roche: 112013819001; RRID: AB_390917; rat: 1:5000x), KAP1 (Abcam: ab_109289; RRID: AB_10863057, rabbit: 1:1000x), phosphor-KAP1 S473 was generated in D. TRONO laboratory using the following peptide krsrSgegevsgl (rabbit: 1:500x), Nucleocapside N SARS-CoV-2 (Genetex: GTX135357; RRID: AB_2868464; rabbit 1:4000x), FLAG (Sigma: F3165; RRID: AB_259529; mouse: 1:2000x), phosphor-Threonine-Serine (BD: 612548; RRID: AB_399843; rabbit: 1:1000x), GPX4 (Cell Signalling: 52455; RRID: AB_2924984, rabbit: 1:1000x), V5-HRP (Sigma: V2260; RRID: AB_261857, mouse : 1:2000x), Actin (Millipore: MAB1501; RRID: AB_2223041, mouse: 1:4000x), MAT1A (Abcam: 129176; RRID: AB_11145300, rabit: 1:2000x), ZNF354A (Santa Cruz: 81140; RRID: AB_1131557, mouse: 1:1000x), 4G10 phospho-Tyrosine (Millipore: 05-321; RRID: AB_309678, mouse: 1:1000x) and HRP-conjugated secondary antibodies (GE-Healthcare). RSL-3 (Sigma: S8155), Hydrogen Peroxide 30% (Fisher: H/1750/15), Rotenone (Cayman Chemical: CAY-13995), Ferrostatin-1 (Sigma: SML0583), Bodipy 581/591 C11 (Sigma: D3861), Liperfluo (Fisher: L248), Propidium Iodide (Thermofisher: P3566), Cell Viability assay (Promega: G9711), JQ1 (Sigma: SML-1524), and Mithramycin (LKT: M3476).

### siRNA

The following human siRNA (5' >3') with the corresponding target sequences were purchased from Qiagen: ZNF354A (TACATCCTTGAGAGAGATGTA, SEQ ID NO: 56),
ZNF 317 (CATGACGGAAATCACACTAAA, SEQ ID NO: 57),
ZNF 308 (CAGTTCATTGGCAACCTCATA, SEQ ID NO: 58),
ZNF 677 (GAGATTGGTTACAGTACAAGA, SEQ ID NO: 59),
ZNF793 (ACCCTGGCATGTAAACGTTTA, SEQ ID NO: 60),
SETDB1 (TCGGGTGGTCGCCAAATACAA, SEQ ID NO: 61),
KAP1 (AGCGTCCTGGCACTAACTCAA, SEQ ID NO: 62),
ATF2 (CCGAGGTAGTCCACATACAGAA, SEQ ID NO: 63),
GPX4 (CGGCTGCGTGGTGAAGCGCTA, SEQ ID NO: 64).

The following human siRNA (5' >3') were purchased from Santa Cruz: ZNF354A (sc-91974), SP1 (sc-44221), FOXA1 (sc-37930). Mouse siRNA were purchased from Qiagen for mouse ZFP354A (TACCACTTTAATAACATTCAA, SEQ ID NO: 61).

As a control siRNA, a sequence targeting the viral glycoprotein VSV-G (5'-ATTGAACAAACGAAACAAGGA-3', SEQ ID NO: 65) was used. Transfections of 50 nM of siRNA were carried out using *TRANS*IT-X2 (MIRUS), and the cells were analyzed at least 72 h after transfection.

### Western Blotting

Cells were washed three times in 1x PBS at 4°C and lysed in Buffer (1x PBS, 0.5% NP40 and protease inhibitor cocktail; Roche) for 30 min on ice. Lysates were then spin down at 10 000 g, and the protein content of supernatant were determined, the samples were boiled in Laemmli buffer for 5 min before separation via SDS-PAGE and western blotting against the different anti-bodies used in this study. Western blots were developed using the ECL protocol and imaged on a Fusion Solo from Vilber Lourmat. Densitometric analysis was performed using the software Bio-1D from the manufacturer.

### Plasmids

Plasmids expressing HA-tagged ZNF354A-HA, ZNF317-HA, ZNF677-HA, ZNF793-HA were obtained from the D. Trono lab previous generated by (Imbeault et al., 2017). Plasmids expressing FLAG-ATF2 were from Origene (RC2118983), and plasmids expressing SP1-FLAG (25543) and FOXA1-V5 (153109) from Addgene. Mutations to change ZNF354A-HA to ZNF354A-FLAG, FLAG-ATF2 to HA-ATF2, HA-ATF2 6A (T52A-T69A-T71A-T73AS490A-S498A), HA-ATF2 6D (T52D-T69D-T71D-T73D-S490D-S498D), ZNF354A-HA RR (D18R-E27R), ZNF354A RA (E45R-N46A), ZNF354A RR-RA (D18R-E27R- E45R-N46A) were done following Quickchange mutagenesis kit (Agilent) instructions.

### Aerolysin purification and cell intoxication

Proaerolysin toxin was produced and purified by our laboratory in Aeromonas salmonicida as previously described (ref). Cells were treated one hour in complete medium with 10 ng/ml of proaerolysin at 37°C. Cells were washed twice in complete medium and further incubated at 37 °C for indicated times.

### GREAT - analysis

ChIP-seq data for ZNF354A-HA binding sites were obtained from KRABopedia (de Tribolet-Hardy et al., 2023) and submitted to the Genomic Regions Enrichment of Annotations Tool (GREAT - http://great.stanford.edu/public/html/) (McLean et al., 2010). The parameters for gene regulatory domains for each gene were defined as domain that extends in both directions of the TSS until the nearest TSS up to a maximum extension of 300 kb.

### Human MucilAir assays

MucilAir-human upper respiratory tissues (Epithelix, Geneve, Switzerland) were maintained according to the manufacturer's protocol. For infection, tissues were washed apically with 200 µl of DPBS, calcium, magnesium (#14040091-Gibco) for 20 min at 37 °C and the basal medium replaced with fresh mucilair medium. Tissues were infected at the MOI of 0.1 PFU (assuming the manufacturer's estimations of 500000 cells per tissue). Tissues were inoculated with 200 µl of SARS-CoV-2 (passage 3) for 3 h (apically) at 33 °C. The apical inoculum was removed and infected tissues were maintained for until harvest. For harvest tissues were lysed using 300 µl lysis buffer for 30 min and processed for western blot.

### Viral Stock production and titration with plaque-based assays

All viral stocks were produced and isolated from supernatants of Vero E6 cells, cultured in T75 culture flasks to a confluency of 80-90%, and infected with an original passage 2 (P2) SARS-CoV-2 virus, for 48 or 72 h, at MOI≈0.05, in 10 ml DMEM supplemented with 2.5% FCS. Original stocks were obtained from the following strain: hCoV-19/Switzerland/GE9586/2020|EPI_ISL_414022|2020-02-27. Passage 3 supernatants were harvested, clear of cell debris by centrifugation (500 g 10 min) and filtration (0.45 µm), aliquoted and stored at -80 C. Viral titers were quantified by determining the number of individual plaque forming units after 48 h of infection in confluent Vero E6 cells as described in S. Mesquita et al., 2023).

### SARS CoV-2 infections

All infections for experimental analysis were done using passage 3 SARS-CoV-2 stocks. Vero E6 cells or CALU-3 cells seeded to a confluency of 90 to 100%, were, washed twice in warm serum-free medium and inoculated with the indicated MOI of SARS-CoV-2, diluted in serum free medium. 1 hour after inoculation cells were washed with complete medium and infection allowed to proceed for the indicated time points in DMEM supplemented with 2.5% FCS, penicillin and streptomycin.

### Drug and toxin treatments

All drug treatments were done for the indicated times in complete culture and/or infection medium. Equivalent volume of Dimethyl sulfoxide (DMSO), used as a solvent, was added for control samples. Specifically, culture media was replaced, cells were washed once in warm culture media and drugs resuspended as indicated were used as: RSL3 (in DMSO - 3 µM, increasing concentrations 0.6 to 10 µM), Ferrostatin-1 (DMSO - 1 - 15 µM), H2O2 (0.5 - 100 µM), Rotenone (1 - 10 µM).

### UCSC genome browser analysis

UCSC genome browser at ZDHHC20 locus was obtained from (Human version hg19). Specific UCSC genome browser tracks were selected for display in Fig. 1 and 2. Transcript annotation corresponds to GENCODE Genes track (version V4 0lift37); Histone Modifications by ChIP-seq from ENCODE/Stanford/Yale/USC/Harvard display ChIP-seq signals for H3K4me1, H3K4me3, H3K27Ac marks for HepG2 and H3K9me3 for U2OS. Transcription Factor CHIP-seq Peaks track shows transcription factor SETDB1, and KAP1 binding sites for U2OS and K562 cells based on ChIP-seq experiments from ENCODE. KZFP CHIP-seq data show binding sites for different KZFP proteins obtained from KRABopedia (https://tronoapps.epfl.ch/web/krabopedia/) (https://doi.org/10.1101/2023.02.27.530095)

### Q-RT-PCR

For the real-time PCR, RNA was extracted from a six-well dish using the RNeasy kit (Qiagen). In all, 0.5-1 mg of the total RNA extracted was used for the reverse transcription using random hexamers and superscript II (Invitrogen). A 1:40 dilution of the cDNA was used to perform the real-time PCR using using Applied Biosystems SYBR Green Master Mix on 7900 HT Fast QPCR System (Applied Biosystems) with SDS 2.4 Software. All data (always in triplicate) were normalized to Ct values from three housekeeping (HK) genes ALAS-1, Guss and TBP. Results were expressed as 2^(-ΔΔCt)*100%.

### RNAseq

RNA quality was controlled on the TapeStation 4200 (Agilent), which confirmed their integrity. Libraries for mRNA-seq were prepared with the Stranded mRNA Ligation method (Illumina), according to manufacturer's instructions, starting from 800ng RNA. Libraries, all bearing unique dual indexes, were subsequently loaded at 100 pM on a Novaseq 6000 flow cell (Illumina) and sequenced according to manufacturer instructions, yielding pairs of 60 nucleotides reads at a depth of about 70 mio reads pairs per sample. Reads were trimmed of their adapters with bclconvert v00.000.000.3.9.3 (Illumina) and quality-controlled with fastQC v0.11.9. FastQ Screen v0.14.0 tool was used for screening FASTQ files reads against multiple reference genomes.

### RNAseq processing and analysis

Sequencing reads were mapped to the human hg19 genome using hisat2 [D. Kim, B. Langmead, and S. L. Salzberg, "HISAT: a fast spliced aligner with low memory requirements," Nature Methods, vol. 12, no. 4, pp. 357-360, Mar. 2015.] with parameters: hisat2 -k 5 --seed 42 --rna-strandness RF.

Counts on genes were generated using featureCounts [Y. Liao, G. K. Smyth, and W. Shi, "featureCounts: an efficient general purpose program for assigning sequence reads to genomic features," Bioinformatics, vol. 30, no. 7, pp. 923-930, Apr. 2014.]. Only uniquely mapped reads were used for counting on genes.

Normalization for sequencing depth was done using the TMM method as implemented in the limma package of Bioconductor [Gentleman et al., 2004].

Differential gene expression analysis was performed using voom (Law et al., 2014) as it has been implemented in the limma package of Bioconductor. A gene was considered to be differentially expressed when the fold change between groups was bigger than 2 and the p-value was smaller than 0.05. A moderated t-test (as implemented in the limma package of R) was used to test significance. P-values were corrected for multiple testing using the Benjamini-Hochberg's method (Benjamini and Hochberg, 1995).

Gene set enrichment analysis (GSEA) was performed using the ClusterProfiler package in R. The analysis utilized a ranked list of genes and gene set collections downloaded from MsigDB. The GSEA was conducted with the following parameters: the number of permutations was set to 10,000, minimum gene set size was 10, maximum gene set size was 800.

### Flow cytometry analysis of membrane permeability and lipid peroxidation

U2OS cells (6 well plate at approximate 1 × 10^6 cells), siRNA depleted as indicated were treated with 5 µM RSL-3 for 24 h. Cells were washed twice in EBSS (10 µM Hepes) and Lipid peroxides stained by Liperfluo (Dojindo) and C11-BODIPY581/591 (Invitrogen). Stock solutions of Liperfluo or BODIPY, prepared in DMSO, according to manufacturer's instructions were resuspended in EBSS-Hepes to 5 µM (Liperfluo) and 10 µM for Bodipy and used for cell incubation 1 ml per well for 30 min at 37°C. After treatment cells were washed, trypsinized and resuspended in 500 ul EBSS-Hepes. For propidium iodide analysis Liperfluor labelled cells were stained with propidium iodide prior analysis at final concentration 1 µg/ml. Analytical flow cytometry was performed using an LSRII or LSR Fortessa (BD; Becton Dickinson) instrument. For Liperfluor, and BODIPY-oxidated cells were analysed at Excitation: 488 nm, Filter: 530/30; For PI and BODIPY-non-oxidated Excitation:561 nm Filter 610/20. 10,000 events were acquired per each replicate per sample. Results were analyzed using FlowJo.

### DSS-induced mice colitis

Six wild-type C57BL/6j mice (8-week-old males) were used. Three (n = 3) mice were used as controls and three mice were given 3% dextran sulfate sodium in the drinking water for 7 days, then switched to regular drinking water for 3 days. Three Control 8-week male were given drinking water that did not contain DSS. During the 10-day experiment, mice were weighted and disease activity index (DAI) was performed daily based on stool consistency, presence of occult blood and body weight loss. On day 10, mice were euthanized with injection of pentobarbital and bled via cardiac puncture, and colon and intestines were collected. For animal experimentation, all procedures were performed according to protocols approved by the Veterinary Authorities of the Canton Vaud and according to the Swiss Law (license VD 3497, EPFL).

### Results

### Differential regulation of the acyl-transferase ZDHCC20.

The Inventors found that the acyl-transferase ZDHHC20, which mediates post-translational lipid modification of proteins (S-acylation), is expressed as several isoforms (S. Mesquita *et al*, 2023). A "short" ~35 kDa isoform is produced under physiological homeostatic conditions, but following aggressions such SARS-CoV-2 infection (Fig. 1A) or exposure to pore-forming bacterial toxins (Fig. 1C), as well as in a dextran sodium sulfate-DSS-induced mouse model of ulcerative colitis (Fig. 1B), N-terminally extended Long-ZDHHC20 isoforms (20L, between 40 and 60 kD in apparent molecular weight) are produced by activation of one or several upstream transcription start sites (**Fig 2A**) (S. Mesquita *et al*, 2023). More recently we also observed that expression of 20L isoforms increases with age, as observed in primary epithelial lung tissues (**Fig 1D**) and is detected in some cancer cell lines, including HCT116 human colorectal carcinoma, the MCF7 breast cancer (**Fig 2E**) and HepG2 hepatocellular carcinoma (S. Mesquita *et al*, 2023) cells.

### Differential expression of Zdhhc20 isoforms is epigenetically regulated.

Chromatin analyses of HEPG2 cells revealed the presence of the H3Ac27 and H3K4m3 activation histone marks at the canonical *zdhhc20* promoter (**Fig 2A**), as well as H4K4me1 over an upstream region coinciding with binding sites for the FOXA1 and SP1 transcriptional activator, consistent with the presence of an enhancer (**Fig 2A**). In U2OS cells, we also detected the repressive mark H3K9me3 further upstream, indicative of heterochromatin (**Fig 2A**). Within this region, ENCODE ChIP-seq peak data indicates that this locus is bound by the SET Domain Bifurcated Histone Lysine Methyltransferase 1 (SETDB1), which mediates trimethylation of histone 3 on lysine 9, and by KAP1 (KRAB-associated protein 1), which recruits SETDB1 to KZFPs, Krüppel-associated box (KRAB) domain-containing zinc-finger proteins (**Fig 2A**). Accordingly, we could demonstrate that silencing of SETDB1 or KAP1 by RNA interference induced the expression of 20L isoforms (**Fig 2B**), a result confirmed in a KAP1 KO HAP cells (**Fig 2C**). It can be concluded that the expression of 20L ZDHHC20 isoforms is repressed by the heterochromatin-inducing KAP1/SETDB1 complex.

### ZNF354A acts as repressor on an upstream zdhhc20 promoter

To identify the KZFP(s) responsible for recruiting the KAP1/SETDB1 complex to the *zdhhc20* locus, the Inventors analyzed ChIP-seq datasets generated in 293T cells overexpressing HA-tagged forms of these proteins (KRABopedia). Several KZFPs (ZNF317, 793 and 677) were found to bind at the *zdhhc20* promoter, but ZNF354A recruitment coincided with two previously identified SETDB1 and H3K9me3 peaks upstream and downstream the TSS (**Fig 3AB**). The Inventors tested the impact of downregulating these KZFPs by RNA interference in U2OS cells, which confirmed that specifically ZNF354A is responsible for repressing the 20L ZDHHC20 isoforms (**Fig. 3B**).

### P38 and JNK-dependent phosphorylation controls activation of the LORD pathway

The Inventors have shown that different stimuli will lead to the accumulation of lipid peroxides, which subsequently trigger the phosphorylation of the three repressive complex components ATF2-KAP1 and ZNF354A, as shown for cells treated with H2O2 (Fig. 4A). Using protein specific antibodies against a given phosphorylated residue, we found that ATF2 undergoes phosphorylation of Thr-69, KAP1 on Ser-463, and ZNF354A on Ser-169 (Fig. 4A-C). The later antibody was generated by the Inventors and is not commercially available (Fig. 4C). These phosphorylation events dependent on the activity of three kinases, p38, JNK and ATM (Fig. 4DE). Inhibitors of p38 or JNK completely abrogated the phosphorylation of ATF2, KAP1 and ZNF354A, and prevented the expression of ZDHHC20L (Fig. 4DE). Inhibiting ATM had a mild effect on these four events (Fig. 4DE). And inhibition of ERK only affected ATF2 phosphorylation (Fig. 4D).

### REFERENCE LIST

Békés, M., Langley, D.R. & Crews, C.M. PROTAC targeted protein degraders: the past is prologue. Nat Rev Drug Discov 21, 181-200 (2022)
Benjamini, Y., Hochberg, Y., 1995. Controlling the False Discovery Rate: A Practical and Powerful Approach to Multiple Testing. Journal of the Royal Statistical Society. Series B (Methodological) 57, 289-300.
de Tribolet-Hardy, J., Thorball, C.W., Forey, R., Planet, E., Duc, J., Coudray, A., Khubieh, B., Offner, S., Pulver, C., Fellay, J., Imbeault, M., Turelli, P., Trono, D., 2023. Genetic features and genomic targets of human KRAB-zinc finger proteins. Genome Res 33, 1409-1423. https://doi.org/10.1101/gr.277722.123
Didovyk A, Borek B, Tsimring L, Hasty J. Transcriptional regulation with CRISPR-Cas9: principles, advances, and applications. Curr Opin Biotechnol. 2016 Aug;40:177-184.
Imbeault, M., Helleboid, P.-Y., Trono, D., 2017. KRAB zinc-finger proteins contribute to the evolution of gene regulatory networks. Nature 543, 550-554. https://doi.org/10.1038/nature21683
McLean, C.Y., Bristor, D., Hiller, M., Clarke, S.L., Schaar, B.T., Lowe, C.B., Wenger, A.M., Bejerano, G., 2010. GREAT improves functional interpretation of cis-regulatory regions. Nat Biotechnol 28, 495-501. https://doi.org/10.1038/nbt.1630
REMINGTON'S PHARMACEUTICAL SCIENCES (Mack Pub. Co., N.J. 1991)
S. Mesquita, F., Abrami, L., Bracq, L., Panyain, N., Mercier, V., Kunz, B., Chuat, A., Carlevaro-Fita, J., Trono, D., van der Goot, F.G., 2023. SARS-CoV-2 hijacks a cell damage response, which induces transcription of a more efficient Spike S-acyltransferase. Nat Commun 14, 7302. https://doi.org/10.1038/s41467-023-43027-2
Sies, H., Jones, D.P., 2020. Reactive oxygen species (ROS) as pleiotropic physiological signalling agents. Nat Rev Mol Cell Biol 21, 363-383. https://doi.org/10.1038/s41580-020-0230-3
Stockwell, B.R., Friedmann Angeli, J.P., Bayir, H., Bush, A.I., Conrad, M., Dixon, S.J., Fulda, S., Gascón, S., Hatzios, S.K., Kagan, V.E., Noel, K., Jiang, X., Linkermann, A., Murphy, M.E., Overholtzer, M., Oyagi, A., Pagnussat, G.C., Park, J., Ran, Q., Rosenfeld, C.S., Salnikow, K., Tang, D., Torti, F.M., Torti, S.V., Toyokuni, S., Woerpel, K.A., Zhang, D.D., 2017. Ferroptosis: A Regulated Cell Death Nexus Linking Metabolism, Redox Biology, and Disease. Cell 171, 273-285. https://doi.org/10.1016/j.cell.2017.09.021
Watson, G., Ronai, Z., Lau, E., 2017. ATF2, a paradigm of the multifaceted regulation of transcription factors in biology and disease. Pharmacol Res 119, 347-357. https://doi.org/10.1016/j.phrs.2017.02.004
Yang, W.S., SriRamaratnam, R., Welsch, M.E., Shimada, K., Skouta, R., Viswanathan, V.S., Cheah, J.H., Clemons, P.A., Shamji, A.F., Clish, C.B., Brown, L.M., Girotti, A.W., Cornish, V.W., Schreiber, S.L., Stockwell, B.R., 2014. Regulation of ferroptotic cancer cell death by GPX4. Cell 156, 317-331. https://doi.org/10.1016/j.cell.2013.12.010
Zhao, L., Zhao, J., Zhong, K. et al. Targeted protein degradation: mechanisms, strategies and application. Sig Transduct Target Ther 7, 113 (2022)
Zheng, J., Conrad, M., 2025. Ferroptosis: when metabolism meets cell death. Physiol Rev 105, 651-706. https://doi.org/10.1152/physrev.00031.2024

## Claims

1. An agent modulating the expression and/or activity of i) a KRAB-containing zinc finger protein ZNF354A (ZNF354A), ii) an mRNA encoding the ZNF354A, and/or iii) the ZNF354A gene, for use in the treatment and/or prevention of disease.

2. The agent for use of claim 1, wherein the disease is selected from the group comprising a cardiovascular disease, a neurodegenerative disease and an inflammatory disease.

3. The agent for use of claim 1 or 2, wherein said agent inhibits the translation of an mRNA encoding ZNF354A.

4. The agent for use of claim 1 or 2, wherein the agent inhibits the transcription of the gene encoding ZNF354A.

5. The agent for use of any one of the preceding claims, wherein the agent inhibits the activity of a ZNF354A protein
i) by enhancing and/or favoring the degradation of a ZNF354A protein,
ii) by preventing the recognition of its targets,
iii) by modulating the phosphorylation of a ZNF354A protein, and/or
iv) by any other means resulting in a loss of function.

6. The agent for use of any one of the preceding claims, wherein the agent is selected from the group comprising a nucleic acid, a chemical compound, a peptide or analog thereof, an antibody or an antigen-binding fragment thereof, and an antibody mimetic, or a combination of two or more thereof.

7. The agent for use of claim 6, wherein the nucleic acid is selected from the group comprising a nucleic acid encoding an siRNA, an miRNA, a piRNA, an hnRNA, an snRNA, a gRNA, a CRISPR-based loss-of-function system, an esiRNA, an shRNA, and an antisense oligonucleotide (ASO), or a combination of two or more thereof.

8. The agent for use of any one of the preceding claims, wherein
- the mRNA encoding ZNF354A is selected from the group comprising a sequence set forth in SEQ ID NO: 1, SEQ ID NO: 3, SEQ ID NO: 5, SEQ ID NO: 7, SEQ ID NO: 9, SEQ ID NO: 11, SEQ ID NO: 13, and a SEQ ID NO: 15 , a fragment or a variant thereof or a combination of two or more thereof, and/or
- the gene encoding ZNF354A is selected from the group comprising a sequence set forth in SEQ ID NO: 17, a fragment or a variant thereof, and/or
- the ZNF354A protein is selected from the group comprising a sequence as set forth in SEQ ID NO: 2, SEQ ID NO: 4, SEQ ID NO: 6, SEQ ID NO: 8, SEQ ID NO: 10, SEQ ID NO: 12, SEQ ID NO: 14, and a SEQ ID NO: 16, a fragment or a variant thereof or a combination of two or more thereof.

9. A short interfering ribonucleic acid (siRNA) for inhibiting the expression of ZNF354A.

10. The siRNA of claim 9, wherein the sense strand of the siRNA targets the ZNF354A mRNA sequence set forth in SEQ ID NO: 1, SEQ ID NO: 3, SEQ ID NO: 5, SEQ ID NO: 7, SEQ ID NO: 9, SEQ ID NO: 11, SEQ ID NO: 13, and a SEQ ID NO: 15, a fragment or a variant thereof or a combination of two or more thereof, and is at least 80% homologous to at least 2 to 30 contiguous nucleotides of the ZNF354A mRNA sequence as set forth in SEQ ID NO: 1, SEQ ID NO: 3, SEQ ID NO: 5, SEQ ID NO: 7, SEQ ID NO: 9, SEQ ID NO: 11, SEQ ID NO: 13, and a SEQ ID NO: 15, a fragment or a variant thereof.

11. The siRNA of claim 9 or 10, wherein the siRNA comprises one or more modified nucleotides.

12. A guide ribonucleic acid (sgRNA or gRNA) targeting a genomic site of the ZNF354A gene set forth in SEQ ID NO: 17, a fragment or a variant thereof or a regulatory sequence of said ZNF354A gene set forth in SEQ ID NO: 32, a fragment or a variant thereof.

13. A plasmid or a vector comprising one or more nucleic acids encoding the siRNA, miRNA, piRNA, hnRNA, snRNA, gRNA, CRISPR-based loss-of-function system, esiRNA, shRNA, and antisense oligonucleotide (e.g. Gapmer antisens), or combination of one or more thereof, of claim 7.

14. A host cell comprising, or modified by the introduction of,
i) a plasmid or vector of claim 13, or
ii) one or more nucleic acids encoding the siRNA, miRNA, piRNA, hnRNA, snRNA, gRNA, CRISPR-based loss-of-function system, esiRNA, shRNA, and antisense oligonucleotide, or combination of two or more thereof, of claim 7.

15. The agent for use of claim 1, wherein the disease is a solid cancer or a liquid (e.g. hematologic) cancer.
